# EUROPEAN PATENT APPLICATION

(11) **EP 1 219 305 A1**
(43) Date of publication of application: **03.07.2002**
(21) Application number: 00128401.7
(22) Date of filing: 27.12.2000
(51) Int. Cl.: A61K 47/48

(54) **Conjugates of integrin receptor antagonists and a cytostatic agent having specifically cleavable linking units**

(71) Applicant: Bayer Aktiengesellschaft, 51368 Leverkusen (DE)
(72) Inventor: Lerchen, Hans-Georg, Dr., 51375 Leverkusen (DE); Baumgarten, Jörg, Dr., 42115 Wuppertal (DE); Lockhoff, Oswald, Dr., 51373 Leverkusen (DE); Albers, Markus, Dr., 51375 Leverkusen (DE); Schoop, Andreas, Dr., 88441 Mittelbiberach (DE)

(57) **Abstract**

The present invention relates to cytostatics which have a tumor-specific action as a result of linkage to αᵥβ₃ integrin antagonists via preferred linking units which can be selectively cleaved by enzymes such as metallo matrixproteases (MMPs), i.e. by enzymes which can especially be found in tumor tissue. The preferred linking units guarantee the serum stability of the conjugate of cytostatic and αᵥβ₃ integrin antagonist and, at the same time, the desired intracellular action within tumour cells as a result of its specific enzymatic or hydrolytic cleavability with release of the cytostatic.

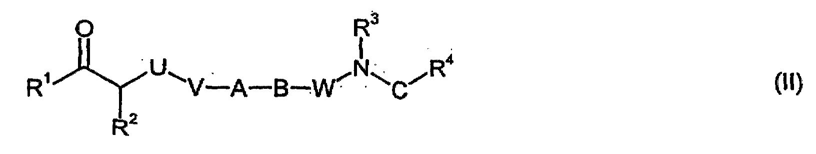

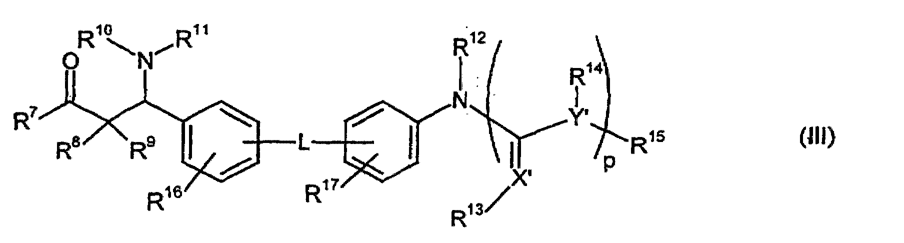

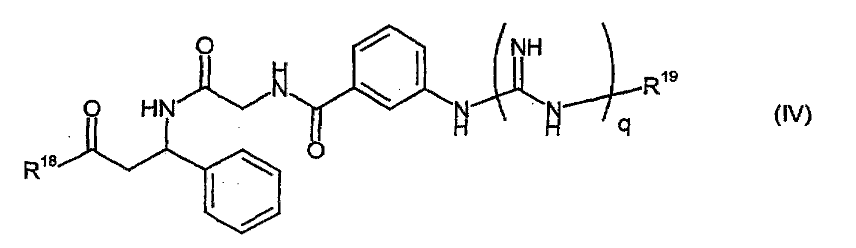

## Description

The present invention relates to cytostatics which have a tumor-specific action as a result of linkage to αᵥβ₃ integrin antagonists via preferred linking units which can be selevtively cleaved by enzymes such as metallo matrixproteases (MMPs, i.e. by enzymes which can especially be found in tumor tissue. The preferred linking units provide sufficient serum stability of the conjugate of cytostatic and αᵥβ₃ integrin antagonist and, at the same time, the desired intracellular action within tumour cells as a result of its specific enzymatic or hydrolytic cleavability with release of the cytostatic.

Chemotherapy in cancer is accompanied by usually serious side effects which are to be attributed to the toxic action of chemotherapeutics on proliferating cells of other tissue types than tumour tissue. For many years, scientists have occupied themselves with the problem of improving the selectivity of active compounds employed. A frequently followed approach is the synthesis of prodrugs which are released more or less selectively in the target tissue, for example, by change of the pH (Tietze et al., e.g. DE-A-4 229 903), by enzymes (e.g. glucuronidases; Jacquesy et al., EP-A-0 511 917; Bosslet et al., EP-A-0 595 133) or by antibody-enzyme conjugates (Bagshawe et al., WO 88/07378; Senter et al., US-4 975 278; Bosslet et al., EP-A-0 595 133). A problem in these approaches is, inter alia, the lack of stability of the conjugates in other tissues and organs, and in particular the ubiquitous active compound distribution which follows the extracellular release of active compound in the tumour tissue.

The marked lectin pattern on tumour cell surfaces (Gabius; Onkologie 12, (1989), 175) opens up the fundamental possibility of addressing these specifically on tumour cells by linkage of appropriate carbohydrate units to cytostatics. This prospect is restricted by the fact that, even in other tissues, in particular in the liver, lectins having similar carbohydrate specificities (galactose, lactose, mannose, N-acetylglucosamine, fucose etc.) occur (Ashwell et al., Annu. Rev. Biochem. 46 (1982), 531; Stahl et al. Proc. Natl. Acad. Sci. USA 74 (1977), 1521; Hill et al., J. Biol. Chem. 262 (1986), 7433; Jansen et al., J. Biol. Chem. 266 (1991), 3343). Accordingly, a marked concentration of active compound-containing glycoconjugates in the liver and other lectin-rich organs must be expected if, in this approach, carbohydrates are used without particular modification establishing a selectivity to tumour tissue.

The heterocyclic amine batracylin (1) shows a good antitumour action in various stomach cancer models (US-4 757 072).

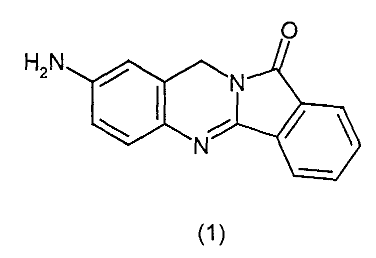

Peptide conjugates of (1) having good in-vitro action and more favourable solubility properties (US-4 180 343) are more poorly tolerable in animal experiments than free batracylin. The fucose conjugates of batracylin (1) described in EP-A-0 501 250 disadvantageously concentrate very strongly in the liver.

Quinolone-a (2), 7-[(3a-R,S, 4-R,S, 7a-S,R)-4-amino-1,3,3a,4,7,7a-hexahydro-iso-indol-2-yl]-8-chloro-1-cyclopropyl-6-fluoro-1,4-dihydro-4-oxo-3-quinolinecarboxylic acid, also shows, in addition to its outstanding antibacterial activity, a very good activity against various tumour cell lines (EP-A-0 520 240, JP-4 253 973). However, considerable toxicological problems face it (e.g. genotoxicity, bone marrow toxicity, high acute toxicity in vivo etc.). 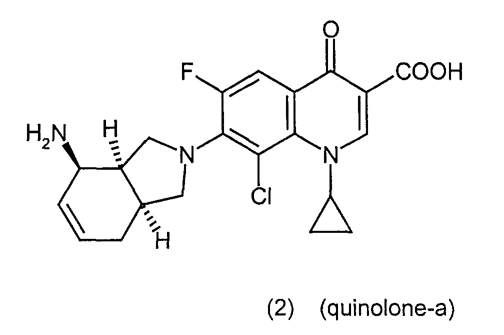 20(S)-Camptothecin is a pentacyclic alkaloid which was isolated in 1966 by Wall et al. (J. Am. Chem. Soc. 88, 3888 (1966)). It has a high active antitumour potential in numerous in-vitro and in-vivo tests. Unfortunately, however, the realization of the promising potential in the clinical investigation phase failed because of toxicity and solubility problems.

By opening of the E ring lactone and formation of the sodium salt, a water-soluble compound was obtained which is in a pH-dependent equilibrium with the ring-closed form. Here too, clinical studies have not led to success as yet.

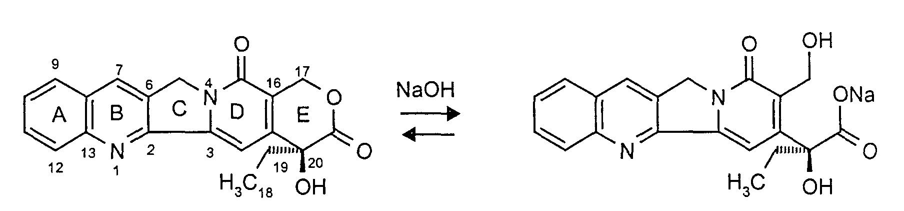

About 20 years later, it was found that the biological activity is to be attributed to enzyme inhibition of topoisomerase I. Since then, the research activities have again been increased in order to find a camptothecin derivative which is more tolerable and which is active in vivo.

For improvement of the water solubility, salts of A ring- and B ring-modified camptothecin derivatives and of 20-O-acyl derivatives with ionizable groups have been described (Vishnuvajjala et al. US 4 943 579). The latter prodrug concept was later also transferred to modified camptothecin derivatives (Wani et al. WO 9602546). The described 20-O-acyl prodrugs, however, have a very short half-life in vivo and are very rapidly cleaved to give the parent structure.

WO 96/31532 describes carbohydrate-modified cytostatics in which both serum stability and release of the cytostatic within the tumour cells and a specific concentration of the cytostatic in tumour tissue is achieved by a novel linkage of selectively modified carbohydrates to cytostatics (for example batracylin, quinolone-a, camptothecin) via preferred spacer and linker groups.

Integrins are heterodimeric transmembrane proteins found on the surface of cells, which play an important part in the adhesion of the cells to an extracellular matrix. They recognize extracellular glycoproteins such as fibronectin or vitronectin on the extracellular matrix via the RGD sequence occurring in these proteins (RGD is the single-letter code for the amino acid sequence arginine-glycine-aspartate).

In general, integrins such as, for example, the vitronectin receptor, which is also called the αᵥβ₃ receptor, or alternatively the αᵥβ₅ receptor or the GpIIb/IIIa receptor play an important part in biological processes such as cell migration, angiogenesis and cell-matrix adhesion and thus for diseases in which these processes are crucial steps. Cancer, osteoporosis, arteriosclerosis, restenosis and ophthalmia may be mentioned by way of example.

The αᵥβ₃ receptor occurs, for example, in large amounts on growing endothelial cells and makes possible their adhesion to an extracellular matrix. The αᵥβ₃ receptor thus plays an important part in angiogenesis, i.e. the formation of new blood vessels, which is a crucial prerequisite for tumour growth and metastasis formation in carcinomatous disorders.

It was possible to show that the blockade of the abovementioned receptors is an important starting point for the treatment of disorders of this type. If the adhesion of growing endothelial cells to an extracellular matrix is suppressed by blocking their corresponding integrin receptors, for example, by a cyclic peptide or a monoclonal antibody, the endothelial cells die. Angiogenesis therefore does not occur, which leads to a stoppage or regression of tumour growth (cf., for example, Brooks et al., Cell, Volume 79, 1157-1164, 1994).

Moreover, the invasive properties of tumour cells and thus their capability to form metastases markedly decrease when their αᵥβ₃ receptor is blocked by an antibody (Brooks et al., J. Clin. Invest., Volume 96, 1815, 1995).

WO 98/10795 describes conjugates in which a molecule adding to tumours is linked to a functional unit such as, for example, a cytostatic or a detectable label such as, for example, a radioactive nuclide. Inter alia, integrin antagonists such as, for example, peptides having the RGD sequence described above are described as molecules adding to tumours. Doxorubicin is described as an example of a cytostatic which is linked to a molecule of this type addressing tumours.

In the case of the compounds of WO 98/10795, the linkage is carried out such that the molecule addressing a tumour and the functional unit are directly bonded to one another with retention of their respective properties (cf., for example, p. 56, 1. 17, to p. 58, 1. 10, and Ex. 6). This has the result that these compounds are indeed selectively concentrated in the immediate vicinity of tumour cells by binding of the entity addressing a tumour (in the case of a radical having αᵥβ₃ integrin-antagonistic action by binding to the αᵥβ₃ integrin receptor which, in particular, is expressed on endothelial cells newly formed by angiogenesis), but on account of the direct combination the functional unit such as, for example, a cytostatic cannot be released into the intracellular space of the tumour tissue.

Fundamentally, the conjugate which on the one hand is selectively concentrated in tumour tissue by the effect of a part addressing αᵥβ₃ or αᵥβ₅ integrin receptors found in the conjugate, but on the other hand comprises a cytostatic which can be released from the conjugate, should have an increased toxophoric effect on tumour tissue due to the possibility of the more direct action of the cytostatic on the tumour cells compared with the conjugates described in WO 98/10795. In particular, such a toxophoric effect should even be higher if the release of the cytostatic takes place in the immediate vicinity of the tumor tissue or even in the tumor cells.

It was therefore the object of the present invention to develop conjugates which comprise a moiety addressing αᵥβ₃ integrin receptors and a cytostatic which can be released from the conjugate preferably at least in the vicinity of tumor tissue, where the moiety in the conjugate addressing αᵥβ₃ integrin receptors retains its ability to bind to the αᵥβ₃ integrin receptor.

The above object is achieved by conjugates which comprise a non-peptide moiety addressing αᵥβ₃ integrin receptors, a cytostatic and a linking unit which is selevtively enzymatically cleavable with release of the cytostatic by enzymes such as metallo matrixproteases (MMPs) i.e. by enzymes which can especially be found in tumor tissue.

In principle, medicament-containing conjugates are complex, difficult-to-prepare compounds, as is explained, for example, in Anti-Cancer Drug Design 10 (1995), 1-9, in particular p. 1. In this article, conjugates of the cytostatic methotrexate, an oligopeptide spacer and a protein (human serum albumin) are described. However, it is also pointed out (cf. p. 7, first paragraph) that the nature of the linking unit and the type of linkage of this unit to the toxophore and the carrier (for example an antibody) can affect the cleavage of the linking unit. This article therefore teaches that the linkage presented there cannot be transferred to other conjugate systems without difficulty. In particular, nothing is said about whether moieties addressed also to αᵥβ₃ integrin receptors in this manner can be linked to toxophores without the moiety addressing αᵥβ₃ integrin receptors by this means losing its ability to bind to αᵥβ₃ integrin receptors.

The linking units disclosed in WO 96/31532 are used specifically for the linkage of a toxophore to an oligosaccharide radical. Nothing is said about whether moieties addressed also to αᵥβ₃ integrin receptors can be linked to toxophores in this manner, without, by this means, the moiety addressing αᵥβ₃ integrin receptors losing its ability to bind to αᵥβ₃ integrin receptors.

According to the present invention, the linking unit can be cleaved by tumour-associated enzymes. This leads to a further increase in the tissue specificity of the conjugates according to the invention and thus to an additional decrease of the conjugates according to the invention in other tissue types.

According to a further preferred embodiment of the invention, the linking unit can be cleaved by enzymes which are coupled to antibodies with selectivity for tumour tissue and are thus addressed to tumour tissue. This is also called the ADEPT approach. This likewise leads to a further increase in the tissue specificity of the conjugates according to the invention and thus to an additional decrease of the conjugates according to the invention in other tissue types.

Particularly preferred conjugates according to the present invention are those of the general formula (I)

CT-LI-Sp-IA (I)

in which
- CT: denotes a cytotoxic radical or a radical of a cytostatic or of a cytostatic derivative, which can additionally carry a hydroxyl, carboxyl or amino group,

- LI: is a linker group comprising 5 to 8 amino acid residues in the D or L configuration, which can each optionally carry protective groups,
- Sp: is absent or a carbonyl or a thiocarbonyl radical,
- IA: is a non-peptide radical addressing an αᵥβ₃ integrin receptor, which is selected from the group consisting of

A) a radical of the formula (II) 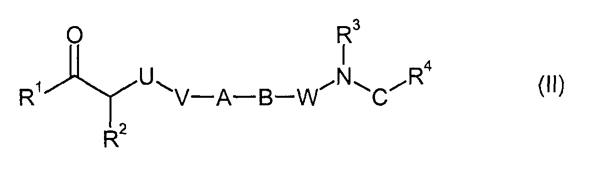 in which
   - R¹: is OH, a substituted or unsubstituted alkoxy or cycloalkoxy radical, a substituted or unsubstituted aryloxy radical or a saturated or unsaturated, optionally substituted heterocyclyloxy radical, or optionally represents a direct bond or an atom from the group consisting of O, N and S, via which the radical of the formula (II) is bonded to the rest of the conjugate;
   - R²: is hydrogen, a substituted or unsubstituted alkyl or cycloalkyl radical, a substituted or unsubstituted aryl radical, a saturated or unsaturated, optionally substituted heterocyclic radical, an optionally substituted alkenyl radical or an optionally substituted alkinyl radical, via which the radical of the formula (II) is optionally bonded to the rest of the conjugate, or is -NR²'₂, -NR²'SO₂R²", -NR²'COOR²", -NR²'COR²', -NR²'CONR²'₂ or -NR²'CSNR²'₂; -NR²'CONR²'₂ or -NR²' CSNR²'₂;
   in which
   - R²': independently of one another is hydrogen, a substituted or unsubstituted alkyl or cycloalkyl radical, a substituted or unsubstituted aryl radical or a saturated or unsaturated, optionally substituted heterocyclic radical, or optionally represents a direct bond, via which the radical of the formula (II) is bonded to the rest of the conjugate;
   - R²": is a substituted or unsubstituted alkyl or cycloalkyl radical, a substituted or unsubstituted aryl radical or a saturated or unsaturated, optionally substituted heterocyclic radical, via which the radical of the formula (II) is optionally bonded to the rest of the conjugate;
   - U: is a direct bond or a substituted or unsubstituted alkylene group, via which the radical of the formula (II) is optionally bonded to the rest of the conjugate;
   - V: is a substituted or unsubstituted alkylene group, -NR²'CO- or -NR²'SO₂ -, via which the radical of the formula (II) is optionally bonded to the rest of the conjugate;
   - A and B: each independently of one another is a 1,3- or 1,4-bridged, optionally additionally substituted phenylene group;
   - W: is a direct bond or a substituted or unsubstituted alkylene group;
   - C: is absent or is 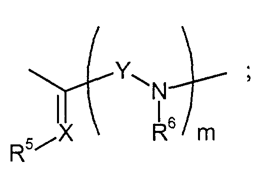
   - R³: is hydrogen, a substituted or unsubstituted alkyl or cycloalkyl radical, a substituted or unsubstituted aryl radical, a saturated or unsaturated, optionally substituted heterocyclic radical, an alkylamine radical, an alkylamide radical or is bonded to one of R⁴, Y, R⁵ or R⁶, if present, with formation of an optionally substituted heterocyclic ring system which includes the nitrogen atom to which R³ is bonded and can be saturated or unsaturated and/or can contain further heteroatoms;
   - R⁴: is hydrogen, a substituted or unsubstituted alkyl or cycloalkyl radical, a substituted or unsubstituted aryl radical, a saturated or unsaturated, optionally substituted heterocyclic radical, an alkylamine radical, an alkylamide radical or is bonded to one of R³, Y, R⁵ or R⁶, if present, with formation of an optionally substituted heterocyclic ring system which includes the nitrogen atom to which R⁴ is bonded and can be saturated or unsaturated and/or can contain further heteroatoms, or optionally represents a direct bond, via which the radical of the formula (II) is bonded to the rest of the conjugate;
   - X: is O, N or S;
   - m: is 0 or 1;
   - Y: is a direct bond or an optionally substituted alkylene or alkine group;
   - R⁵: is absent, -NO₂, -CN, -COR⁵', -COOR⁵', or is bonded to one of R³, Y, R⁴ or R⁶, if present, with formation of an optionally substituted carbocyclic or heterocyclic ring system which includes X and can be saturated or unsaturated and/or can contain further heteroatoms;
   - R⁵': is hydrogen, a substituted or unsubstituted alkyl or cycloalkyl radical, a substituted or unsubstituted aryl radical or a saturated or unsaturated, optionally substituted heterocyclic radical which can be saturated or unsaturated and/or can contain further heteroatoms;
   - R⁶: is hydrogen, a substituted or unsubstituted alkyl or cycloalkyl radical, a substituted or unsubstituted aryl radical, a saturated or unsaturated, optionally substituted heterocyclic radical, an alkylamine radical, an alkylamide radical or is bonded to one of R³, R⁴, Y or R⁵, if present, with formation of an optionally substituted heterocyclic ring system which includes the nitrogen atom to which R⁶ is bonded and can be saturated or unsaturated and/or can contain further heteroatoms;
   or
B) a radical of the formula (III) 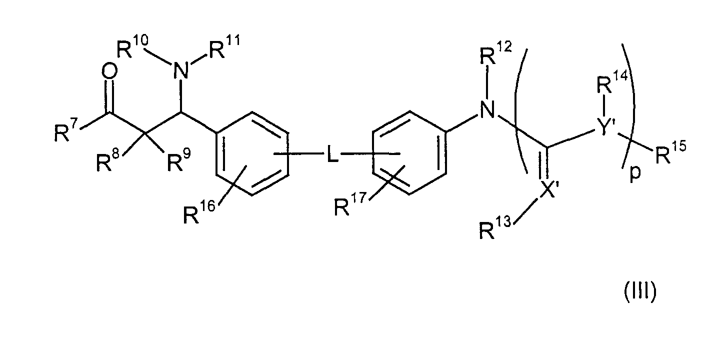 in which
   - R⁷: is OH, a substituted or unsubstituted alkoxy or cycloalkoxy radical, a substituted or unsubstituted aryloxy radical or a saturated or unsaturated, optionally substituted heterocyclyloxy radical, or optionally represents a direct bond or an atom from the group consisting of N, O and S, via which the radical of the formula (III) is bonded to the rest of the conjugate;
   - R⁸: is hydrogen, a substituted or unsubstituted alkyl or cycloalkyl radical, a substituted or unsubstituted aryl radical, a saturated or unsaturated, optionally substituted heterocyclic radical, an optionally substituted alkenyl radical, an optionally substituted alkinyl radical, a hydroxyl radical or an alkoxy radical or is bonded to R⁹ with formation of an optionally substituted carbocyclic or heterocyclic ring system which includes the carbon atom to which R⁸ is bonded and can optionally contain heteroatoms;
   - R⁹: is hydrogen, a substituted or unsubstituted alkyl or cycloalkyl radical, a substituted or unsubstituted aryl radical, a saturated or unsaturated, optionally substituted heterocyclic radical, an optionally substituted alkenyl radical, an optionally substituted alkinyl radical, a hydroxyl radical or an alkoxy radical or is bonded to R⁸ with formation of an optionally substituted carbocyclic or heterocyclic ring system which includes the carbon atom to which R⁹ is bonded and can optionally contain heteroatoms;
   - R¹⁰: is -SO₂R¹⁰', -COOR¹⁰", -COR¹⁰', -CONR¹⁰'₂ or -CS-NR¹⁰'₂, or represents a direct bond via which the radical of the formula (III) is optionally bonded to the rest of the conjugate;
   - R¹⁰': independently of one another is hydrogen, a substituted or unsubstituted alkyl or cycloalkyl radical, a substituted or unsubstituted aryl radical or a saturated or unsaturated, optionally substituted heterocyclic radical, via which the radical of the formula (III) is optionally bonded to the rest of the conjugate;
   - R¹⁰": is a substituted or unsubstituted alkyl or cycloalkyl radical, a substituted or unsubstituted aryl radical or a saturated or unsaturated, optionally substituted heterocyclic radical, via which the radical of the formula (III) is optionally bonded to the rest of the conjugate;
   - R¹¹: is hydrogen, a substituted or unsubstituted alkyl or cycloalkyl radical or a substituted or unsubstituted aryl radical,
   - R¹⁶: is hydrogen, CN, a substituted or unsubstituted alkyl or cycloalkyl radical, a substituted or unsubstituted alkoxy radical or a halogen atom;
   - R¹⁷: is hydrogen, CN, a substituted or unsubstituted alkyl or cycloalkyl radical, a substituted or unsubstituted alkoxy radical or a halogen atom;
   - L: is -(CH₂)ₙNHSO₂(CH₂)ₒ-, -(CH₂)ₙSO₂NH(CH₂)ₒ-, -(CH₂)ₙNH-CO(CH₂)ₒ-, -(CH₂)ₙCONH(CH₂)ₒ-, -(CH₂)ₙOCH₂(CH₂)ₒ-, -(CH₂)ₙCH₂O(CH₂)ₒ-, -(CH₂)ₙCOO(CH₂)ₒ-, -(CH₂)ₙOOC-(CH₂)ₒ-, -(CH₂)ₙCH₂CO(CH₂)ₒ-, -(CH₂)ₙCOCH₂(CH₂)ₒ-, -NHCONH-, -(CH₂)ₙSCH₂(CH₂)ₒ-, -(CH₂)ₙCH₂S(CH₂)ₒ-, -(CH₂)ₙCH₂SO(CH₂)ₒ-, -(CH₂)ₙSOCH₂(CH₂)ₒ-, -(CH₂)ₙCH₂- SO₂(CH₂)ₒ- or -(CH₂)ₙSO₂CH₂(CH₂)ₒ-, where n and o each is an integer of 0 or 1 and n + o ≤ 1;
   - R¹²: is hydrogen, a substituted or unsubstituted alkyl or cycloalkyl radical, a substituted or unsubstituted aryl radical, a saturated or unsaturated, optionally substituted heterocyclic radical or is bonded to one of R¹³, R¹⁴ or R¹⁵, if present, with formation of an optionally substituted heterocyclic ring system which includes the nitrogen atom, to which R¹² is bonded and can be saturated or unsaturated and/or can contain further heteroatoms;
   - X': isN,OorS;
   - p: is 0 or 1;
   - R¹³: is absent, is -H, a substituted or unsubstituted alkyl or cycloalkyl radical, -NO₂, -CN, -COR¹³', -COOR¹³', or is bonded to one of R¹², R¹⁴ or R¹⁵ with formation of an optionally substituted heterocyclic ring system which includes X' and can be saturated or unsaturated and/or can contain further heteroatoms;
   - R¹³': is hydrogen, a substituted or unsubstituted alkyl or cycloalkyl radical, a substituted or unsubstituted aryl radical or a saturated or unsaturated, optionally substituted heterocyclic radical which can be saturated or unsaturated and/or can contain further heteroatoms;
   - Y': is N or S;
   - R¹⁴: is absent, hydrogen, a substituted or unsubstituted alkyl or cycloalkyl radical, a substituted or unsubstituted aryl radical, a saturated or unsaturated, optionally substituted heterocyclic radical or is bonded to one of R¹², R¹³ or R¹⁵, if present, with formation of an optionally substituted heterocyclic ring system which includes the nitrogen atom to which R¹⁴ is bonded and can be saturated or unsaturated and/or can contain further heteroatoms;
   - R¹⁵: is hydrogen, a substituted or unsubstituted alkyl or cycloalkyl radical, a substituted or unsubstituted aryl radical, a saturated or unsaturated, optionally substituted heterocyclic radical or is bonded to one of R¹², R¹³ or R¹⁴, if present, with formation of an optionally substituted heterocyclic ring system which includes the nitrogen atom to which R¹⁵ is bonded and can be saturated or unsaturated and/or can contain further heteroatoms, or optionally represents a direct bond via which the radical of the formula (III) is bonded to the rest of the conjugate;
   or
C) a radical of the formula (IV) 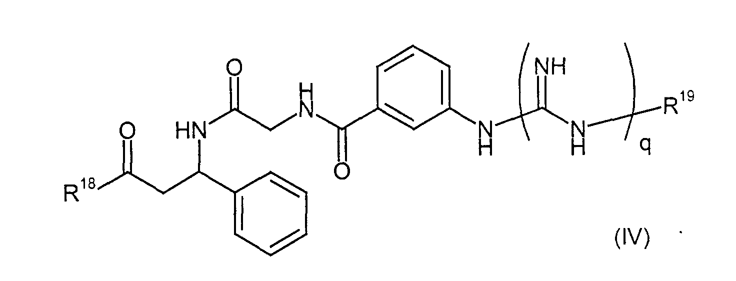 in which
   - R¹⁸: is OH, a substituted or unsubstituted alkoxy or cycloalkoxy radical, a substituted or unsubstituted aryloxy radical or a saturated or unsaturated, optionally substituted heterocyclyloxy radical, or optionally represents a direct bond or an atom from the group consisting of N, O and S, via which the radical of the formula (IV) is bonded to the rest of the conjugate;
   - q: is 0 or 1;
   - R¹⁹: is hydrogen, a substituted or unsubstituted alkyl or cycloalkyl radical, a substituted or unsubstituted aryl radical, a saturated or unsaturated, optionally substituted heterocyclic radical, an alkylamine radical, an alkylamide radical, or optionally represents a direct bond, via which the radical of the formula (IV) is bonded to the rest of the conjugate;
and their physiologically acceptable salts and stereoisomers.

Of the conjugates of the formula (I), according to a preferred embodiment those conjugates are preferred in which
- LI: is a linker group having the formula

-AA1-AA2-AA3-AA4-AA5-AA6-AA7-AA8-

wherein at least 5 of the radicals AA1 to AA8 are present, AA1 is bonded to the radical CT and
AA1 is a naturally occurring amino acid in the D or L configuration, which is selected from the group consisting of glycine, alanine, valine, leucine, isoleucine, histidine, glutamate, aspartate, serine, lysine, ornithine and phenylalanine;
AA2 is absent or is a naturally occurring amino acid in the D or L configuration, which is selected from the group consisting of alanine, valine, phenylalanine, tyrosine, threonine, serine, isoleucine, lysine, glutamate, histidine, glycine, arginine, asparagine, glutamine, S-methyl-cysteine, methionine, arginine, aspartate, tryptophane, proline, ornithine and leucine, and can optionally carry protective groups,
AA3 is absent or is a naturally occurring amino acid in the D or L configuration, which is selected from the group consisting of alanine, valine, phenylalanine, tyrosine, serine, isoleucine, lysine, glutamate, histidine, glycine, arginine, aspartate, tryptophane, proline, ornithine, methionine, S-methyl-cysteine, norvaline and leucine, and can optionally carry protective groups,
AA4 is absent or is a naturally occurring amino acid in the D or L configuration, which is selected from the group consisting of glycine, alanine, valine, leucine, isoleucine, cysteine and norvaline;
AA5 is absent or is a naturally occurring amino acid in the D or L configuration, which is selected from the group consisting of glycine, alanine, valine, leucine, isoleucine, histidine, tyrosine, glutamine, asparagine, proline, methionine, phenylalanine and cysteine;
AA6 is absent or is a naturally occurring amino acid in the D or L configuration, which is selected from the group consisting of glycine, alanine, valine, leucine, isoleucine, histidine, glutamine, asparagine, aspartate and proline;
AA7 is absent or is a naturally occurring amino acid in the D or L configuration, which is selected from the group consisting of glycine, alanine, valine, leucine, isoleucine, histidine, γ-aminobutyric acid, aspartate, glutamate, lysine and proline;
AA8 is absent or is a naturally occurring amino acid in the D or L configuration, which is selected from the group consisting of glycine, alanine, valine, leucine, isoleucine, histidine, lysine, proline and γ-aminobutyric acid;
and the other radicals CT, Sp and IA are as defined above.

Particularly preferred are conjugates according to formula (I), in which
- LI: is a linker group having the formula

-AA1-AA2-AA3-AA4-AA5-AA6-AA7-AA8-

wherein 5 to 7 of the radicals AA1 to AA8 are present, AA1 is bonded to the radical CT and
AA1 is valine, glycine, leucine, histidine;
AA2 is absent or is a naturally occurring amino acid in the D or L configuration, which is selected from the group consisting of alanine, phenylalanine, serine, isoleucine, glutamate, asparagine, glutamine, histidine, glycine, aspartate, tryptophane, proline, and leucine, and can optionally carry protective groups,
AA3 is absent or is a naturally occurring amino acid in the D or L configuration, which is selected from the group consisting of alanine, phenylalanine, serine, isoleucine, norvaline, S-methylcysteine, methionine, glutamate, histidine, glycine, aspartate, tryptophane, and leucine, and can optinally carry protective groups,
AA4 is absent or is a naturally occurring amino acid in the D or L configuration, which is selected from the group consisting of glycine, leucine, cysteine and norvaline, and can optionally carry protective groups,
AA5 is absent or is a naturally occurring amino acid in the D or L configuration, which is selected from the group consisting of glycine, alanine, valine, leucine, histidine, glutamine, phenylalanine, isoleucine, and methionine,
AA6 is absent or is a naturally occurring amino acid in the D or L configuration, which is selected from the group consisting of glycine, proline, glutamine, methionine, and leucine;
AA7 is absent or is a naturally occurring amino acid in the D or L configuration, which is selected from the group consisting of glycine, leucine, aspartate, histidine, γ-aminobutyric acid and proline;
AA8 is absent or is a naturally occurring amino acid in the D or L configuration, which is selected from the group consisting of glycine, proline and γ-aminobutyric acid ;
and the other radicals CT, Sp and IA are as defined above.

Of the conjugates of the formula (I), according to a preferred embodiment those conjugates are preferred in which
- CT: is camptothecin or a camptothecin derivative, which can be bonded to the rest of the conjugate via the C20-OH group,
- LI: is as defined above;
- Sp: is absent, or is a carbonyl or a thiocarbonyl radical,
- IA: denotes a non-peptide radical of the formula (II) addressing an αᵥβ₃ integrin receptor,
in which
R¹ is OH, methoxy, ethoxy, propoxy, isopropoxy, butoxy, isobutoxy, t-butoxy, pentoxy, isopentoxy, neopentoxy, hexoxy, cyclopropoxy, cyclopropylmethoxy, cyclobutoxy, cyclopentoxy, cyclohexoxy, phenoxy, benzyloxy, tolyloxy or a substituted derivative thereof, or optionally represents a direct bond or an atom from the group consisting of N, O and S, via which the radical of the formula (II) is bonded to the rest of the conjugate;
R² is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, phenyl, benzyl, tolyl or a substituted derivative thereof, an optionally substituted alkenyl radical or an optionally substituted alkinyl radical, via which the radical of the formula (II) is optionally bonded to the rest of the conjugate, or is -NR²'₂, -NR²'SO₂R²", -NR²'COOR²", -NR²'COR²',-NR²'CONR²'₂ or -NR²'CSNR²'₂,
in which
R²' independently of one another is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, phenyl, benzyl, tolyl or a substituted derivative thereof, or optionally represents a direct bond via which the radical of the formula (II) is bonded to the rest of the conjugate;
R²" is methyl, ethyl, propyl, isopropyl, butyl, isobutyl, t-butyl, pentyl, isopentyl, neopentyl, hexyl, cyclopropyl, cyclobutyl, cyclopentyl, cyclohexyl, phenyl, benzyl, tolyl or a substituted derivative thereof, -C₆H₂(CH₃)₃, 3-aminophenyl, 4-aminophenyl, 2-chlorophenyl, 4-chlorophenyl, 4-methoxyphenyl, 2,5-dichlorophenyl, 4-trifluoromethylphenyl, camphor-10-yl, 4-t-butylphenyl, 2,5-dimethylphenyl, 3-chlorophenyl, 2-methoxy-5-methylphenyl, 2,3,5,6-tetramethylphenyl, 2,3-dichlorophenyl, 2,6-dichlorophenyl, 2-naphthyl, 3-trifluoromethylphenyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline or 8-quinolinyl, via which the radical of the formula (II) is optionally bonded to the rest of the conjugate;
U is a direct bond,
V is an optionally substituted C₁₋₅-alkylene group, via which the radical of the formula (II) is optionally bonded to the rest of the conjugate;
A is a 1,3- or 1,4-bridged phenylene group which is unsubstituted or contains at least one alkoxy radical;
B is a 1,3- or 1,4-bridged phenylene group which is unsubstituted or contains at least one alkyl radical;
W is a direct bond or an optionally substituted C₁₋₄-alkylene group;
C is a direct bond or 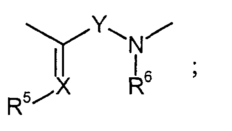
R³ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, 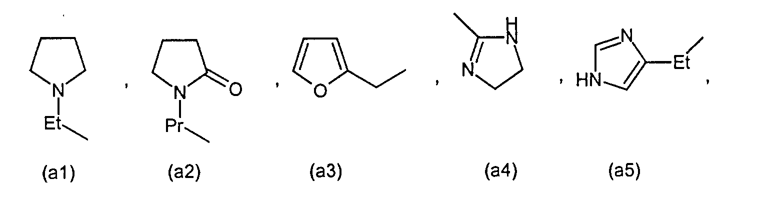 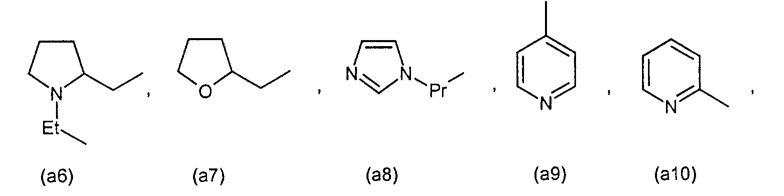 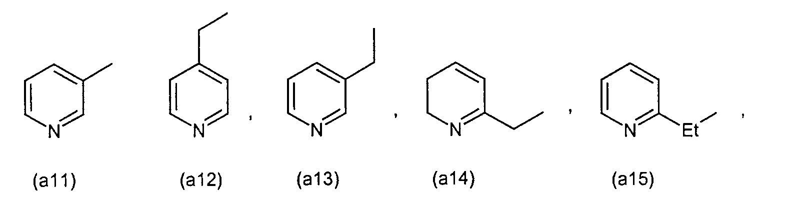 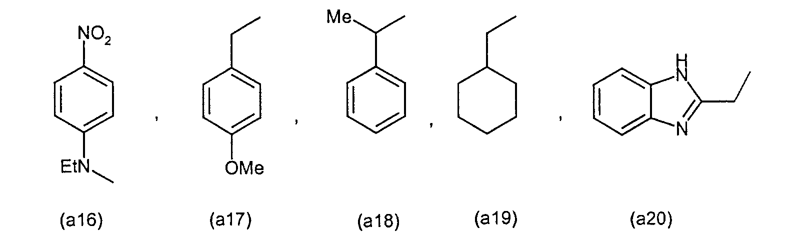 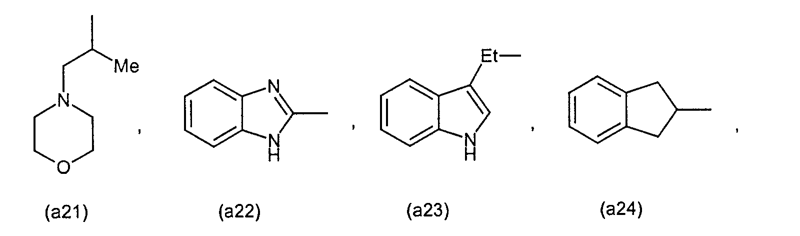 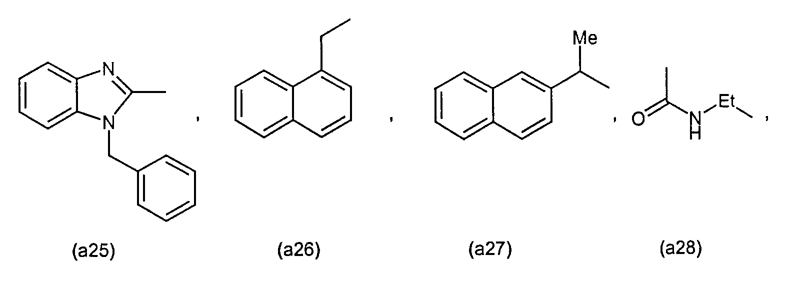 or is bonded to one of R⁴, Y, R⁵ or R⁶, if present, with formation of an optionally substituted heterocyclic 4- to 6-membered ring system, which includes the nitrogen atom to which R³ is bonded and can be saturated or unsaturated and/or can contain further heteroatoms;
R⁴ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28) or is bonded to one of R³, Y, R⁵ or R⁶, if present, with formation of an optionally substituted heterocyclic 4- to 6-membered ring system which includes the nitrogen atom to which R⁴ is bonded and can be saturated or unsaturated and/or can contain further heteroatoms, or optionally represents a direct bond via which the radical of the formula (II) is bonded to the rest of the conjugate;
X is O, N or S;
Y is a direct bond or a substituted or unsubstituted methylene or methine group;
R⁵ is absent, is -NO₂, -CN, -COR⁵', -COOR⁵' or is bonded to one of R³, Y, R⁴ or R⁶, if present, with formation of an optionally substituted carbocyclic or heterocyclic 4-to 6-membered ring system which includes X and which can be saturated or unsaturated and/or can contain further heteroatoms;
R⁵' is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, phenyl, benzyl, tolyl or a substituted derivative thereof;
R⁶ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkyl-amino-C₁₋₄-alkyl, C₁₋₄-dialkylamino-C₁₋₄-alkyl, aminoC₁₋₄-alkyl, C₁₋₄-alkyloxy-C₁₋₄-alkyl, one of the radicals (a1) to (a28) or is bonded to one of R³, Y, R⁴ or R⁵, if present, with formation of an optionally substituted heterocyclic 4- to 6-membered ring system which includes the nitrogen atom to which R⁶ is bonded and can be saturated or unsaturated and/or can contain further heteroatoms.

Particularly preferred conjugates of the formula (I) in this preferred embodiment are those in which R¹ represents a direct bond or an atom from the group consisting of N, O and S, via which the radical of the formula (II) is bonded to the rest of the conjugate, and the other radicals of the formula (II) are as defined above.

Likewise, particularly preferred conjugates of the formula (I) in this preferred embodiment are those in which R⁴ represents a direct bond, via which the radical of the formula (II) is bonded to the rest of the conjugate, and the other radicals of the formula (II) are as defined above.

Likewise, particularly preferred conjugates of the formula (I) in this preferred embodiment are those in which the radical of the formula (II) is linked to the rest of the conjugate via a radical in the α- or β-position relative to the carboxyl group, and the other radicals of the formula (II) are as defined above.

Of the conjugates of the formula (I), according to a further preferred embodiment those conjugates are particularly preferred in which
- CT: is camptothecin or a camptothecin derivative, which can be linked to the rest of the conjugate via the C20-OH group
- LI: is as defined above;
- Sp: is absent, or a carbonyl or a thiocarbonyl radical,
- IA: is a non-peptide radical of the formula (II) addressing an αᵥβ₃ integrin receptor,
in which
R¹ is OH, methoxy, ethoxy, propoxy, isopropoxy, butoxy, isobutoxy, t-butoxy, pentoxy, isopentoxy, neopentoxy, hexoxy, cyclopropoxy, cyclopropylmethoxy, cyclobutoxy, cyclopentoxy, cyclohexoxy, phenoxy, benzyloxy, tolyloxy or a substituted derivative thereof, or optionally represents a direct bond or an atom from the group consisting of N, O and S, via which the radical of the formula (II) is bonded to the rest of the conjugate;
R² is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, phenyl, benzyl, 4-aminobenzyl, tolyl, phenylethyl, a substituted derivative such as 4-aminobenzyl or a saturated or unsaturated, optionally substituted heterocyclic analogue thereof, an optionally substituted alkenyl radical, an optionally substituted alkinyl radical, via which the radical of the formula (II) is optionally bonded to the rest of the conjugate;
U is a direct bond or an optionally substituted C₁₋₃-alkylene group such as -CH(C₆H₄-3-NH)- or -CH(C₆H₄-4-NH)-, via which the radical of the formula (II) is optionally bonded to the rest of the conjugate;
V is -NR²⁰CO- or -NR²⁰SO₂-;
R²⁰ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, phenyl, benzyl, tolyl, phenylethyl, phenylpropyl, phenoxyethyl or a substituted derivative thereof;
A is a 1,3- or 1,4-bridged phenylene group which is unsubstituted or contains at least one alkoxy radical;
B is a 1,3- or 1,4-bridged phenylene group which is unsubstituted or contains at least one alkyl radical;
W is a direct bond or an optionally substituted C₁₋₃-alkylene group;
C is 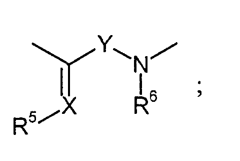
R³ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28) or is bonded to one of R⁴, Y or R⁶, if present, with formation of an optionally substituted heterocyclic 4- to 6-membered ring system, which includes the nitrogen atom to which R³ is bonded, and can be saturated or unsaturated and/or can contain further heteroatoms;
R⁴ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28) or is bonded to one of R³, Y or R⁶, if present, with formation of an optionally substituted heterocyclic 4- to 6-membered ring system, which includes the nitrogen atom to which R⁴ is bonded and can be saturated or unsaturated and/or can contain further heteroatoms, or optionally represents a direct bond via which the radical of the formula (II) is bonded to the rest of the conjugate;
X is O or S;
Y is a direct bond or a substituted or unsubstituted methylene or methine group;
R⁵ is absent;
R⁶ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28) or is bonded to one of R³, Y or R⁴, if present, with formation of an optionally substituted heterocyclic 4- to 6-membered ring system which includes the nitrogen atom to which R⁶ is bonded, and can be saturated or unsaturated and/or can contain further heteroatoms.

Particularly preferred conjugates of the formula (I) in this further preferred embodiment are those in which R¹ represents a direct bond or an atom from the group consisting of N, O and S, via which the radical of the formula (II) is bonded to the rest of the conjugate, and the other radicals of the formula (II) are as defined above.

Likewise, particularly preferred conjugates of the formula (I) in this further preferred embodiment are those in which R⁴ represents a direct bond, via which the radical of the formula (II) is bonded to the rest of the conjugate, and the other radicals of the formula (II) are as defined above.

Likewise, particularly preferred conjugates of the formula (I) in this further preferred embodiment are those in which the radical of the formula (II) is linked to the rest of the conjugate via a radical in the α- or β-position relative to the carboxyl group, and the other radicals of the formula (II) are as defined above.

Of the conjugates of the formula (I), according to yet a further preferred embodiment those conjugates are particularly preferred in which
- CT: is camptothecin, which can be linked to the rest of the conjugate via the C20-OH group;
- LI: is as defined above;
- Sp: is absent, or a carbonyl or a thiocarbonyl radical,
- IA: is a non-peptide radical of the formula (III) addressing an αᵥβ₃ integrin receptor,
in which
R⁷ is OH, methoxy, ethoxy, propoxy, isopropoxy, butoxy, isobutoxy, t-butoxy, pentoxy, isopentoxy, neopentoxy, hexoxy, cyclopropoxy, cyclopropylmethoxy, cyclobutoxy, cyclopentoxy, cyclohexoxy, phenoxy, benzyloxy, tolyloxy or a substituted derivative thereof, or optionally represents a direct bond or an atom from the group consisting of N, O and S, via which the radical of the formula (III) is bonded to the rest of the conjugate;
R⁸ is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -OH, methoxy, ethoxy, propoxy, butoxy, pentoxy, hexoxy, benzyloxy or is bonded to R⁹ with formation of an optionally substituted 3- to 6-membered carbocyclic or heterocyclic ring system, which includes the carbon atom to which R⁸ is bonded and can optionally contain heteroatoms;
R⁹ is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -OH, methoxy, ethoxy, propoxy, butoxy, pentoxy, hexoxy or is bonded to R⁸ with formation of an optionally substituted 3- to 6-membered carbocyclic or heterocyclic ring system which includes the carbon atom to which R⁹ is bonded and can optionally contain heteroatoms;
R¹⁰ is SO₂R¹⁰', -COOR¹⁰", -COR¹⁰', -CONR¹⁰'₂ or -CS-NR¹⁰'₂ or represents a direct bond, via which the radical of the formula (III) is optionally bonded to the rest of the conjugate;
R¹⁰' is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -C₆H₂(CH₃)₃, -C₆(CH₃)₅, -CH₂C₆H₂(CH₃)₃, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 4-chlorophenylmethyl, 2,4-dichlorophenylmethyl, 2,6-dichlorophenylmethyl, 3-aminophenyl, 4-aminophenyl, 2-methoxycarbonylphenylmethyl, 3-trifluoromethylphenyl, 4-trifluoromethylphenyl, 3,5-bis(trifluoromethyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimeth-oxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methylbenzothiazol-2-yl, N-methoxycarbonyl-piperidin-3-yl, thiophen-2-yl, isoxazol-5-yl, ethoxy, 2-chloropyridin-3-yl, pyridin-3-yl, benzyloxy, 5-methylisoxazol-3-yl, 1-adamantyl, 4-chlorophenoxymethyl, 2,2-dimethylethenyl, 2-chloropyridine-5-methyl, 5,7-dimethyl-1,3,4-triazaindolizin-2-yl, (S)-camphan-1-yl, (R)-camphan-1-yl or 8-quinolinyl;
R¹⁰" is a C₁₋₆-alkyl radical, a C₃₋₇-cycloalkyl radical, a substituted or unsubstituted aryl radical or a saturated or unsaturated, optionally substituted heterocyclic radical, via which the radical of the formula (III) is optionally bonded to the rest of the conjugate;
R¹¹ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, dialkylamino-C₁₋₄-alkyl, amino-C₁₋₄-alkyl, C₁₋₄-alkyloxy-C₁₋₄-alkyl or 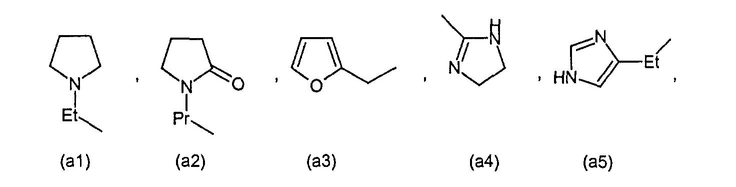 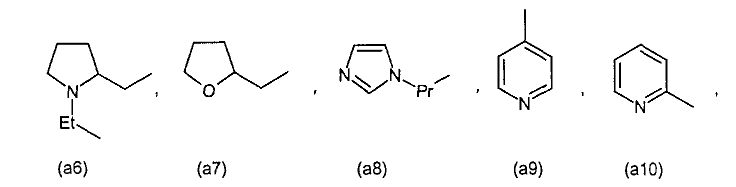 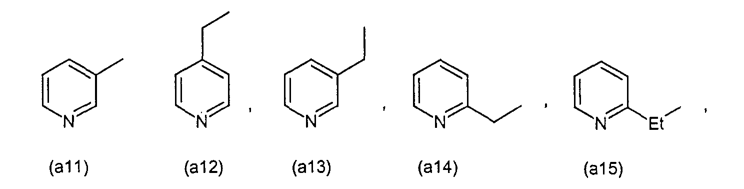 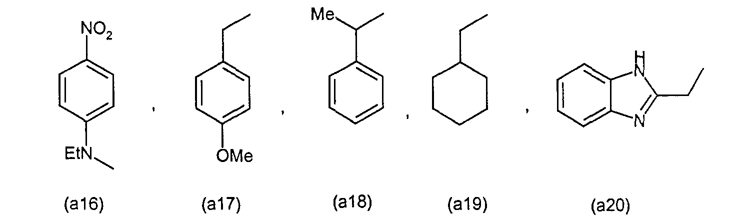 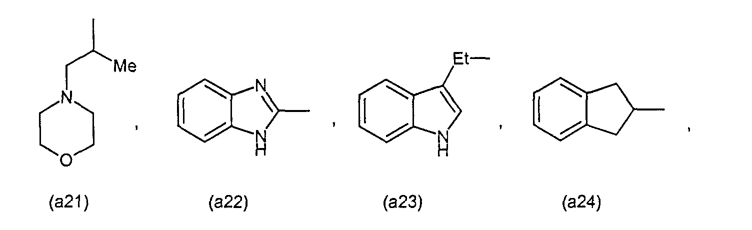 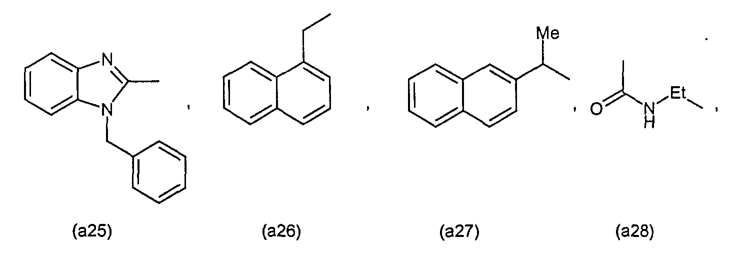
R¹⁶ is hydrogen, CN, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, methoxy, trifluoromethoxy, ethoxy, propoxy, butoxy, pentoxy or hexoxy, fluorine, chlorine, bromine or iodine;
R¹⁷ is hydrogen, CN, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, methoxy, ethoxy, trifluoromethoxy, propoxy, butoxy, pentoxy or hexoxy, fluorine, chlorine, bromine or iodine;
L is -NHSO₂-, -CH₂NHSO₂-, -NHSO₂CH₂-, -SO₂NH-, -CH₂SO₂NH-, -SO₂NHCH₂-, -NHCO-, -CH₂NHCO-, -NHCOCH₂-, -CONH-, -CH₂CONH-, -CONHCH₂-, -OCH₂-, -CH₂OCH₂, -OCH₂CH₂-, -CH₂O- or -CH₂CH₂O-;
R¹² is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28) or is bonded to one of R¹³, R¹⁴ or R¹⁵, if present, with formation of an optionally substituted heterocyclic 4- to 6-membered ring system which includes the nitrogen atom to which R¹² is bonded and can be saturated or unsaturated and/or can contain further heteroatoms;
X' is N, O or S;
p is 0 or 1;
R¹³ is absent, is -H, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, -NO₂, -CN, -COR⁷', -COOR⁷', or is connected to one of R¹², R¹⁴ or R¹⁵ with formation of an optionally substituted carbocyclic or heterocyclic 4- to 6-membered ring system which includes X' and can be saturated or unsaturated and/or can contain further heteroatoms;
R¹³' is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof;
Y' is N or S;
R¹⁴ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28), or is connected to one of R¹², R¹³ or R¹⁵, if present, with formation of an optionally substituted heterocyclic 4- to 6-membered ring system which includes the nitrogen atom to which R¹⁴ is bonded and can be saturated or unsaturated and/or can contain further hetero atoms; and
R¹⁵ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28), or is bonded to one of R¹², R¹³ or R¹⁴, if present, with formation of an optionally substituted heterocyclic 4- to 6-membered ring system which includes the nitrogen atom to which R¹⁵ is bonded and can be saturated or unsaturated and/or can contain further heteroatoms, and or optionally represents a direct bond via which the radical of the formula (III) is bonded to the rest of the conjugate.

Particularly preferred conjugates of the formula (I) in this further preferred embodiment are those in which R⁷ represents a direct bond or an atom from the group consisting of N, O and S, via which the radical of the formula (III) is bonded to the rest of the conjugate, and the other radicals of the formula (III) are as defined above.

Likewise, particularly preferred conjugates of the formula (I) in this further preferred embodiment are those in which R¹⁵ represents a direct bond, via which the radical of the formula (III) is bonded to the rest of the conjugate, and the other radicals of the formula (III) are as defined above.

Likewise particularly preferred conjugates of the formula (I) in this further preferred embodiment are those in which the radical of the formula (III) is linked to the rest of the conjugate via a radical in the α- or β-position relative to the carboxyl group, and the other radicals of the formula (III) are as defined above.

According to a further preferred embodiment, conjugates of the formula (I) are preferred in which R¹⁸ represents a direct bond or an atom from the group consisting of N, O and S, via which the radical of the formula (IV) is bonded to the rest of the conjugate, and the other radicals of the formula (IV) are as defined above.

Likewise particularly preferred conjugates of the formula (I) in this further preferred embodiment are those in which R¹⁹ represents a direct bond, via which the radical of the formula (IV) is bonded to the rest of the conjugate, and the other radicals of the formula (IV) are as defined above.

The compounds of the formula (I) according to the invention can also be present in the form of their salts. In general salts with organic or inorganic bases or acids may be mentioned here.

In particular, the compounds of the formula (I) according to the invention can be employed in the form of their physiologically acceptable salts. Physiologically acceptable salts are understood according to the invention as meaning non-toxic salts which in general are accessible by reaction of the compounds of the formula (I) according to the invention with an inorganic or organic base or acid conventionally used for this purpose. Examples of preferred salts of the compounds of the formula (I) according to the invention are the corresponding alkali metal salt, e.g. lithium, potassium or sodium salt, the corresponding alkaline earth metal salt such as the magnesium or calcium salt, a quaternary ammonium salt such as, for example, the triethylammonium salt, acetate, benzenesulphonate, benzoate, dicarbonate, disulphate, ditartrate, borate, bromide, carbonate, chloride, citrate, dihydrochloride, fumarate, gluconate, glutamate, hexylresorcinate, hydrobromide, hydrochloride, hydroxynaphthoate, iodide, isethionate, lactate, laurate, malate, maleate, mandelate, mesylate, methylbromide, methylnitrate, methylsulphate, nitrate, oleate, oxalate, palmitate, pantothenate, phosphate, diphosphate, polygalacturonate, salicylate, stearate, sulphate, succinate, tartrate, tosylate and valerate and other salts used for medicinal purposes.

The present invention includes both the individual enantiomers or diastereomers and the corresponding racemates, diastereomer mixtures and salts of the compounds according to the invention. In addition, all possible tautomeric forms of the compounds described above are included according to the present invention. Furthermore, the present invention includes both the pure E and Z isomers of the compounds of the formula (I) and their E/Z mixtures in all ratios. The diastereomer mixtures or E/Z mixtures can be separated into the individual isomers by chromatographic processes. The racemates can be resolved into the respective enantiomers either by chromatographic processes on chiral phases or by resolution.

In the context of the present invention, the substituents, if not stated otherwise, in general have the following meaning:
Alkyl in general represents a straight-chain or branched hydrocarbon radical having 1 to 20 carbon atoms. Examples which may be mentioned are methyl, ethyl, propyl, isopropyl, butyl, isobutyl, pentyl, isopentyl, hexyl, isohexyl, heptyl, isoheptyl, octyl and isooctyl, nonyl, decyl, dodeyl, eicosyl.
Alkenyl in general represents a straight-chain or branched hydrocarbon radical having 2 to 20 carbon atoms and one or more, preferably having one or two, double bonds. Examples which may be mentioned are allyl, propenyl, isopropenyl, butenyl, isobutenyl, pentenyl, isopentenyl, hexenyl, isohexenyl, heptenyl, isoheptenyl, octenyl, isooctenyl.
Alkinyl in general represents a straight-chain or branched hydrocarbon radical having 2 to 20 carbon atoms and one or more, preferably having one or two, triple bonds. Examples which may be mentioned are ethinyl, 2-butinyl, 2-pentinyl and 2-hexinyl.
Acyl in general represents straight-chain or branched lower alkyl having 1 to 9 carbon atoms, which is bonded via a carbonyl group. Examples which may be mentioned are: acetyl, ethylcarbonyl, propylcarbonyl, isopropylcarbonyl, butylcarbonyl and isobutylcarbonyl.
Alkoxy in general represents a straight-chain or branched hydrocarbon radical having 1 to 14 carbon atoms and bonded via an oxygen atom. Examples which may be mentioned are methoxy, ethoxy, propoxy, isopropoxy, butoxy, isobutoxy, pentoxy, isopentoxy, hexoxy, isohexoxy, heptoxy, isoheptoxy, octoxy or isooctoxy, The terms "alkoxy" and "alkyloxy" are used synonymously.
Alkoxyalkyl in general represents an alkyl radical having up to 8 carbon atoms, which is substituted by an alkoxy radical having up to 8 carbon atoms.
Alkoxycarbonyl can be represented, for example, by the formula

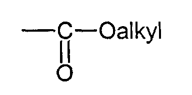

Alkyl here in general represents a straight-chain or branched hydrocarbon radical having 1 to 13 carbon atoms. Examples which may be mentioned are the following alkoxycarbonyl radicals: methoxycarbonyl, ethoxycarbonyl, propoxycarbonyl, isopropoxycarbonyl, butoxycarbonyl or isobutoxycarbonyl.
Cycloalkyl in general represents a cyclic hydrocarbon radical having 3 to 8 carbon atoms. Cyclopropyl, cyclopentyl and cyclohexyl are preferred. Examples which may be mentioned are cyclopentyl, cyclohexyl, cycloheptyl and cyclooctyl.
Cycloalkoxy in the context of the invention represents an alkoxy radical whose hydrocarbon radical is a cycloalkyl radical. The cycloalkyl radical in general has up to 8 carbon atoms. Examples which may be mentioned are: cyclopropyloxy and cyclohexyloxy. The terms "cycloalkoxy" and "cycloalkyloxy" are used synonymously.
Aryl in general represents an aromatic radical having 6 to 10 carbon atoms. Preferred aryl radicals are phenyl, benzyl and naphthyl.
Halogen in the context of the invention represents fluorine, chlorine, bromine and iodine.
Heterocycle in the context of the invention in general represents a saturated, unsaturated or aromatic 3- to 10-membered, for example 5- or 6-membered, heterocycle which can contain up to 3 heteroatoms from the group consisting of S, N and/or O and which, in the case of a nitrogen atom, can also be bonded via this. Examples which may be mentioned are: oxadiazolyl, thiadiazolyl, pyrazolyl, pyridyl, pyrimidinyl, pyridazinyl, pyrazinyl, thienyl, furyl, pyrrolyl, pyrrolidinyl, piperazinyl, tetrahydropyranyl, tetrahydrofuranyl, 1,2,3-triazolyl, thiazolyl, oxazolyl, imidazolyl, morpholinyl or piperidyl. Thiazolyl, furyl, oxazolyl, pyrazolyl, triazolyl, pyridyl, pyrimidinyl, pyridazinyl and tetrahydropyranyl are preferred. The term "heteroaryl" (or "hetaryl") represents an aromatic heterocyclic radical.

The conjugates according to the invention are characterized in that a cytotoxic radical or a radical of a cytostatic or of a cytostatic derivative is bonded via a linking unit to a non-peptide moiety addressing αᵥβ₃ integrin receptors.

The non-peptide moiety of the conjugate addressing αᵥβ₃ integrin receptors serves to bring the toxophoric part of the conjugate into or into the vicinity of tumour cells and thus to achieve tissue selectivity. Growing tumour tissue stimulates the formation of new blood vessels, i.e. angiogenesis, to a considerable extent in order to cover its increasing nutritional need. The blood vessels newly formed by angiogenesis differ from conventional tissue by specific markers on the surfaces of the endothelial cells formed. Moreover, the αᵥβ₃ integrin receptor is expressed by many human tumours (cf. WO 98/10795 and the references indicated there). Thus the conjugate is brought selectively into or into the vicinity of the tumour tissue to be treated by the interaction of its non-peptide part addressing αᵥβ₃ integrin receptors with αᵥβ₃ integrin receptors found on endothelial cells or on tumour cells formed by angiogenesis.

Unlike peptide radicals addressing αᵥβ₃ integrin receptors (such as disclosed, for example, in WO 98/10795), the non-peptide moieties according to the invention addressing αᵥβ₃ integrin receptors are distinguished by an increased serum stability, whereby the transport of the toxophore in the conjugate to the tumour tissue is ensured to an increased extent.

The abovementioned compounds having antagonistic action against αᵥβ₃ integrin receptors must be able to retain their property of addressing αᵥβ₃ integrin receptors in the conjugate. This means that these compounds must be linked to a toxophore in such a way that no or only a slight impairment of the abovementioned action of the compounds results thereby. In the normal case, the linkage with the linking unit will take place via a functional group suitable for this in the molecule, for example via an amino, hydroxyl or carboxyl function. If the abovementioned compounds have no functional group, one of these is easily insertable into the molecule by conventional processes known to the person in the art without the loss of the antagonistic action against αᵥβ₃ integrin receptors occurring here.

The conjugate according to the invention can release its toxophoric radical at its target site and this can thus make possible penetration into the tumour tissue. This is carried out by the specific choice of a unit linking the toxophoric radical to the moiety addressing αᵥβ₃ integrin receptors. The linking unit of the conjugates of the present invention is designed such that it can be cleaved by tumor-associated enzymes such as matrix metalloproteases (MMPs).

A further suitable starting point for promoting the tissue selectivity of the action of the conjugates according to the invention consists in the so-called ADEPT approach. In this, conjugates are cleaved by certain enzymes. These enzymes are introduced into the body coupled to antibodies together with the conjugates according to the invention, the antibodies serving as vehicles specifically addressing tumour tissue. This leads to a selective concentration both of the conjugate and of the enzyme/antibody system in the tumour tissue, whereby the toxophore is released in the tumour tissue with even greater selectivity and can display its action there.

Suitable linking units according to the invention are all linking units which fulfil the abovementioned criteria and can be linked to the moiety addressing αᵥβ₃ integrin receptors in such a way that this retains its binding action to αᵥβ₃ integrin receptors.

In the conjugates according to the invention, toxophores used can be all cytotoxic radicals or radicals of a cytostatic or of a cytostatic derivative which are conventionally employed in tumour therapy.

According to a preferred embodiment, conjugates according to the invention which can be employed are compounds of the formula (I) in which a toxophore is linked via a linking unit consisting of 5 to 8 amino acids, preferably 5 to 7 amino acids and particularly preferably 6 amino acids, and, if appropriate, of a non-peptide spacer group, to a non-peptide moiety addressing αᵥβ₃ integrin receptors from the group of radicals of the formulae (II) to (IV): 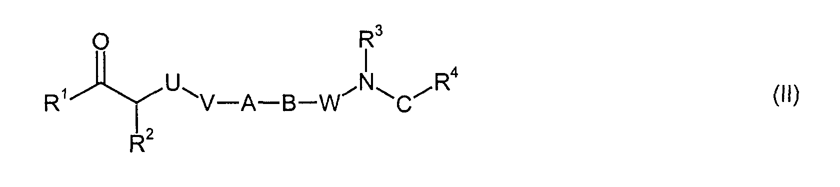 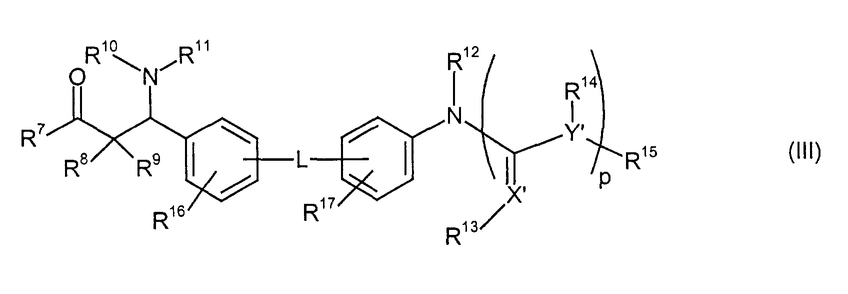 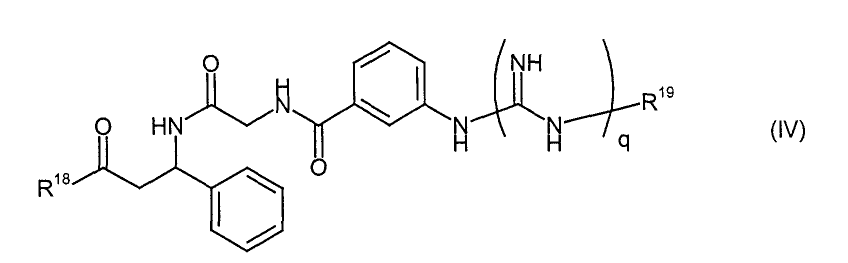 where the radicals in the formulae (II) to (IV) have the meanings indicated above.

In the conjugates of the formula (I) according to the invention, the toxophore used can be cytostatic radicals or radicals of a cytostatic or of a cytostatic derivative which are conventionally employed in tumour therapy. Camptothecin or derivatives of camptothecin such as 9-aminocamptothecin are preferred here, which can be linked to the rest of the conjugate via the C20-OH group or via a functional group which is optionally present in the molecule, such as the amino group in the case of 9-aminocamptothecin. According to this preferred embodiment, the camptothecin unit used as a starting compound can be present in the 20(R) or in the 20(S) configuration or as a mixture of these two stereoisomeric forms. The 20(S) configuration is preferred.

In the conjugates of the formula (I), the linking unit preferably consists of a unit of the formula

-LI-Sp-

wherein the unit LI preferably comprises amino acid residues

-AA1-AA2-AA3-AA4-AA5-AA6-AA7-AA8-

wherein AA1 is bonded to the radical CT, i.e. to the toxophor, and the radical AA8 - or the corresponding radical AA7, AA6 and so on constituting the termination of the linking unit at this end if AA8 is absent - is bonded to the spacer unit Sp.

According to a preferred embodiment of the present invention, at least 5 of the radicals AA1 to AA8 are present. Particularly preferred is a linking unit wherein 5 to 8 of the radicals AA1 to AA8 are present. Especially preferred is a linking unit wherein 5 to 7 of the radicals AA1 to AA8 are present.According to the most preffered embodiment of the present invention the linking unit comprises 6 of the radicals AA1 to AA8.

The radicals AA1 to AA8, if they are present, each represent an amino acid in the D or L configuration. In this context, they are particularly preferably one of the naturally occurring amino acids glycine, alanine, valine, leucine, isoleucine, serine, threonine, cysteine, methionine, aspartate, glutamate, asparagine, glutamine, arginine, lysine, histidine, tryptophan, phenylalanine, tyrosine or proline. The amino acids used in the process according to the invention can occur in the L or in the D configuration or alternatively as a mixture of D and L form.

The term "amino acids" refers, according to the invention, in particular to the α-amino acids occurring in nature, but moreover also includes their homologues, isomers and derivatives. An example of isomers which can be mentioned is enantiomers. Derivatives can be, for example, amino acids provided with protective groups.

According to the present invention, the amino acids can each be linked to one another and to the toxophore or to the moiety addressing αᵥβ₃ integrin receptors via their α-carboxyl or α-amino functions, but also via functional groups optionally present in side chains, such as, for example, amino functions.

In the case of amino acids having functional groups in the side chains, these functional groups can be either deblocked or protected by conventional protective groups used in peptide chemistry. Protective groups employed for these functional groups of the amino acids can be the protective groups known in peptide chemistry, for example of the urethane, alkyl, acyl, ester or amide type.

Amino protective groups in the context of the invention are the customary amino protective groups used in peptide chemistry. These preferably include: benzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 4-nitrobenzyloxycarbonyl, 2-nitrobenzyloxycarbonyl, 2-nitro-4,5-dimethoxybenzyloxycarbonyl, methoxycarbonyl, ethoxycarbonyl, tert-butoxycarbonyl (Boc), allyloxycarbonyl, vinyloxycarbonyl, 3,4,5-trimethoxybenzyloxycarbonyl, phthaloyl, 2,2,2-trichloroethoxycarbonyl, 2,2,2-trichloro-tert-butoxycarbonyl, menthyloxycarbonyl, 4-nitrophenoxycarbonyl, fluorenyl-9-methoxycarbonyl (Fmoc), formyl, acetyl, propionyl, pivaloyl, 2-chloroacetyl, 2-bromoacetyl, 2,2,2-trifluoroacetyl, 2,2,2-trichloroacetyl, benzoyl, benzyl, 4-chlorobenzoyl, 4-bromobenzoyl, 4-nitrobenzoyl, phthalimido, isovaleroyl or benzyloxymethylene, 4-nitrobenzyl, 2,4-dinitrobenzyl, 4-nitrophenyl or 2-nitrophenylsulphenyl. The Fmoc group and the Boc group are particularly preferred.

The removal of protective groups in appropriate reaction steps can be carried out, for example, by the action of acid or base, hydrogenolytically or reductively in another manner.

According to the present invention, AA1 preferably is a naturally occurring amino acid in the D or L configuration, which is selected from the group consisting of glycine, alanine, valine, leucine, isoleucine, histidine, glutamate, aspartate, serine, lysine, ornithine and phenylalanine. According to the present invention, AA1 most preferably is valine or glycine.

According to the present invention, AA2 preferably is absent or is a naturally occurring amino acid in the D or L configuration, which is selected from the group consisting of alanine, valine, phenylalanine, tyrosine, threonine, serine, isoleucine, lysine, glutamate, histidine, glycine, arginine, asparagine, glutamine, S-methyl-cysteine, methionine, arginine, aspartate, tryptophane, proline, ornithine and leucine. According to the present invention, AA2 most preferably is a naturally occurring amino acid in the D or L configuration, which is selected from the group consisting of phenylalanine, histidine, asparagine.

According to the present invention, AA3 preferably is absent or is a naturally occurring amino acid in the D or L configuration, which is selected from the group consisting of alanine, valine, phenylalanine, tyrosine, serine, isoleucine, lysine, glutamate, histidine, glycine, arginine, aspartate, tryptophane, proline, ornithine, methionine, S-methyl-cysteine, norvaline and leucine. According to the present invention, AA3 most preferably is a naturally occurring amino acid in the L configuration, which is selected from the group consisting of leucine, norvaline and S-methylcysteine.

According to the present invention, AA4 preferably is absent or is a naturally occurring amino acid in the D or L configuration, which is selected from the group consisting of glycine, alanine, valine, leucine, isoleucine, cysteine and norvaline.

According to the present invention, AA4 most preferably is a naturally occurring amino acid in the L configuration, which is selected from the group consisting of glycine, and alanine.

According to the present invention, AA5 preferably is absent or is a naturally occurring amino acid in the D or L configuration, which is selected from the group consisting of glycine, alanine, valine, leucine, isoleucine, histidine, tyrosine, glutamine, asparagine, proline, methionine, phenylalanine and cysteine. According to the present invention, AA5 most preferably is a naturally occurring amino acid in the L-configuration, which is selected from the group consisting of glycine, leucine and glutamine.

According to the present invention, AA6 preferably is absent or is a naturally occurring amino acid in the D or L configuration, which is selected from the group consisting of glycine, alanine, valine, leucine, isoleucine, histidine, glutamine, asparagine, aspartate and proline. According to the present invention, AA6 most preferably is a naturally occurring amino acid in the D or L configuration, which is selected from the group consisting of proline and leucine.

According to the present invention, AA7 preferably is absent or is a naturally occurring amino acid in the D or L configuration, which is selected from the group consisting of glycine, alanine, valine, leucine, isoleucine, histidine, γ-aminobutyric acid, aspartate, glutamate, lysine and proline. According to the present invention, AA7 most preferably is absent or is a naturally occurring amino acid in the D or L configuration, which is selected from the group consisting of glycine, histidine, γ-aminobutyric acid and proline.

According to the present invention, AA8 preferably is absent or is a naturally occurring amino acid in the D or L configuration, which is selected from the group consisting of glycine, alanine, valine, leucine, isoleucine, histidine, lysine, proline and γ-aminobutyric acid. According to the present invention, AA8 most preferably is absent or is a naturally occurring amino acid in the D or L configuration, which is selected from the group consisting of glycine, histidine and γ-aminobutyric acid .

The linking unit LI of the conjugates according to the present invention is designed such that it can selectively be cleaved by tumor-associated enzymes, i.e. enzymes which occur in the tumor tissue and preferably cannot be found in normal tissue or are present in normal tissue only in a significant lower amount as compared with their presence in tumor tissue. As an exampleof a family of tumor-associated enzymes which selectively cleave the linking unit LI of the conjugates according to the present invention may be mentioned matrix metalloproteases (MMPs). According to a particularly preferred embodiment the linking unit LI of the conjugates according to the present invention is designed such that it can selectively be cleaved by certain members of such families of enzymes. Especially preferred the linking unit LI of the conjugates according to the present invention can selectively be cleaved by MMP-2 or MMP-9.

It is preferred according to the invention that the linking unit consists of six to seven amino acids AA1 to AA8 and the spacer unit Sp, it being possible, in particular, for the unit AA2 to be modified on the side chain by protective groups. However, it is also possible for the linking unit to consist of five or eight amino acids AA1 to AA8 and a spacer unit Sp. In these cases, the linkage to the toxophore as a rule takes place via the carboxyl function of the amino acid AA1 and the linkage to the moiety addressing αᵥβ₃ integrin receptors via the spacer unit Sp takes place using an amino group or hydroxyl group of the moiety addressing αᵥβ₃ integrin receptors.

In the case in which the linkage is to take place via a carboxyl function of the moiety addressing αᵥβ₃ integrin receptors, it is preferred, however, to use linking units without the spacer unit Sp. In this case, the linkage between the linking unit and the moiety addressing αᵥβ₃ integrin receptors takes place via an amino function of an amino acid. The moiety addressing αᵥβ₃ integrin receptors can be, for example, a radical of the formula (II): 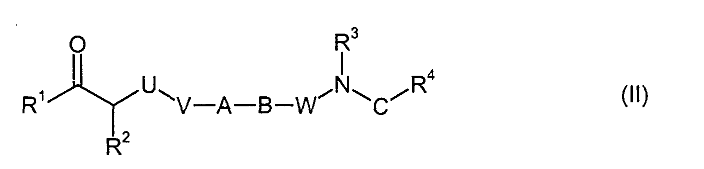 where the radicals in the formula (II) have the meaning defined above.

In the description below, bivalent substituents are indicated such that their respective left end is connected to the group indicated left of the corresponding substituent in formula (II) and their respective right end is connected to the group indicated right of the corresponding substituent in formula (II). If, for example, in formula (II) the radical V is equal to -NR²⁰SO₂-, the nitrogen atom is connected to the radical U and the sulphur atom to the radical A. The following embodiments additionally relate to the radical of the formula (II) in the unlinked state. The linkage of the radical of the formula (II) to the toxophore via the linking unit can take place either via the terminal carboxyl group, the terminal amino group, amide group, urea group, thioamide group, thiourea group, amidine group or guanidine group or via a functional group in the side chain of the radical of the formula (II), i.e. via the radical R² or a substituent on the group U or V, whereby in the linked state the terminal carboxyl group or the terminal amino group, amide group, urea group, thioamide group, thiourea group, amidine group or guanidine group is converted into corresponding bridging units.

The radicals of the formula (II) according to the invention are characterized in that they have, as a main structural element, a biphenyl nucleus which bridges a radical having a terminal carboxyl group with a radical including at least one nitrogen atom in the main chain, which is a constituent of an amino group, amide group, urea group, thioamide group, thiourea group, amidine group or guanidine group optionally incorporated into a cyclic ring system. The biphenyl nucleus can moreover carry further substituents in addition to the abovementioned radicals.

The terminal carboxyl unit, if the bonding of the radical of the formula (II) does not take place via this, can be present as a free carboxylic acid or as an ester. In the case in which the terminal carboxyl unit is esterified, fundamentally all carboxylic acid esters obtainable by conventional processes, such as the corresponding alkyl esters, cycloalkyl esters, aryl esters and hetereocyclic analogues thereof, can be used according to the invention, alkyl esters, cycloalkyl esters and aryl esters being preferred and it being possible for the alcoholic radical to carry further substituents. C₁₋₆-Alkyl esters such as the methyl ester, ethyl ester, propyl ester, isopropyl ester, butyl ester, isobutyl ester, t-butyl ester, pentyl ester, isopentyl ester, neopentyl ester, hexyl ester, cyclopropyl ester, cyclopropylmethyl ester, cyclobutyl ester, cyclopentyl ester, cyclohexyl ester, or aryl esters such as the phenyl ester, benzyl ester or tolyl ester are particularly preferred.

Preferably, however, the radicals of the formula (II) according to the invention are used in a form in which the terminal carboxyl unit is present as a free carboxylic acid.

The terminal carboxyl unit is connected to the biphenyl nucleus via an alkylene chain which can optionally carry further substituents. Within certain limits, it is possible to control the biological activity of the radicals of the formula (II) according to the invention against integrin receptors such as, in particular, the αᵥβ₃ receptor, by means of the distance between the terminal carboxyl unit and the nitrogen atom of an amino group, amide group, urea group, thioamide group, thiourea group, amidine group or guanidine group which is found in the main chain of the radical linked to the phenyl ring B of the biphenyl nucleus, it being possible in the case in which more than one nitrogen atom is present in the main chain of the corresponding radical for the nitrogen atom found near to the phenyl ring B of the biphenyl nucleus to be decisive. In addition to the biphenyl nucleus, preferably not more than 6 atoms should be found in the main chain between these two structural elements. More preferred, however, are radicals of the formula (II), in which, additionally to the biphenyl nucleus in the main chain between the terminal carboxyl unit and the nitrogen atom of the amino group, amide group, urea group, thioamide group, thiourea group, amidine group or guanidine group which is found in the main chain of the radical linked to the phenyl ring B of the biphenyl nucleus, less than 6 additional atoms are found. According to the present invention, radicals of the formula (II) are particularly preferred in which the abovementioned nitrogen atom of the amino group, amide group, urea group, thioamide group, thiourea group, amidine group or guanidine group is bonded directly to the phenyl ring B of the biphenyl nucleus and at the same time the terminal carboxyl unit is separated from the phenyl ring A of the biphenyl nucleus by two to four atoms in the main chain.

The alkylene chain which connects the terminal carboxyl group to the phenyl ring A of the biphenyl nucleus can alternatively carry additional substituents of any of the carbon atoms forming the alkylene chain. These substituents can be selected from the group which consists of hydrogen, a substituted or unsubstituted alkyl or cycloalkyl radical, a substituted or unsubstituted aryl radical, a saturated or unsaturated, optionally substituted heterocyclic radical, an optionally substituted alkenyl radical, an optionally substituted alkinyl radical, -NR²'₂, -NR²'SO₂R²", -NR²'COOR²", -NR²'COR²', -NR²'CONR²'₂ or -NR²'CSNR²'₂, where R²' can be hydrogen, a substituted or unsubstituted alkyl or cycloalkyl radical, a substituted or unsubstituted aryl radical or a saturated or unsaturated, optionally substituted heterocyclic radical and R²" can be a substituted or unsubstituted alkyl or cycloalkyl radical, a substituted or unsubstituted aryl radical or a saturated or unsaturated, optionally substituted heterocyclic radical. The alkyl radical can preferably be a C₁₋₆-alkyl such as, for example, methyl, ethyl, propyl, isopropyl, butyl, isobutyl, t-butyl, pentyl, isopentyl, neopentyl or hexyl. The cycloalkyl radical can preferably be a C₃₋₇-cycloalkyl such as, for example, cyclopropyl, cyclopropylmethyl, cyclobutyl, cyclopentyl or cyclohexyl. The aryl radical can preferably be phenyl, benzyl or tolyl. The heterocyclic radical can preferably be pyrrole, pyridine, tetrahydrofuran, furan, thiophene, tetrahydrothiophene, oxathiazole, benzofuran, quinoline, isoquinoline or pyrimidine. The alkenyl radical can be a terminal or internal E- or Z-alkene unit. The abovementioned radicals can alternatively be substituted by one or more C₁₋₆-alkyl radicals such as methyl, ethyl, propyl, isopropyl, butyl, isobutyl, t-butyl, pentyl, isopentyl, neopentyl or hexyl, C₃₋₇-cycloalkyl radicals such as cyclopropyl, cyclopropylmethyl, cyclobutyl, cyclopentyl or cyclohexyl, aryl radicals such as phenyl, benzyl, tolyl, naphthyl, heterocyclic radicals such as pyrrole, pyridine, tetrahydrofuran, furan, thiophene, tetrahydrothiophene, oxazole, thiazole, oxathiazole, benzofuran, benzoxazole, benzothiazole, quinoline, isoquinoline, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, benzofuranyl, benzoxazolyl, benzothiazolyl, quinolinyl or isoquinolinyl unit or a partially or completely hydrogenated analogue thereof. -NR²'₂, -NR²'SO₂R²", -NR²'COOR²", -NR²'COR²', -NR²'CONR²'₂ or -NR²'CSNR²'₂ are preferred among the substituents optionally found on the alkylene chain connecting the terminal carboxyl group to the phenyl ring A of the biphenyl nucleus, where R²' can be hydrogen, a substituted or unsubstituted alkyl or cycloalkyl radical, a substituted or unsubstituted aryl radical or a saturated or unsaturated, optionally substituted heterocyclic radical and R²" can be a substituted or unsubstituted alkyl or cycloalkyl radical, a substituted or unsubstituted aryl radical or a saturated or unsaturated, optionally substituted heterocyclic radical. Preferably, R²' is selected from the group which consists of hydrogen, a C₁₋₆-alkyl such as, for example, methyl, ethyl, propyl, isopropyl, butyl, isobutyl, t-butyl, pentyl, isopentyl, neopentyl, hexyl, a C₃₋₇-cycloalkyl such as, for example, cyclopropyl, cyclobutyl, cyclopentyl, cyclohexyl, phenyl, benzyl, tolyl or a substituted derivative thereof, while R²" is preferably selected from the group which consists of a C₁₋₆-alkyl such as, for example, methyl, ethyl, propyl, isopropyl, butyl, isobutyl, t-butyl, pentyl, isopentyl, neopentyl, hexyl, a C₃₋₇-cydoalkyl such as, for example, cyclopropyl, cyclobutyl, cyclopentyl, cyclohexyl, phenyl, benzyl, tolyl or a substituted derivative thereof, -C₆H₂(CH₃)₃, 2-chlorophenyl, 4-chlorophenyl, 2,5-dichlorophenyl, 4-trifluoromethylphenyl, camphor-10-yl, 4-methoxyphenyl, 4-t-butylphenyl, 2,5-dimethylphenyl, 3-chlorophenyl, 2-methoxy-5-methylphenyl, 2,3,5,6-tetramethylphenyl, 2,3-dichlorophenyl, 2,6-dichlorophenyl, 2-naphthyl, 3-trifluoromethylphenyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-aminophenyl, 4-aminophenyl, 3-(N-acetyl-6-methoxy)aniline or 8-quinolinyl.

According to the invention, particularly preferred radicals of the formula (II) are those in which an amide, urea, sulphonamide or carbamate group is found in the alkylene chain which connects the terminal carboxyl group to the phenyl ring A of the biphenyl nucleus. Preferably, the amide, urea, sulphonamide or carbamate group is found in the α- or β-position to the terminal carboxyl group. However, there can also be more than 2 carbon atoms between the carboxyl carbon of the terminal carboxyl group and the nitrogen atom of the sulphonamide or carbamate unit. According to the present invention, the sulphonamide group, if present, particularly preferably carries a radical R²" on the sulphur atom, which is selected from the group consisting of phenyl, benzyl, tolyl or a substituted derivative thereof, -C₆H₂(CH₃)₃, 2-chlorophenyl, 4-chlorophenyl, 2,5-dichlorophenyl, 4-trifluoromethylphenyl, 3-aminophenyl, 4-aminophenyl, camphor-10-yl, 4-methoxyphenyl, 4-t-butylphenyl, 2,5-dimethylphenyl, 3-chlorophenyl, 2-methoxy-5-methylphenyl, 2,3,5,6-tetramethylphenyl, 2,3-dichlorophenyl, 2,6-dichlorophenyl, 2-naphthyl, 3-trifluoromethylphenyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 3-aminobenzyl, 4-aminobenzyl, 2-trifluoromethylphenyl, 2-alkylsulphonylphenyl, 2-arylsulphonylphenyl, 3-(N-acetyl-6-methoxy)aniline or 8-quinolinyl. The carbamate group, if present, particularly preferably carries a radical R²" as an alcoholic portion, which is selected from the group consisting of phenyl, benzyl, tolyl or a substituted derivative thereof, and particularly preferably a benzyl radical, 3-aminobenzyl or 4-aminobenzyl.

According to a further embodiment, the present invention relates to radicals of the formula (II), in which the terminal carboxyl group is bonded to the phenyl ring A of the biphenyl nucleus via an alkylenesulphonamide unit or an alkyleneamide unit, i.e. an -NRSO₂- or -NR-CO group is inserted between the alkylene chain and the phenyl ring A of the biphenyl nucleus, the phenyl ring A of the biphenyl nucleus being bonded to the sulphur atom of the sulphonamide unit or the carboxyl carbon atom of the amide unit. The alkylene chain between the terminal carboxyl group and the sulphonamide or amide unit can in this case optionally carry further substituents in accordance with the above details, where a C₁₋₆-alkyl such as, for example, methyl, ethyl, propyl, isopropyl, butyl, isobutyl, t-butyl, pentyl, isopentyl, neopentyl or hexyl, a C₃₋₇-cycloalkyl such as, for example, cyclopropyl, cyclopropylmethyl, cyclobutyl, cyclopentyl or cyclohexyl, an aryl such as, for example, phenyl, benzyl, phenylethyl or tolyl, a heterocyclic radical such as pyrrole, pyridine, tetrahydrofuran, furan, thiophene, tetrahydrothiophene, oxathiazole, benzofuran, quinoline, isoquinoline or pyrimidine, or a terminal or internal E- or Z-alkene unit is preferred, which can alternatively be substituted by one or more C₁₋₆-alkyl radicals such as methyl, ethyl, propyl, isopropyl, butyl, isobutyl, t-butyl, pentyl, isopentyl, neopentyl or hexyl, C₃₋₇-cycloalkyl radicals such as cyclopropyl, cyclopropylmethyl, cyclobutyl, cyclopentyl or cyclohexyl, aryl radicals such as phenyl, benzyl, tolyl, naphthyl, heterocyclic radicals such as pyrrole, pyridine, tetrahydrofuran, furan, thiophene, tetrahydrothiophene, oxathiazole, benzofuran, quinoline, isoquinoline or pyrimidine, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, benzofuranyl, benzoxazolyl, benzothiazolyl, quinolinyl or isoquinolinyl unit or a partially or completely hydrogenated analogue thereof.

Particularly preferred radicals of the formula (II) according to this embodiment are those in which the alkylene chain which connects the terminal carboxyl group and the bridging sulphonamide or amide unit has an optionally substituted phenyl or benzyl radical such as, for example, β-3-aminophenyl, β-4-aminophenyl or α-4-aminobenzyl in the α- or β-position relative to the terminal carboxyl unit.

In the radicals of the formula (II) of this embodiment, in which a sulphonamide or amide unit is inserted between the corresponding alkylene chain and the phenyl ring A of the biphenyl nucleus, the alkylene chain between the terminal carboxyl group and the bridging sulphonamide or amide unit should preferably include not more than two carbon atoms in this main chain in order that, as mentioned above, in addition to the biphenyl nucleus preferably not more than five atoms are present between the terminal carboxyl group and the nitrogen atom of the amino group, amide group, urea group, thioamide group, thiourea group, amidine group or guanidine group which is next to the phenyl ring B in the main chain of the radical linked to the phenyl ring B of the biphenyl nucleus.

The nitrogen atom of the bridging sulphonamide or amide unit can optionally be substituted by a radical which is selected from the group consisting of hydrogen, a C₁₋₆-alkyl such as, for example, methyl, ethyl, propyl, isopropyl, butyl, isobutyl, t-butyl, pentyl, isopentyl, neopentyl, hexyl, a C₃₋₇-cycloalkyl such as, for example, cyclopropyl, cyclopropylmethyl, cyclobutyl, cyclopentyl, cyclohexyl, an aryl such as, for example, phenyl, benzyl, tolyl or a substituted derivative thereof such as, for example, phenylethyl, phenylpropyl or phenoxyethyl.

The biphenyl nucleus is the central structural element of the radicals of the formula (II) according to the invention. In the unlinked state, it bridges the radical including the terminal carboxyl group on the phenyl ring A to the radical on the phenyl ring B which includes at least one nitrogen atom of an amino group, amide group, urea group, thioamide group, thiourea group, amidine group or guanidine group in its main chain. Preferably, it moreover carries no further substituents. Each of the two phenyl rings, however, can carry additional substituents. Preferably, the phenyl ring A, i.e. the ring connected to the radical including the terminal carboxyl group, carries one or more additional alkoxy radicals, preferably a C₁₋₆-alkoxy radical such as methoxy, ethoxy, propoxy, butoxy, pentoxy or hexoxy, particularly preferably one or more methoxy radicals, and the phenyl ring B, i.e. the ring to which the radical including at least one nitrogen atom of an amino group, amide group, urea group, thioamide group, thiourea group, amidine group or guanidine group is bonded in its main chain, carries one or more alkyl radicals, preferably a C₁₋₆-alkyl radical such as methyl, ethyl, propyl, isopropyl, butyl, isobutyl, t-butyl, pentyl, isopentyl, neopentyl, hexyl, a C₃₋₇-cycloalkyl radical such as cyclopropyl, cyclobutyl, cyclopentyl, cyclohexyl, and particularly preferably one or more methyl groups. In this case, the phenyl rings A and B can independently of one another carry one or more of the abovementioned additional substituents.

The two phenyl rings can be 1,3- or 1,4-linked to one another and to the radical including the terminal carboxyl group and the radical including at least one nitrogen atom of an amino group, amide group, urea group, thioamide group, thiourea group, amidine group or guanidine group in its main chain, i.e. the radical including the terminal carboxyl group and the phenyl ring B can be substituted in the meta- or para-position relative to one another in the phenyl ring A, and at the same time the phenyl ring A and the radical including at least one nitrogen atom of an amino group, amide group, urea group, thioamide group, thiourea group, amidine group or guanidine group in its main chain can be substituted in the meta- or para-position on the phenyl ring B relative to one another, each combination of the abovementioned substitution patterns being possible for the biphenyl nucleus of the radicals of the formula (II) according to the invention. According to the present invention, particularly preferred radicals of the formula (II) are those whose biphenyl nucleus consists according to the above definition of a p-substituted phenyl ring A and a p-substituted phenyl ring B, a p-substituted phenyl ring A and an m-substituted phenyl ring B, an m-substituted phenyl ring A and a p-substituted phenyl ring B, or an m-substituted phenyl ring A and an m-substituted phenyl ring B. According to the present invention, particularly preferred radicals of the formula (II) are those whose biphenyl nucleus consists according to the present definition of a p-substituted phenyl ring A and an m-substituted phenyl ring B.

As a third structural element, the radicals of the formula (II) according to the invention in the unlinked state contain, in addition to the biphenyl nucleus and the radical including a terminal carboxyl group, a group which in its main chain comprises at least one nitrogen atom of an amino group, amide group, urea group, thioamide group, thiourea group, amidine group or guanidine group. This nitrogen atom can be bonded to the phenyl ring B of the biphenyl nucleus directly or via an alkylene chain. This alkylene chain preferably consists of at most 4 carbon atoms in the main chain, where, from the abovementioned considerations, not more than 6 further atoms should be present in addition to the biphenyl nucleus between the terminal carboxyl group and the nitrogen atom of the amino group, amide group, urea group, thioamide group, thiourea group, amidine group or guanidine group which is next to the phenyl ring B. Alternatively, this alkylene chain can carry further substituents which are selected from the group consisting of hydrogen, a C₁₋₆-alkyl such as, for example, methyl, ethyl, propyl, isopropyl, butyl, isobutyl, t-butyl, pentyl, isopentyl, neopentyl or hexyl, a C₃₋₇-cycloalkyl such as, for example, cyclopropyl, cyclopropylmethyl, cyclobutyl, cyclopentyl or cyclohexyl, an aryl such as, for example, phenyl, benzyl or tolyl, a heterocyclic radical such as pyrrole, pyridine, tetrahydrofuran, furan, thiophene, tetrahydrothiophene, oxathiazole, benzofuran, quinoline, isoquinoline or pyrimidine, or a terminal or internal E- or Z-alkene unit, and can alternatively be substituted by one or more C₁₋₆-alkyl radicals such as methyl, ethyl, propyl, isopropyl, butyl, isobutyl, t-butyl, pentyl, isopentyl, neopentyl or hexyl, cycloalkyl radicals such as cyclopropyl, cyclopropylmethyl, cyclobutyl, cyclopentyl or cyclohexyl, aryl radicals such as phenyl, benzyl, tolyl, naphthyl, indolyl, heterocyclic radicals such as pyrrole, pyridine, tetrahydrofuran, furan, thiophene, tetrahydrothiophene, oxathiazole, benzofuran, quinoline, isoquinoline or pyrimidine, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, indolyl, benzofuranyl, benzoxazolyl, benzothiazolyl, benzimidazolyl, quinolinyl or isoquinolinyl unit or a partially or completely hydrogenated analogue thereof.

The nitrogen atom found in the main chain of the radical bonded to the phenyl ring B of the biphenyl nucleus, which is next to the phenyl ring B, can, if the bonding of the radical of the formula (II) does not take place via this, either be a constituent of an optionally substituted amino group or be in the direct vicinity of a -C=O unit, -CONR₂ unit, -C=S unit, -CSNR₂ unit, -C=NR unit or a -CNRNR₂ unit and thus be a constituent of an amide group, urea group, thioamide group, thiourea group, amidine group or guanidine group.

In the case in which the nitrogen atom found in the main chain of the radical bonded to the phenyl ring B of the biphenyl nucleus, which is next to the phenyl ring B, is a constituent of an amino group, it can be unsubstituted or can carry one or two substituents, i.e. be a constituent of a primary, secondary or tertiary amino group. These substituents can be independent of one another or, simultaneously, hydrogen, a substituted or unsubstituted alkyl or cycloalkyl radical, a substituted or unsubstituted aryl radical, a saturated or unsaturated, optionally substituted heterocyclic radical, an alkylamine radical, an alkylamide radical or can be connected to one another and thus form, together with the nitrogen atom to which they are bonded, a heterocyclic ring system. In this case, preferred substituents are those which are selected from the group consisting of hydrogen, a C₁₋₆-alkyl such as, for example, methyl, ethyl, propyl, isopropyl, butyl, isobutyl, t-butyl, pentyl, isopentyl, neopentyl or hexyl, a C₃₋₇-cycloalkyl such as, for example, cyclopropyl, cyclopropylmethyl, cyclobutyl, cyclopentyl or cyclohexyl, an aryl such as, for example, phenyl, benzyl or tolyl, a heterocyclic radical such as, for example, pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or a terminal or internal E- or Z-alkene unit, and can alternatively be substituted by one or more C₁₋₆-alkyl radicals such as methyl, ethyl, propyl, isopropyl, butyl, isobutyl, t-butyl, pentyl, isopentyl, neopentyl or hexyl, C₃₋₇-cycloalkyl radicals such as cyclopropyl, cyclopropylmethyl, cyclobutyl, cyclopentyl or cyclohexyl, aryl radicals such as phenyl, benzyl, tolyl, naphthyl, indolyl, heterocyclic radicals such as pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, indolyl, benzofuranyl, benzoxazolyl, benzothiazolyl, benzimidazolyl, quinolinyl or isoquinolinyl unit or a partially or completely hydrogenated analogue thereof. Particularly preferred substituents are those such as hydrogen, methyl, ethyl, propyl, isopropyl, butyl, isobutyl, t-butyl, pentyl, isopentyl, neopentyl, hexyl, cyclopropyl, cyclo-propyl-methyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl,

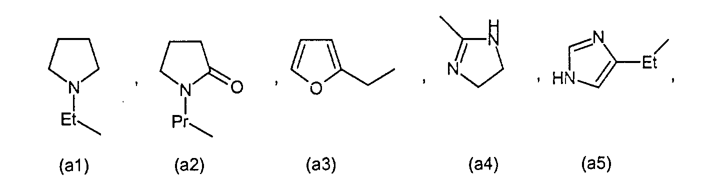

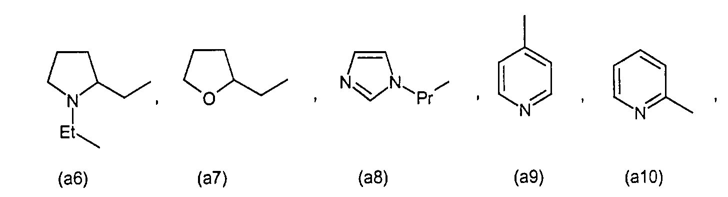

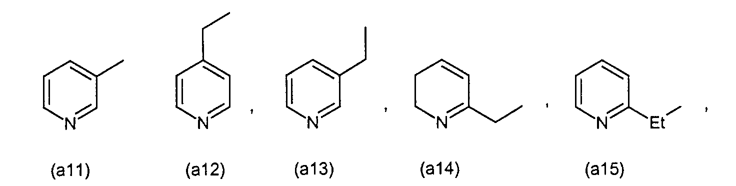

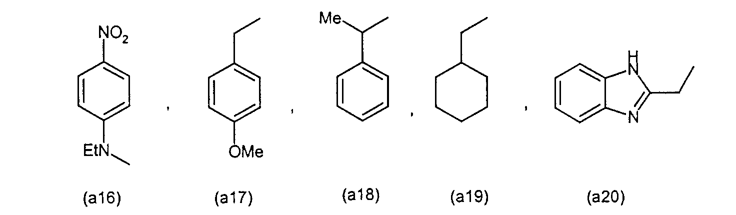

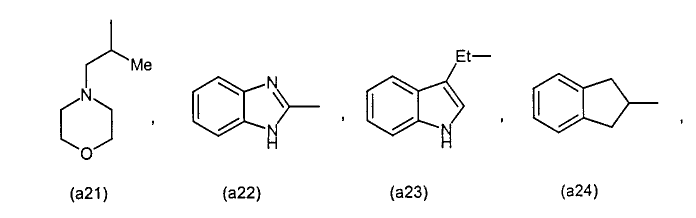

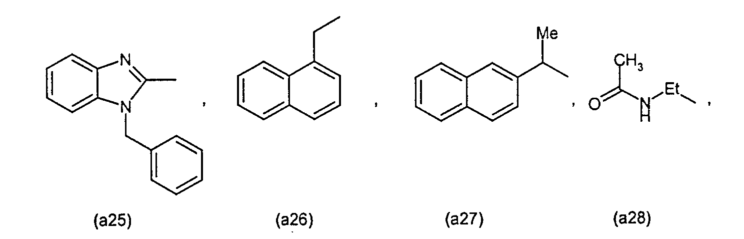

In the case in which the two substituents on the nitrogen atom which is next to the phenyl ring B are bonded to one another and thus form a heterocyclic system with the nitrogen atom, the heterocyclic system formed can be selected, for example, from the following, non-exclusive list: 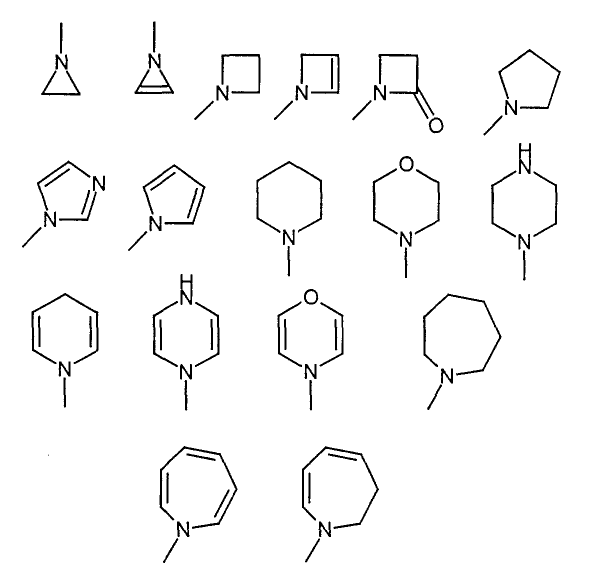 where the ring systems shown can carry one or more radicals which are selected from the group consisting of hydrogen, a C₁₋₆-alkyl such as, for example, methyl, ethyl, propyl, isopropyl, butyl, isobutyl, t-butyl, pentyl, isopentyl, neopentyl or hexyl, a C₃₋₇-cycloalkyl such as, for example, cyclopropyl, cyclopropylmethyl, cyclobutyl, cyclopentyl or cyclohexyl, an aryl such as, for example, phenyl, benzyl or tolyl, a heterocyclic radical such as, for example, pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or a terminal or internal E- or Z-alkene unit, and can alternatively be substituted by one or more C₁₋₆-alkyl radicals such as methyl, ethyl, propyl, isopropyl, butyl, isobutyl, t-butyl, pentyl, isopentyl, neopentyl or hexyl, C₃₋₇-cycloalkyl radicals such as cyclopropyl, cyclopropylmethyl, cyclobutyl, cyclopentyl or cyclohexyl, aryl radicals such as phenyl, benzyl, tolyl, naphthyl, indolyl, heterocyclic radicals such as pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or functional groups such as a double bond to a heteroatom such as oxygen, sulphur or nitrogen, an optionally substituted amino group, a nitro group or a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, indolyl, benzofuranyl, benzoxazolyl, benzothiazolyl, benzimidazolyl, quinolinyl or isoquinolinyl unit or a partially or completely hydrogenated analogue thereof.

Of the ring systems shown above, the four- to six-membered ring systems are preferred.

As mentioned above, the nitrogen atom in the main chain of the radical bonded to the phenyl ring B of the biphenyl nucleus, which is next to the phenyl ring B, can also be a constituent of one of the following preferred functional units: 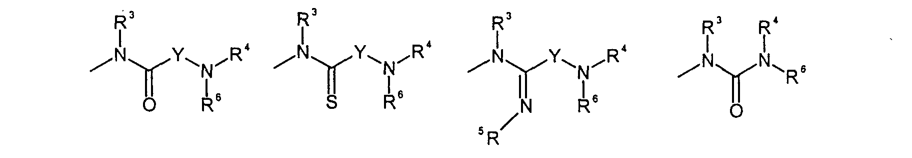 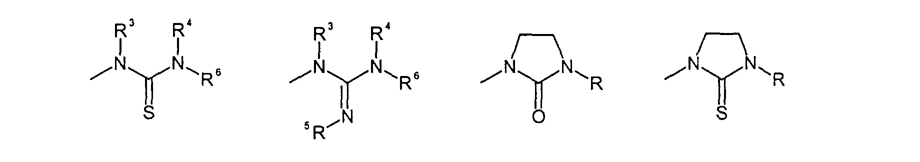 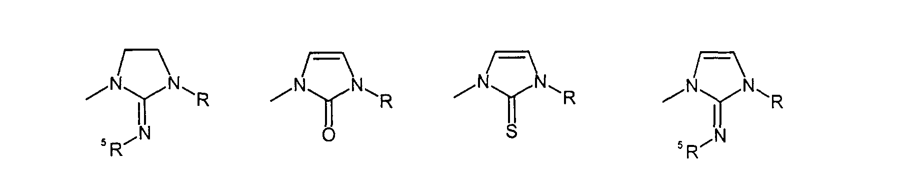 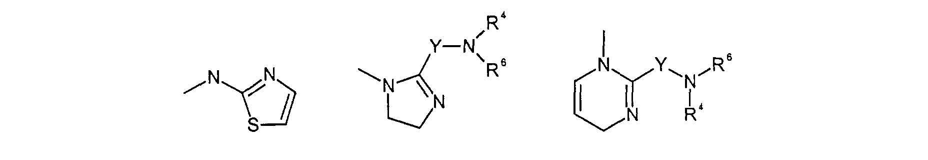 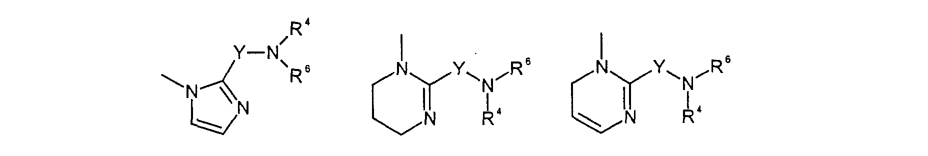 where the above list is not an exclusive enumeration of all possible structural units.

According to the invention, in addition to the abovementioned preferred structural units, their analogues are also included in which one or more 4- to 6-membered ring systems are fused to the heterocycle, such as, for example, the corresponding benzo-fused analogues of the above structural units.

In the structural units shown above, R³, R⁴ and R⁶ can each be hydrogen, a C₁₋₆-alkyl such as, for example, methyl, ethyl, propyl, isopropyl, butyl, isobutyl, t-butyl, pentyl, isopentyl, neopentyl or hexyl, a C₃₋₇-cycloalkyl such as, for example, cyclopropyl, cyclopropylmethyl, cyclobutyl, cyclopentyl or cyclohexyl, an aryl such as, for example, phenyl, benzyl or tolyl, a heterocyclic radical such as, for example, pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or a terminal or internal E- or Z-alkene unit and can alternatively be substituted by one or more C₁₋₆-alkyl radicals such as methyl, ethyl, propyl, isopropyl, butyl, isobutyl, t-butyl, pentyl, isopentyl, neopentyl or hexyl, C₃₋₇-cycloalkyl radicals such as cyclopropyl, cyclopropylmethyl, cyclobutyl, cyclopentyl or cyclohexyl, aryl radicals such as phenyl, benzyl, tolyl, naphthyl, indolyl, heterocyclic radicals such as pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Particularly preferred substituents are those such as hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, or one of the abovementioned radicals (a1) to (a28).

In the above structural units, R⁴ and R⁶, however, can also be bonded to one another and, with the nitrogen atom to which they are bonded, form a heterocyclic ring system. Examples of this which can be mentioned are: 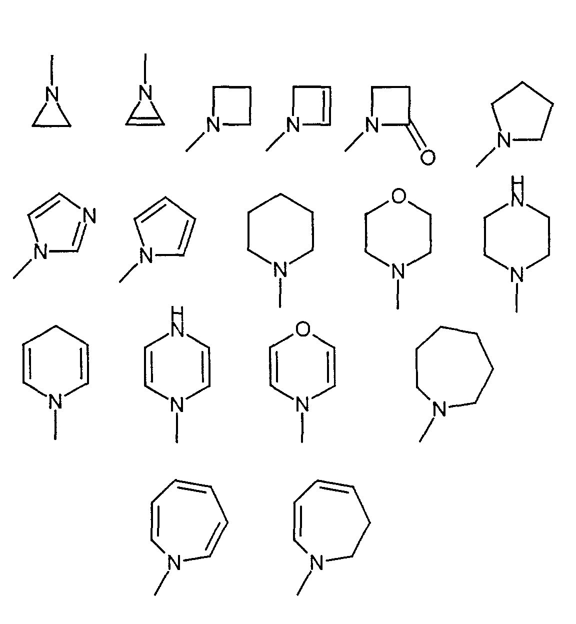 where the above enumeration is non-exclusive and the ring systems formed from the combination of R⁴ and R⁶ can carry one or more radicals which are selected from the group consisting of hydrogen, a C₁₋₆-alkyl such as, for example, methyl, ethyl, propyl, isopropyl, butyl, isobutyl, t-butyl, pentyl, isopentyl, neopentyl or hexyl, a C₃₋₇-cycloalkyl such as, for example, cyclopropyl, cyclopropylmethyl, cyclobutyl, cyclopentyl or cyclohexyl, an aryl such as, for example, phenyl, benzyl or tolyl, a heterocyclic radical such as, for example, pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or a terminal or internal E- or Z-alkene unit, and can alternatively be substituted by one or more C₁₋₆-alkyl radicals such as methyl, ethyl, propyl, isopropyl, butyl, isobutyl, t-butyl, pentyl, isopentyl, neopentyl or hexyl, C₃₋₇-cycloalkyl radicals such as cyclopropyl, cyclopropylmethyl, cyclobutyl, cyclopentyl or cyclohexyl, an aryl radical such as phenyl, benzyl, tolyl, naphthyl, indolyl, heterocyclic radicals such as pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, indolyl, benzofuranyl, benzoxazolyl, benzothiazolyl, benzimidazolyl, quinolinyl or isoquinolinyl unit or a partially or completely hydrogenated analogue thereof. Of the ring systems formed from the combination of R⁴ and R⁶, the four- to six-membered ring systems are preferred.

Furthermore, in the above structural units R⁵ can be -NO₂, -CN, -COR⁵' or -COOR⁵', where R⁵' can be a substituted or unsubstituted alkyl or cycloalkyl radical, a substituted or unsubstituted aryl radical or a saturated or unsaturated, optionally substituted heterocyclic radical, which can be saturated or unsaturated and/or can contain further heteroatoms, and is preferably a C₁₋₆-alkyl such as, for example, methyl, ethyl, propyl, isopropyl, butyl, isobutyl, t-butyl, pentyl, isopentyl, neopentyl, hexyl, a C₃₋₇-cycloalkyl such as, for example, cyclopropyl, cyclobutyl, cyclopentyl, cyclohexyl, an aryl such as, for example, phenyl, benzyl or tolyl or a substituted derivative thereof.

Furthermore, in the above structural units Y can be absent or can be an alkylene or alkine unit which in its main chain carries 1 to 5 carbon atoms. According to the invention, Y, if present, preferably has a main chain consisting of one carbon atom.

Y can moreover carry one or more radicals which are selected from the group consisting of hydrogen, a C₁₋₆-alkyl such as, for example, methyl, ethyl, propyl, isopropyl, butyl, isobutyl, t-butyl, pentyl, isopentyl, neopentyl or hexyl, a C₃₋₇-cycloalkyl such as, for example, cyclopropyl, cyclopropylmethyl, cyclobutyl, cyclopentyl or cyclohexyl, an aryl such as, for example, phenyl, benzyl or tolyl, a heterocyclic radical such as, for example, pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or a terminal or internal E- or Z-alkene unit, and can alternatively be substituted by one or more C₁₋₆-alkyl radicals such as methyl, ethyl, propyl, isopropyl, butyl, isobutyl, t-butyl, pentyl, isopentyl, neopentyl or hexyl, C₃₋₇-cycloalkyl radicals such as cyclopropyl, cyclopropylmethyl, cyclobutyl, cyclopentyl or cyclohexyl, aryl radicals such as phenyl, benzyl, tolyl, naphthyl, indolyl, heterocyclic radicals such as pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazol, pyrimidine, purine, cytosine, thymine, uracil, adenine, guanine or xanthine, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, indolyl, benzofuranyl, benzoxazolyl, benzothiazolyl, benzimidazolyl, quinolinyl or isoquinolinyl unit or a partially or completely hydrogenated analogue thereof.

According to the invention, particularly preferred radicals of the formula (II) are those in which, if the linkage to the rest of the conjugate does not take place via this, the nitrogen atom found in the main chain of the radical bonded to the phenyl ring B, which is next to the phenyl ring B, is a constituent of the urea or thiourea unit. Particularly preferred radicals of the formula (II) in this case are those in which a urea or thiourea unit is bonded directly to the phenyl ring B of the biphenyl nucleus.

The moiety addressing αᵥβ₃ integrin receptors can furthermore be a radical of the formula (III): 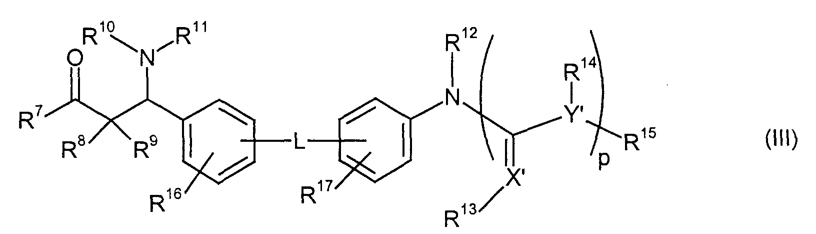 where the radicals in the formula (III) have the meaning defined above.

In the description below, bivalent substituents are indicated such that their respective left end is connected to the group indicated left of the corresponding substituent in formula (III) and their respective right end is connected to the group indicated right of the corresponding substituent in formula (III). If, for example, the radical L is equal to -(CH₂)ₘNHSO₂(CH₂)ₙ- in formula (III), the nitrogen atom is connected to the phenylene group found left of the radical L in formula (III) via the group (CH₂)ₘ. The following details additionally relate to the radical of the formula (III) in the unlinked state. The linkage of the radical of the formula (III) to the toxophore via the linking unit can take place either via the terminal carboxyl group, the terminal amino group, urea group, thiourea group, guanidine group or the group NR¹²CX'R¹³S- or via a functional group in the side chain of the radical of the formula (III), i.e. via the amino group or a substituent attached thereon in the β-position relative to the terminal carboxyl group, whereby in the linked state the terminal carboxyl group and the terminal amino group, urea group, thiourea group, guanidine group or the group NR¹²CX'R¹³S- are converted into corresponding bridging units.

The radicals of the formula (III) according to the invention are characterized in that they have, as a main structural element, two phenyl units connected via a linker group L, one phenylene group of which has a radical derived from a β-amino acid, while the other phenylene group has an amino group, urea group, thiourea group or guanidine group optionally incorporated into a cyclic ring system. The phenylene units connected via a linker group L can moreover carry further substituents in addition to the abovementioned radicals.

The terminal carboxyl units included in the radical derived from a β-amino acid can, if the linkage to the radical of the conjugate does not take place via this, be present as a free carboxylic acid or as an ester. In the case in which the terminal carboxyl unit is esterified, fundamentally all carboxylic acid esters obtainable by conventional processes, such as the corresponding alkyl esters, cycloalkyl esters, aryl esters and heterocyclic analogues thereof can be used according to the invention, where alkyl esters, cycloalkyl esters and aryl esters are preferred and the alcoholic radical can carry further substituents. Particularly preferred C₁₋₆-alkyl esters are those such as the methyl ester, ethyl ester, propyl ester, isopropyl ester, butyl ester, isobutyl ester, t-butyl ester, pentyl ester, isopentyl ester, neopentyl ester, hexyl ester, cyclopropyl ester, cyclopropylmethyl ester, cyclobutyl ester, cyclopentyl ester, cyclohexyl ester, or aryl esters such as the phenyl ester, benzyl ester or tolyl ester.

Preferably, the radicals of the formula (III) according to the invention are used in a form in which the terminal carboxyl unit is present as a free carboxylic acid.

The radical bonded to one of the two central phenylene units and derived from a β-amino acid can alternatively carry one or two additional substituents in the α-position relative to the carboxyl group. These substituents can each be selected from the group which consists of hydrogen, a substituted or unsubstituted alkyl or cycloalkyl radical, a substituted or unsubstituted aryl radical, a saturated or unsaturated, optionally substituted heterocyclic radical, an optionally substituted alkenyl radical, an optionally substituted alkinyl radical, a hydroxyl radical or an alkoxy radical. The alkyl radical can preferably be a C₁₋₆-alkyl such as, for example, methyl, ethyl, propyl, isopropyl, butyl, isobutyl, t-butyl, pentyl, isopentyl, neopentyl or hexyl. The cycloalkyl radical can preferably be a C₃₋₇-cycloalkyl such as, for example, cyclopropyl, cyclopropylmethyl, cyclobutyl, cyclopentyl, cyclohexyl or cycloheptyl. The aryl radical can preferably be phenyl, benzyl or tolyl. The heterocyclic radical can preferably be pyrrole, pyridine, tetrahydrofuran, furan, thiophene, tetrahydrothiophene, oxathiazole, benzofuran, quinoline, isoquinoline or pyrimidine. The alkenyl radical can be a terminal or internal E- or Z-alkene unit. The alkoxy radical can preferably be a C₁₋₆-alkoxy radical such as, for example, methoxy, ethoxy, propoxy, butoxy, pentoxy, hexoxy or benzyloxy. The abovementioned radicals can alternatively be substituted by one or more C₁₋₆-alkyl radicals such as methyl, ethyl, propyl, isopropyl, butyl, isobutyl, t-butyl, pentyl, isopentyl, neopentyl or hexyl, C₃₋₇-cycloalkyl radicals such as cyclopropyl, cyclopropylmethyl, cyclobutyl, cyclopentyl or cyclohexyl, aryl radicals such as phenyl, benzyl, tolyl, naphthyl, heterocyclic radicals such as pyrrole, pyridine, tetrahydrofuran, furan, thiophene, tetrahydrothiophene, oxazole, thiazole, oxathiazole, benzofuran, benzoxazole, benzothiazole, quinoline, isoquinoline, or functional groups such as a double bond to a heteroatom such as oxygen, sulphur or nitrogen, an optionally substituted amino group, a nitro group, a halogen group, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, benzofuranyl, benzoxazolyl, benzothiazolyl, quinolinyl or isoquinolinyl unit or a partially or completely hydrogenated analogue thereof.

Furthermore, the two substituents in the α-position relative to the terminal carboxyl group can, if present, be connected to one another and thus, together with the α-carbon atom of the radical derived from a β-amino acid, form a carbocyclic or heterocyclic ring system. This ring system can optionally carry further substituents and/or contain further heteroatoms. According to the invention, the above ring system, if present, is preferably a 3- to 6-membered carbocyclic or heterocyclic ring system such as, for example, a cyclopropane ring, cyclobutane ring, cyclopentane ring, cyclohexane ring, dihydrofuran ring, tetrahydrofuran ring, dihydropyran ring, tetrahydropyran ring, dioxane ring, dihydrothiophene ring, tetrahydrothiophene ring or a substituted derivative thereof.

In the groups according to the invention, the amino group included in the radical derived from a β-amino acid, if the linkage to the rest of the conjugate does not take place via this, is substituted by one of the radicals -SO₂R¹⁰', -COOR¹⁰", -COR¹⁰', -CONR¹⁰'₂ or -CSNR¹⁰'₂, where R¹⁰' can be hydrogen, a substituted or unsubstituted alkyl or cycloalkyl radical, a substituted or unsubstituted aryl radical or a saturated or unsaturated, optionally substituted heterocyclic radical and R¹⁰" can be a substituted or unsubstituted alkyl or cycloalkyl radical, a substituted or unsubstituted aryl radical or a saturated or unsaturated, optionally substituted heterocyclic radical. Preferably, the alkyl radical in this case is a C₁₋₆-alkyl such as, for example, methyl, ethyl, propyl, isopropyl, butyl, isobutyl, t-butyl, pentyl, isopentyl, neopentyl, hexyl, the cycloalkyl radical is a C₃₋₇-cycloalkyl such as, for example, cyclopropyl, cyclobutyl, cyclopentyl, cyclohexyl, cycloheptyl, the aryl radical is an aryl such as phenyl, benzyl, tolyl or a substituted derivative thereof such as -C₆H₂(CH₃)₃, -C₆(CH₃)₅, -CH₂C₆H₂(CH₃)₃, 3-aminophenyl, 4-aminophenyl, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 4-chlorophenylmethyl, 2,4-dichlorophenylmethyl, 2,6-dichlorophenylmethyl, 2-methoxycarbonylphenylmethyl, 3-trifluoromethylphenyl, 4-trifluoromethylphenyl, 3,5-bis(trifluoromethyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methylbenzothiazol-2-yl, N-methoxycarbonylpiperidin-3-yl, thiophen-2-yl, isoxazol-5-yl, ethoxy, 2-chloropyridin-3-yl, pyridin-3-yl, benzyloxy, 5-methylisoxazol-3-yl, 1-adamantyl, 4-chlorophenoxymethyl, 2,2-dimethylethenyl, 2-chloropyridin-5-methyl, 5,7-dimethyl-1,3,4-triazaindolizin-2-yl, (S)-camphan-1-yl, (R)-camphan-1-yl or 8-quinolinyl.

According to the invention, the amino group included in the radical derived from a β-amino acid is particularly preferably substituted by -SO₂R¹⁰', -COOR¹⁰", -CONR¹⁰'₂ or -COR¹⁰', where R¹⁰' and R¹⁰" are as defined above. In particular, radicals of the formula (III) are preferred here in which the radical derived from a β-amino acid has no substituent in the α-position relative to the carboxyl unit and the amino group included in this radical is substituted by -SO₂R¹⁰', -CONR¹⁰'₂ or -COR¹⁰', where R¹⁰' is as defined above.

In addition to one of the abovementioned radicals, the nitrogen atom of the amino group found in the β-position can have a substituent which is selected from the group consisting of hydrogen, a substituted or unsubstituted alkyl or cycloalkyl radical; a substituted or unsubstituted aryl radical, a saturated or unsaturated, optionally substituted heterocyclic radical or are bonded to one another and thus, together with the nitrogen atom to which they are bonded, form a heterocyclic ring system. Preferred substituents here are those which can be selected from the group consisting of hydrogen, a C₁₋₆-alkyl such as, for example, methyl, ethyl, propyl, isopropyl, butyl, isobutyl, t-butyl, pentyl, isopentyl, neopentyl or hexyl, a C₃₋₇-cycloalkyl such as, for example, cyclopropyl, cyclopropylmethyl, cyclobutyl, cyclopentyl, cyclohexyl or cycloheptyl, an aryl such as, for example, phenyl, benzyl or tolyl, a heterocyclic radical such as, for example, pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine and can alternatively be substituted by one or more C₁₋₆-alkyl radicals such as methyl, ethyl, propyl, isopropyl, butyl, isobutyl, t-butyl, pentyl, isopentyl, neopentyl or hexyl, C₃₋₇-cycloalkyl radicals such as cyclopropyl, cyclo-propylmethyl, cyclobutyl, cyclopentyl or cyclohexyl, aryl radicals such as phenyl, benzyl, tolyl, naphthyl, indolyl, heterocyclic radicals such as pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, indolyl, benzofuranyl, benzoxazolyl, benzothiazolyl, benzimidazolyl, quinolinyl or isoquinolinyl unit or a partially or completely hydrogenated analogue thereof. The additional substituent on the nitrogen atom of the β-amino group is particularly preferably hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methyl-cyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl,

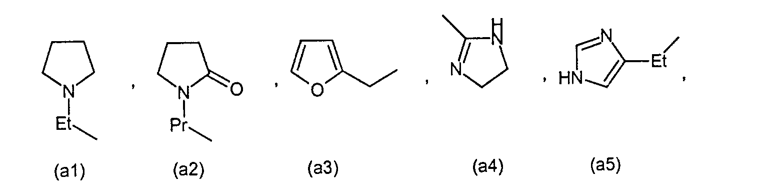

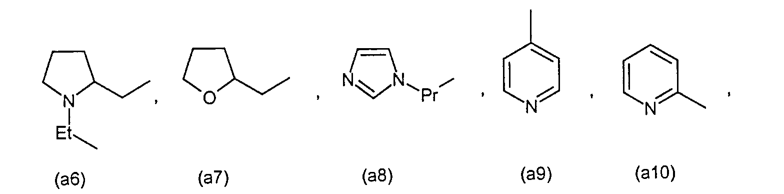

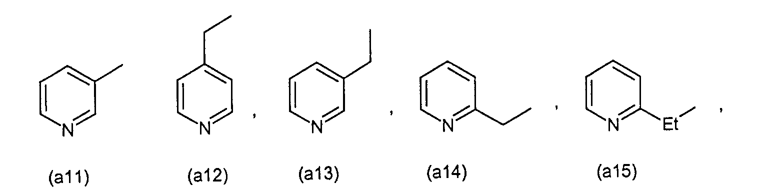

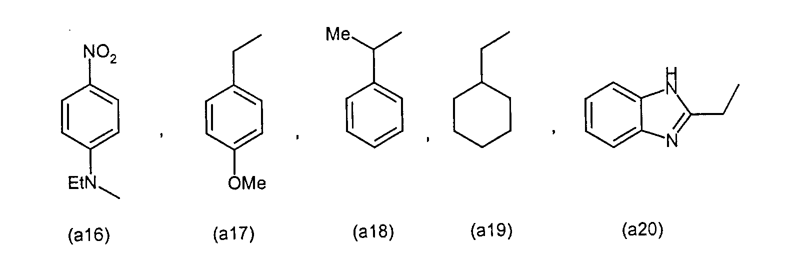

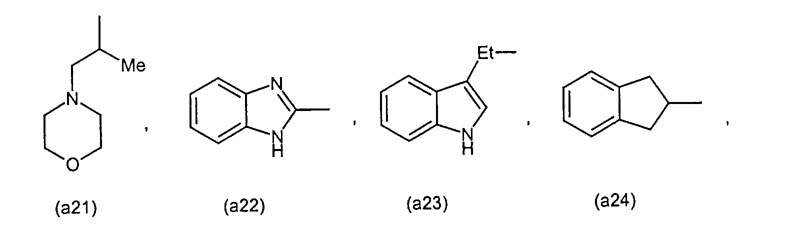

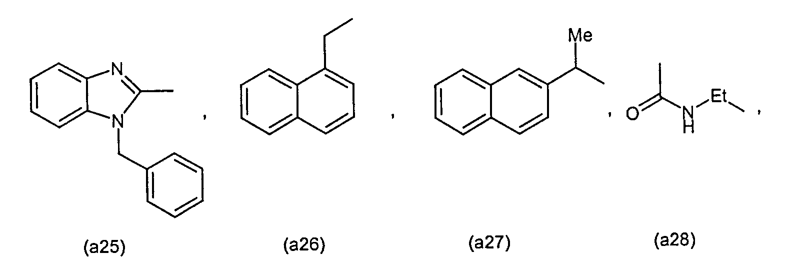

The radical derived from a β-amino acid is bonded to one of the two central phenylene units connected via a linker group L, which is to be designated here as phenylene unit A. In addition to the radical derived from a β-amino acid and the linker group L, the phenylene unit A preferably carries no further substituents, but can have one or more radicals which are selected from the group consisting of hydrogen, CN, a substituted or unsubstituted alkyl or cycloalkyl radical, a substituted or unsubstituted alkoxy radical or a halogen atom. The alkyl radical(s) is/are preferably C₁₋₆-alkyl radicals such as methyl, ethyl, propyl, isopropyl, butyl, isobutyl, t-butyl, pentyl, isopentyl, neopentyl, hexyl. The cycloalkyl radical(s) is/are preferably C₃₋₇-cycloalkyl radicals such as cyclopropyl, cyclobutyl, cyclopentyl, cyclohexyl, cycloheptyl. The alkoxy radical(s) is/are preferably C₁₋₆-alkoxy radicals such as methoxy, trifluoromethoxy, ethoxy, propoxy, butoxy, pentoxy or hexoxy, and the halogen atom(s) is/are preferably F, Cl, Br or I.

With respect to the linker group L and the radical derived from a β-amino acid or the amino, guanidine, urea or thiourea unit, the two central phenylene units can be 1,3-or 1,4-linked, i.e. the radical derived from a β-amino acid and the linker group L can be substituted in the meta- or para-position relative to one another in the phenylene unit A, and at the same time the linker group L and the amino, guanidine, urea or thiourea unit in the phenylene unit B can be substituted in the meta- or para-position relative to one another, where each combination of the abovementioned substitution patterns is possible for the central A-linker L-phenylene B unit of the radicals of the formula (III) according to the invention. Particularly preferred according to the present invention are those radicals of the formula (III) whose central phenylene A-linker L-phenylene B unit consists according to the above definition of a p-substituted phenylene unit A and a p-substituted phenylene unit B, a p-substituted phenylene unit A and an m-substituted phenylene unit B, an m-substituted phenylene unit A and a p-substituted phenylene unit B or an m-substituted phenylene unit A and an m-substituted phenylene unit B. Particularly preferred according to the present invention are radicals of the formula (III) whose central phenylene A-linker L-phenylene B unit consists according to the present definition of an m-substituted phenylene unit A and an m-substituted phenylene unit B.

According to the present invention, the linker group L is selected from the group which consists of the elements -(CH₂)ₘNHSO₂(CH₂)ₙ-, -(CH₂)ₘSO₂NH(CH₂)ₙ-, -(CH₂)ₘNHCO(CH₂)ₙ-, -(CH₂)ₘCONH(CH₂)ₙ-, -(CH₂)ₘOCH₂(CH₂)ₙ-, -(CH₂)ₘCH₂O(CH₂)ₙ-, -(CH₂)ₘCOO(CH₂)ₙ-, -(CH₂)ₘOOC(CH₂)ₙ-, -(CH₂)ₘCH₂CO(CH₂)ₙ-, -(CH₂)ₘCOCH₂(CH₂)ₙ-, -NHCONH-, -(CH₂)ₘSCH₂(CH₂)ₙ-, -(CH₂)ₘCH₂S(CH₂)ₙ-, -(CH₂)ₘCH₂SO(CH₂)ₙ-, -(CH₂)ₘSOCH₂(CH₂)ₙ, -(CH₂)ₘCH₂SO₂(CH₂)ₙ- or -(CH₂)ₘSO₂CH₂(CH₂)ₙ-,
where m and n each are an integer of 0 or 1 and m + n ≤ 1.

According to the invention, the linker group L is preferably -NHSO₂-, -CH₂NHSO₂-, -NHSO₂CH₂-, -SO₂NH-, -CH₂SO₂NH-, -SO₂NHCH₂-, -NHCO-, -CH₂NHCO-, -NH-COCH₂-, -CONH-, -CH₂CONH-, -CONHCH₂-, -OCH₂-, -CH₂OCH₂, -OCH₂CH₂-, -CH₂O-, -CH₂CH₂O-, -COO-, -CH₂COO-, -COOCH₂-, -OOC-, -OOCCH₂-, -CH₂OOC-, -CH₂CO-, -COCH₂-, -CH₂CH₂CO-, -COCH₂CH₂-, -CH₂COCH₂-, -NHCONH-, -SCH₂-, -CH2S-, -CH₂SCH₂, -SCH₂CH₂-, CH₂CH₂S-, -SOCH₂-, -CH₂SO-, -CH₂SOCH₂-, -SOCH₂CH₂-, -CH₂CH₂SO-, -SO₂CH₂-, -CH₂SO₂-, -CH₂SO₂CH₂-, -CH₂CH₂- SO₂- or -SO₂CH₂CH₂-. Particularly preferred linker groups L here are -NHSO₂-, -CH₂NHSO₂-, -NHSO₂CH₂-, -SO₂NH-, -CH₂SO₂NH-, -SO₂NHCH₂-, -NHCO-, -CH₂NHCO-, -NHCOCH₂-, -CONH-, -CH₂CONH-, -CONHCH₂-, -OCH₂-, -CH₂OCH₂, -OCH₂CH₂-, -CH₂O- or -CH₂CH₂O-.

The central phenylene unit B carries as a substituent a radical which, if the linkage to the radical of the conjugate does not take place via this, is selected from the group consisting of a group NR¹²CX'R¹³S-, an amino, guanidine, urea or thiourea unit. This group NR¹²CX'R¹³S-, amino, guanidine, urea or thiourea unit can be either open-chain or a constituent of a cyclic system. The nitrogen atoms of the respective unit, which are optionally both present and bonded only via single bonds, can carry additional substituents R¹², R¹⁴ and R¹⁵. These substituents can independently of one another or simultaneously be hydrogen, a substituted or unsubstituted alkyl or cycloalkyl radical, a substituted or unsubstituted aryl radical, a saturated or unsaturated, optionally substituted heterocyclic radical or can be bonded to one another and thus, together with the nitrogen atom(s) to which they are bonded, form a heterocyclic ring system. Preferred substituents here are those which are selected from the group consisting of hydrogen, a C₁₋₆-alkyl such as, for example, methyl, ethyl, propyl, isopropyl, butyl, isobutyl, t-butyl, pentyl, isopentyl, neopentyl or hexyl, a C₃₋₇-cycloalkyl such as, for example, cyclopropyl, cyclopropylmethyl, cyclobutyl, cyclopentyl, cyclohexyl or cycloheptyl, an aryl such as, for example, phenyl, benzyl or tolyl, a heterocyclic radical such as, for example, pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine and can alternatively be substituted by one or more C₁₋₆-alkyl radicals such as methyl, ethyl, propyl, isopropyl, butyl, isobutyl, t-butyl, pentyl, isopentyl, neopentyl or hexyl, C₃₋₇-cycloalkyl radicals such as cyclopropyl, cyclopropylmethyl, cyclobutyl, cyclopentyl or cyclohexyl, aryl radicals such as phenyl, benzyl, tolyl, naphthyl, indolyl, heterocyclic radicals such as pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydro-quinoline, tetrahydroisoquinoline, triazole, tetrazole, pyrimidine, purine, cytosine, thymine, uracil, adenine, guanine or xanthine, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, indolyl, benzofuranyl, benzoxazolyl, benzothiazolyl, benzimidazolyl, quinolinyl or isoquinolinyl unit or a partially or completely hydrogenated analogue thereof. Particularly preferred substituents are those such as hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl or one of the abovementioned radicals (a1) to (a28). If the linkage of the radical of the formula (III) to the rest of the conjugate takes place via this group, the radical R¹⁵ represents a direct bond via which the corresponding linkage between the radical of the formula (III) and the rest of the conjugate takes place.

The two radicals R¹⁴ and R¹⁵ or the radicals R¹² and R¹⁵, if p in the formula (III) represents 0, can be connected to one another and thus with the nitrogen atom form a heterocyclic ring system which can be selected, for example, from the following, non-exclusive list: 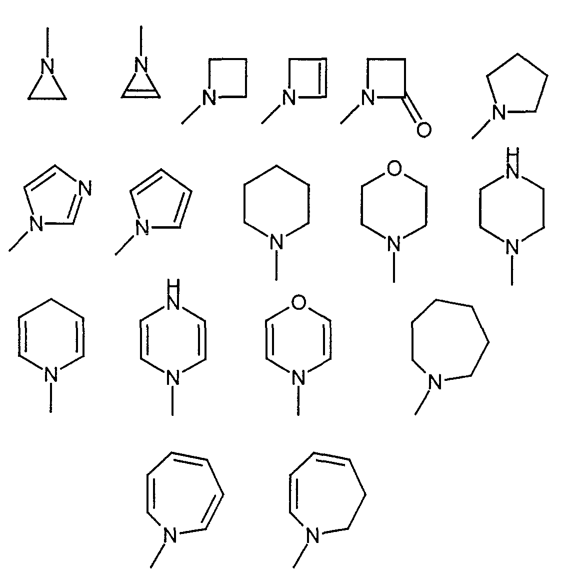 where the ring systems shown can carry one or more radicals which are selected from the group consisting of hydrogen, a C₁₋₆-alkyl such as, for example, methyl, ethyl, propyl, isopropyl, butyl, isobutyl, t-butyl, pentyl, isopentyl, neopentyl or hexyl, a C₃₋₇-cycloalkyl such as, for example, cyclopropyl, cyclopropylmethyl, cyclobutyl, cyclopentyl or cyclohexyl, an aryl such as, for example, phenyl, benzyl or tolyl, a heterocyclic radical such as, for example, pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or a terminal or internal E- or Z-alkene unit, and can alternatively be substituted by one or more C₁₋₆-alkyl radicals such as methyl, ethyl, propyl, isopropyl, butyl, isobutyl, t-butyl, pentyl, isopentyl, neopentyl or hexyl, C₃₋₇-cycloalkyl radicals such as cyclopropyl, cyclopropylmethyl, cyclobutyl, cyclopentyl or cyclohexyl, aryl radicals such as phenyl, benzyl, tolyl, naphthyl, indolyl, heterocyclic radicals such as pyrrolidine, piperidine, piperazine, pyrrole, pyridine, tetrahydrofuran, furan, thiophene, tetrahydrothiophene, imida-zolidine, imidazole, oxazolidine, oxazole, thiazolidine, thiazole, oxathiazole, benz-ofuran, benzoxazole, benzothiazole, benzimidazole, quinoline, isoquinoline, tetra-hydroquinoline, tetrahydroisoquinoline, triazole, tetrazole, pyrimidine, purine, cytosine, thymine, uracil, adenine, guanine or xanthine, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, indolyl, benzofuranyl, benzoxazolyl, benzothiazolyl, benzimidazolyl, quinolinyl or isoquinolinyl unit or a partially or completely hydrogenated analogue thereof.

Of the ring systems shown above, the four- to six-membered ring systems are preferred.

As mentioned above, the group NR¹²CX'R¹³S-, the amino, urea, thiourea or guanidine unit can be open-chain or incorporated into a cyclic system and thus be a constituent of one of the following preferred functional units: 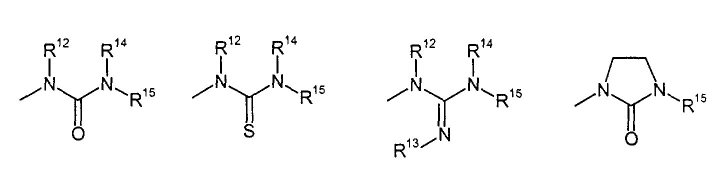 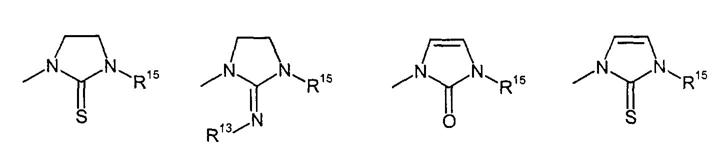 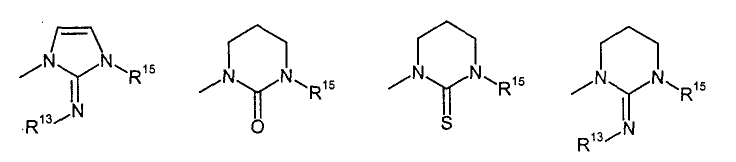 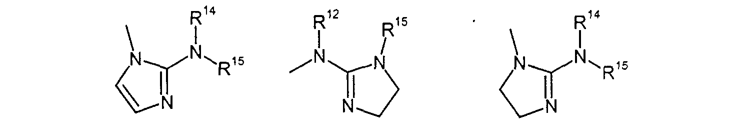 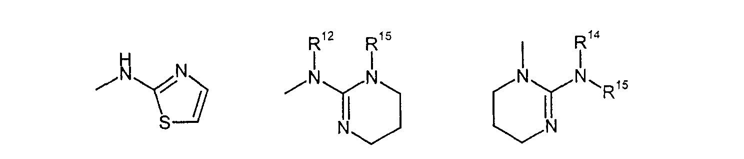 where the above list represents a non-exclusive enumeration of all possible structural units.

According to the invention, in addition to the abovementioned preferred structural units, their analogues are also included in which one or more 4- to 6-membered ring systems are fused to the heterocycle, such as, for example, the corresponding benzo-fused analogues of the above structural units.

In the structural units shown above, R¹², R¹⁴ and R¹⁵ are as defined above.

Furthermore, in the above structural units R¹³ can be absent, hydrogen, a substituted or unsubstituted alkyl or cycloalkyl radical such as, for example, a C₁₋₆-alkyl such as methyl, ethyl, propyl, isopropyl, butyl, isobutyl, t-butyl, pentyl, isopentyl, neopentyl, hexyl or a C₃₋₇-cycloalkyl such as cyclopropyl, cyclobutyl, cyclopentyl, cyclohexyl, -NO₂, -CN, -COR¹³' or -COOR¹³', where R¹³' can be hydrogen, a substituted or unsubstituted alkyl or cycloalkyl radical, a substituted or unsubstituted aryl radical or a saturated or unsaturated, optionally substituted heterocyclic radical, which can be saturated or unsaturated and/or can contain further heteroatoms, and is preferably a C₁₋₆-alkyl such as, for example, methyl, ethyl, propyl, isopropyl, butyl, isobutyl, t-butyl, pentyl, isopentyl, neopentyl, hexyl, a C₃₋₇-cycloalkyl such as, for example, cyclopropyl, cyclobutyl, cyclopentyl, cyclohexyl, an aryl such as, for example, phenyl, benzyl, tolyl or a substituted derivative.

According to the invention, particularly preferred radicals of the formula (III) are those in which the amino group included in the radical derived from a β-amino acid carries a radical-SO₂R¹⁰', where R¹⁰' is preferably methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -C₆H₂(CH₃)₃, -C₆(CH₃)₅, -CH₂C₆H₂(CH₃)₃, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 3-aminophenyl, 4-aminophenyl, 4-chlorophenylmethyl, 2,4-dichlorophenylmethyl, 2,6-dichlorophenylmethyl, 2-methoxycarbonylphenylmethyl, 3-trifluoro-methyl-phenyl, 4-trifluoromethylphenyl, 3,5-bis(trifluoromethyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methylbenzothiazol-2-yl, N-methoxycarbonylpiperidin-3-yl, thiophen-2-yl, isoxazol-5-yl, ethoxy, 2-chloropyridin-3-yl, pyridin-3-yl, benzyloxy, 5-methylisoxazol-3-yl, 1-adamantyl, 4-chlorophenoxymethyl, 2,2-dimethylethenyl, 2-chloropyridin-5-methyl, 5,7-dimethyl-1,3,4-triazaindolizin-2-yl, (S)-camphan-1-yl, (R)-camphan-1-yl or 8-quinolinyl, the linker group L is -NHSO₂-, -CH₂NHSO₂-, -NHSO₂CH₂-, and the radical found on the phenylene unit is an open-chain or cyclic guanidine unit, a cyclic guanidine unit such as, for example, a 4,5-dihydro-1H-imidazol-2-ylamino unit being particularly preferred.

Furthermore, according to the present invention radicals of the formula (III) are particularly preferred in which the amino group included in the radical derived from a β-amino acid carries a radical-SO₂R¹⁰' or a radical -COOR¹⁰", where R¹⁰' or R¹⁰" is preferably methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -C₆H₂(CH₃)₃, -C₆(CH₃)₅, -CH₂C₆H₂(CH₃)₃, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 3-aminophenyl, 4-aminophenyl, 4-chloro-phenyl-methyl, 2,4-dichlorophenylmethyl, 2,6-dichlorophenylmethyl, 2-methoxycarbonyl-phenylmethyl, 3-trifluoromethylphenyl, 4-trifluoromethylphenyl, 3,5-bis(trifluoro-methyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetra-methylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methyl-benzothiazol-2-yl, N-methoxycarbonyl-piperidin-3-yl, thiophen-2-yl, isoxazol-5-yl, ethoxy, 2-chloro-pyridin-3-yl, pyridin-3-yl, benzyloxy, 5-methylisoxazol-3-yl, 1-adamantyl, 4-chlorophenoxymethyl, 2,2-dimethylethenyl, 2-chloropyridin-5-methyl, 5,7-dimethyl-1,3,4-triazaindolizin-2-yl, (S)-camphan-1-yl, (R)-camphan-1-yl or 8-quinolinyl, the linker group L is -NHSO₂-, -CH₂NH SO₂-, -NHSO₂CH₂- or -OCH₂-, -CH₂O-, -CH₂OCH₂-, -CH₂CH₂O-, -OCH₂CH₂-, and the radical found on the phenylene unit is an open-chain or cyclic guanidine unit, a cyclic guanidine unit such as, for example, a 4,5-dihydro-lH-imidazol-2-ylamino unit being particularly preferred.

Moreover, according to the present invention radicals of the formula (III) are particularly preferred in which the amino group included in the radical derived from a β-amino acid carries a radical-COR¹⁰', where R¹⁰' is preferably hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -C₆H₂(CH₃)₃, -C₆(CH₃)₅, -CH₂C₆H₂(CH₃)₃, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 3-aminophenyl, 4-aminophenyl, 4-chlorophenylmethyl, 2,4-dichlorophenylmethyl, 2,6-dichlorophenylmethyl, 2-methoxycarbonylphenylmethyl, 3-trifluoromethylphenyl, 4-trifluoromethylphenyl, 3,5-bis(trifluoromethyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoro-methyl-phenyl, 2-alkylsulphonylphenyl, 2-arylsulphonylphenyl, 3-(N-acetyl-6-methoxy)-aniline, 4-acetamidophenyl, 2,2,2-trifluoroethyl, 5-chloro-3-methylbenzothiazol-2-yl, N-methoxycarbonyl-piperidin-3-yl, thiophen-2-yl, isoxazol-5-yl, ethoxy, 2-chloropyridin-3-yl, pyridin-3-yl, benzyloxy, 5-methylisoxazol-3-yl, 1-adamantyl, 4-chlorophenoxymethyl, 2,2-dimethylethenyl, 2-chloropyridin-5-methyl, 5,7-dimethyl-1,3,4-triazaindolizin-2-yl, (S)-camphan-1-yl, (R)-camphan-1-yl or 8-quinolinyl, the linker group L is -NHSO₂-, -CH₂NHSO₂-, -NHSO₂CH₂-, and the radical found on the phenylene unit is an open-chain or cyclic guanidine unit, a cyclic guanidine unit such as, for example, a 4,5-dihydro-1H-imidazol-2-ylamino unit being particularly preferred.

Moreover, according to the present invention radicals of the formula (III) are particularly preferred in which the amino group included in the radical derived from a β-amino acid carries a radical -COR¹⁰', where R¹⁰' is preferably hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -C₆H₂(CH₃)₃, -C₆(CH₃)₅, -CH₂C₆H₂(CH₃)₃, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 3-aminophenyl, 4-aminophenyl, 4-chlorophenylmethyl, 2,4-dichlorophenylmethyl, 2,6-dichlorophenylmethyl, 2-methoxycarbonylphenylmethyl, 3-trifluoromethylphenyl, 4-trifluoromethylphenyl, 3,5-bis(trifluoromethyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoro-methyl-phenyl, 2-alkylsulphonylphenyl, 2-arylsulphonylphenyl, 3-(N-acetyl-6-methoxy)-aniline, 4-acetamidophenyl, 2,2,2-trifluoroethyl, 5-chloro-3-methylbenzothiazol-2-yl, N-methoxycarbonyl-piperidin-3-yl, thiophen-2-yl, isoxazol-5-yl, ethoxy, 2-chloropyridin-3-yl, pyridin-3-yl, benzyloxy, 5-methylisoxazol-3-yl, 1-adamantyl, 4-chlorophenoxymethyl, 2,2-dimethylethenyl, 2-chloropyridin-5-methyl, 5,7-dimethyl-l,3,4-triazaindolizin-2-yl, (S)-camphan-1-yl, (R)-camphan-1-yl or 8-quinolinyl, the linker group L is -NHSO₂-, -CH₂NHSO₂-, -NHSO₂CH₂-, and the radical found on the phenylene unit is an open-chain or cyclic guanidine unit, a cyclic guanidine unit such as, for example, a 4,5-dihydro-lH-imidazol-2-ylamino unit being particularly preferred.

The moiety addressing αᵥβ₃ integrin receptors can furthermore be a radical of the formula (IV): 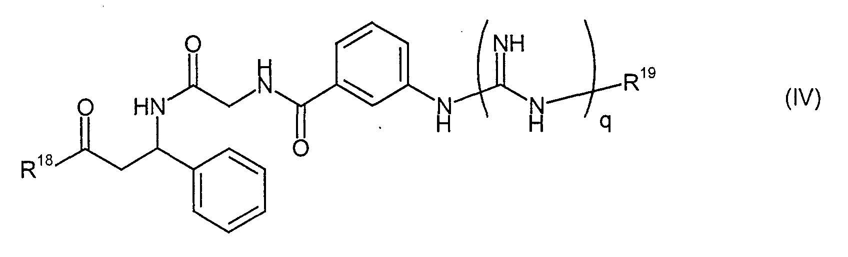 where the radicals in the formula (IV) have the meaning defined above.

The terminal carboxyl unit can, if the linkage to the rest of the conjugate does not take place via this, be present as a free carboxylic acid or as an ester. In the case in which the terminal carboxyl unit is esterified, fundamentally all carboxylic esters obtainable by conventional processes, such as the corresponding alkyl esters, cycloalkyl esters, aryl esters and hetereocyclic analogues thereof can be used according to the invention, where alkyl esters, cycloalkyl esters and aryl esters are preferred and the alcoholic radical can carry further substituents. Particularly preferred C₁₋₆-alkyl esters are those such as the methyl ester, ethyl ester, propyl ester, isopropyl ester, butyl ester, isobutyl ester, t-butyl ester, pentyl ester, isopentyl ester, neopentyl ester, hexyl ester, cyclopropyl ester, cyclopropylmethyl ester, cyclobutyl ester, cyclopentyl ester, cyclohexyl ester, or aryl esters such as the phenyl ester, benzyl ester or tolyl ester.

The radicals of the formula (IV) according to the invention are preferably used in a form in which the terminal carboxyl unit is present as the free carboxylic acid.

The radicals of the formula (IV) according to the invention can contain a terminal guanidine or amino unit. The radical R¹⁹ here can be hydrogen, a substituted or unsubstituted alkyl or cycloalkyl radical, a substituted or unsubstituted aryl radical or a saturated or unsaturated, optionally substituted heterocyclic radical. A substituent is preferred here which is selected from the group consisting of hydrogen, a C₁₋₆-alkyl such as, for example, methyl, ethyl, propyl, isopropyl, butyl, isobutyl, t-butyl, pentyl, isopentyl, neopentyl or hexyl, a C₃₋₇-cycloalkyl such as, for example, cyclopropyl, cyclopropylmethyl, cyclobutyl, cyclopentyl, cyclohexyl or cycloheptyl, an aryl such as, for example, phenyl, benzyl or tolyl, a heterocyclic radical such as, for example, pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine and can alternatively be substituted by one or more C₁₋₆-alkyl radicals such as methyl, ethyl, propyl, isopropyl, butyl, isobutyl, t-butyl, pentyl, isopentyl, neopentyl or hexyl, C₃₋₇-cycloalkyl radicals such as cyclopropyl, cyclopropylmethyl, cyclobutyl, cyclopentyl or cyclohexyl, aryl radicals such as phenyl, benzyl, tolyl, naphthyl, indolyl, heterocyclic radicals such as pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, indolyl, benzofuranyl, benzoxazolyl, benzothiazolyl, benzimidazolyl, quinolinyl or isoquinolinyl unit or a partially or completely hydrogenated analogue thereof. Particularly preferred substituents are those such as hydrogen, methyl, ethyl, propyl, isopropyl, butyl, isobutyl, t-butyl, pentyl, isopentyl, neopentyl, hexyl, cyclopropyl, cyclo-propylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄alkyl, C₁₋₄-alkyloxy-C₁₋₄-alkyl or one of the abovementioned radicals (a1) to (a28). If the linkage of the radical of the formula (IV) to the rest of the conjugate takes place via this group, the radical R¹⁷ represents a direct bond, via which the corresponding linkage between the radical of the formula (IV) and the rest of the conjugate takes place.

The novel conjugates according to Claim 1 can be prepared by linkage of the toxophore to the linking unit and subsequent linkage to the moiety addressing αᵥβ₃ integrin receptors. However, it is also possible to first connect the moiety addressing αᵥβ₃ integrin receptors to the linking unit and then to bind the toxophore to the linking unit.

The combination of the individual units of the conjugates according to the invention can preferably be carried out by means of functional groups which can be reacted with one another and, as a result, can be linked by conventional processes known to the person skilled in the art. For example carboxyl functions can be reacted with amino functions with formation of an amide bond. It is also possible to synthesize the linking unit stepwise on one of the two radicals to be connected, i.e. the toxophore or the moiety addressing αᵥβ₃ integrin receptors, by conventional processes known to the person skilled in the art and then to link the finished linking unit to the radical which is still to be bound.

The present invention in particular relates to a process for the preparation of conjugates according to formula (I),
comprising
[A] the reaction of a compound from the group of compounds of the formulae (II), (III) and (IV), which has a free or optionally activated carboxyl function,
   with a compound of the formula (Ia) which has a free primary or secondary amino group

   CT-LI (Ia)

   in which all radicals have the meaning indicated in Claim 1,
   in the presence of a base;
   or
[B] the reaction of a compound from the group of compounds of the formulae (II), (III) and (IV), which has a free primary or secondary amino function,
   with a carbonic acid derivative such as, for example, phosgene, thiophosgene or a chloroformic acid ester, if appropriate in the presence of a base,
   followed by the reaction with a compound of the formula (Ia) which has a free primary or secondary amino group

   CT-LI (Ia)

   in which all radicals have the meaning indicated in Claim 1,
   and
   if appropriate the removal of protective groups and/or derivatization of nitrogen atoms present at preferred points of time in the preparation process and/or conversion of the compound obtained into the free acid
   and/or conversion of the compound obtained into one of its physiological salts by reaction with an inorganic or organic base or acid;
   or
[C] the reaction of a compound from the group of compounds of the formulae (II), (III) and (IV), which contains a free primary or secondary amino function,
   with a compound of the formula (Ia) which contains a free or optionally activated carboxyl function

   CT-LI (Ia)

   in which all radicals have the meaning indicated in Claim 1,
   in the presence of a base;
   and
   if appropriate the removal of protective groups and/or derivatization of nitrogen atoms present at preferred points in time in the preparation process and/or conversion of the compound obtained into the free acid and/or conversion of the compound obtained into one of its physiological salts by reaction with an inorganic or organic base or acid.

According to a preferred embodiment, several steps of the preparation process are carried out on a solid phase.

In variant [A] of the preparation process according to the invention, a moiety addressing αᵥβ₃ integrin receptors from the group of radicals of the formulae (II), (III) or (IV) is linked via its free carboxyl function to the amino function of a toxophore-linking unit conjugate (Ia) with formation of an amide bond. This reaction can be carried out by conventional methods known to the person skilled in the art (cf., for example, J. March, Advanced organic chemistry, 3^{rd} ed., Wiley, p. 370 ff.). It is preferred according to the invention to activate the carboxyl function of the moiety addressing αᵥβ₃ integrin receptors and then to react with the compound (Ia) in an organic solvent in the presence of a base.

For the activation of the carboxyl group, the coupling reagents known in peptide chemistry can be used, such as are described, for example, in Jakubke/Jeschkeit: Aminosäuren, Peptide, Proteine [Amino acids, Peptides, Proteins]; Verlag Chemie 1982 or Tetrahedr. Lett. 34, 6705 (1993). Examples mentioned are N-carboxylic acid anhydrides, acid chlorides or mixed anhydrides, adducts with carbodiimides, e.g. N,N'-diethyl-, N,N'-diisopropyl- or N,N'-dicyclohexylcarbodiimide, N-(3-dimethylaminopropyl)N'-ethyl-carbodiimide hydrochloride, N-cyclohexyl-N'-(2-morpholinoethyl)-carbodiimide metho-p-toluenesulphonate, or carbonyl compounds such as carbonyldiimidazole, or 1,2-oxazolium compounds such as 2-ethyl-5-phenyl-1,2-oxazolium-3-sulphate or 2-tert-butyl-5-methyl-isoxazolium perchlorate, or acylamino compounds such as 2-ethoxy-1-ethoxycarbonyl-1,2-dihydroquinoline or propanephosphonic anhydride or isobutyl chloroformate or benzotriazolyloxy-tris-(dimethylamino)-phosphonium hexafluorophosphate, 1-hydroxybenzotriazole or N-hydroxysuccinimide esters. It is furthermore proposed to employ the acid components in the form of a Leuchs' anhydride.

Variant [A] of the above preparation process according to the invention can be carried out under various pressure and temperature conditions, for example 0.5 to 2 bar and preferably under normal pressure, or -30 to +100°C and preferably -10 to +80°C, in suitable solvents such as dimethylformamide (DMF), tetrahydrofuran (THF), dichloromethane, chloroform, lower alcohols, acetonitrile, dioxane, water or in mixtures of the solvents mentioned. As a rule, reaction in DMF, dichloromethane, THF, dioxane/water or THF/dichloromethane at room temperature or with icecooling and under normal pressure is preferred.

Bases which can be employed in variant [A] of the preparation process according to the invention are, for example, triethylamine, ethyl-diisopropylamine, pyridine, N,N-dimethylaminopyridine or other bases conventionally used in steps of this type.

In variant [B] of the process according to the invention, a moiety addressing αᵥβ₃ integrin receptors from the group of radicals of the formulae (II), (III) and (IV) is reacted via its free amino function first with a carbonic acid derivative with formation of a corresponding isocyanate, isothiocyanate or carbamate, which is then linked to the amino function of a toxophore-linking unit conjugate (Ia) with formation of the conjugate (I).

The reaction of the moiety addressing αᵥβ₃ integrin receptors from the group of radicals of the formulae (II), (III) or (IV) via its free amino function with a carbonic acid derivative can be carried out by conventional methods known to the person skilled in the art (cf., for example, J. March, Advanced organic chemistry, 3^{rd} ed., Wiley, p. 370 ff.). According to the invention, the reaction is preferably carried out with phosgene or a substitute for phosgene such as, for example, trichloromethyl chloroformate, thiophosgene or a chloroformic acid ester in a solvent such as dimethylformamide (DMF) or a mixture of dioxane and water (1:1) or of tetrahydrofuran (THF) and dichloromethane (DCM) (1:1) at room temperature or with cooling, preferably at room temperature, and stirring for approximately 10 minutes up to approximately 3 hours, if appropriate in the presence of a base.

The subsequent reaction of the isocyanate, isothiocyanate or carbamate thus obtained with the amino function of a toxophore-linking unit conjugate (Ia) with formation of a corresponding thiourea or urea bond can be carried out by conventional methods known to the person skilled in the art (cf., for example, J. March, Advanced organic chemistry, 3^{rd} ed., Wiley, p. 802 ff.).

According to the invention, the carbamate or thiocyanate or isothiocyanate is preferably reacted with the amino function of the compound (Ia) at room temperature with stirring for approximately 1 to 5 hours, preferably approximately 2 to 3 hours, in the presence of a base in a solvent such as dimethylformamide (DMF).

Bases which can be employed in variant [B] of the preparation process according to the invention are, for example, triethylamine, ethyldiisopropylamine, pyridine, N,N-dimethylaminopyridine or other bases conventionally used in steps of this type.

In variant [C] of the preparation process according to the invention, a moiety addressing αᵥβ₃ integrin receptors from the group of radicals of the formulae (II), (III) and (IV) is linked via its free amino function to the carboxyl function of a toxophore-linking unit conjugate (Ia) with formation of an amide bond. This reaction can be carried out by conventional methods known to the person skilled in the art (cf., for example, J. March, Advanced organic chemistry, 3^{rd} ed., Wiley, p. 370 ff.). It is preferred according to the invention to activate the carboxyl function of the compound (Ia) and then to react it with a moiety addressing αᵥβ₃ integrin receptors from the group of radicals of the formulae (II), (III) and (IV) in an organic solvent in the presence of a base.

For activation of the carboxyl group, the coupling reagents known in peptide chemistry can be used, such as are described, for example, in Jakubke/Jeschkeit: Aminosäuren, Peptide, Proteine [Amino acids, Peptides, Proteins]; Verlag Chemie 1982 or Tetrahedr. Lett. 34, 6705 (1993). Examples mentioned are N-carboxylic anhydrides, acid chlorides or mixed anhydrides, adducts with carbodiimides, e.g. N,N'-diethyl-, N,N'-diisopropyl- or N,N'-dicyclohexylcarbodiimide, N-(3-dimethylaminopropyl)-N'-ethyl-carbodiimide hydrochloride, N-cyclohexyl-N'-(2-morpholinoethyl)-carbodiimide metho-p-toluenesulphonate, or carbonyl compounds such as carbonyldiimidazole, or 1,2-oxazolium compounds such as 2-ethyl-5-phenyl-1,2-oxazolium-3-sulphate or 2-tert-butyl-5-methyl-isoxazolium perchlorate, or acylamino compounds such as 2-ethoxy-1-ethoxycarbonyl-1,2-dihydroquinoline, or propanephosphonic anhydride, or isobutyl chloroformate, or benzotriazolyloxy-tris-(dimethylamino)-phosphonium hexafluorophosphate, 1-hydroxybenzotriazole or N-hydroxysuccinimide esters. It is furthermore proposed to employ the acid components in the form of a Leuchs' anhydride.

Variant [C] of the above preparation process according to the invention can be carried out under various pressure and temperature conditions, for example 0.5 to 2 bar and preferably under normal pressure, or -30 to +100°C and preferably -10 to +80°C, in suitable solvents such as dimethylformamide (DMF), tetrahydrofuran (THF), dichloromethane, chloroform, lower alcohols, acetonitrile, dioxane, water or in mixtures of the solvents mentioned. As a rule, reaction in DMF, dichloromethane, THF, dioxane/water or THF/dichloromethane at room temperature or with icecooling and at normal pressure is preferred.

Bases which can be employed in variant [C] of the preparation process according to the invention are, for example, triethylamine, ethyldiisopropylamine, pyridine, N,N-dimethylaminopyridine or other bases conventionally used in steps of this type such as, for example, Hünig's base.

The compounds obtained according to the process explained above can furthermore be derivatized by removal of protective groups which may be present, further substitution of nitrogen atoms present at preferred positions in the preparation process and/or conversion of the compound obtained into the free acid and/or its physiologically acceptable salts. By way of example, the t-butoxymethoxycarbonyl groups conventionally used as protective groups for nitrogen atoms are removed in acidic medium, for example by addition of trifluoroacetic acid. Suitable alkylating agents for the derivatization of nitrogen atoms in this step are reagents conventionally used for this purpose, using which, for example, a substituted or unsubstituted alkyl or cycloalkyl radical, a substituted or unsubstituted aryl radical or a saturated or unsaturated, optionally substituted heterocyclic radical can be bonded to the appropriate nitrogen atom. With respect to the substituents preferably bonded to the respective nitrogen atoms, reference is made to the above description of the compounds according to the invention. The above reactions and their implementation are well known to the person skilled in the art and are described in detail in standard works such as, for example, Houben-Weyl, Methoden der organischen Chemie [Methods of Organic Chemistry], Georg Thieme Verlag, Stuttgart.

The ester derivatives according to the invention can be converted into the corresponding free carboxylic acids in a conventional manner, such as, for example, by basic ester hydrolysis.

If desired, the compounds according to the invention can be converted into their physiologically acceptable salts. This can be carried out either by reaction with an organic or inorganic base such as, for example, an alkali metal hydroxide or alkaline earth metal hydroxide such as KOH, NaOH, LiOH, Mg(OH)₂ or Ca(OH)₂, as a result of which the terminal carboxyl group is deprotonated and the corresponding carboxylate is formed, or by reaction with an organic or inorganic acid such as, for example, hydrochloric acid, sulphuric acid, phosphoric acid, mandelic acid, oleic acid, linoleic acid or p-toluenesulphonic acid, as a result of which one or more of the nitrogen atoms present are protonated.

The compounds of the formula (Ia) serving as starting substances can be prepared by conventional methods. The linkage of the toxophore to amino acid units can be carried out by conventional methods of peptide chemistry (cf., for example, Jakubke/Jeschkeit: Aminosäuren, Peptide, Proteine [Amino acids, Peptides, Proteins]; Verlag Chemie 1982, Houben-Weyl, Methoden der Organischen Chemie [Methods of Organic Chemistry], Georg Thieme Verlag Stuttgart, Fourth Edition; Volume 15.1 and 15.2, edited by E.Wünsch) and is also described, for example, in WO 96/31532 and WO 98/51703, whose contents are inserted here by means of reference.

The bonding of the appropriate carbonyl or thiocarbonyl radicals can be carried out as described above by reaction of the toxophore or of the toxophore-amino acid conjugate with phosgene or a substitute for phosgene such as, for example, trichloromethyl chloroformate or thiophosgene.

Although according to the invention it is preferred to first synthesize the toxophore-linking unit conjugate (Ia), it is also possible, of course, to build up the linking unit in series first on the moiety addressing αᵥβ₃ integrin receptors or to bond it as a whole and then to connect the conjugate thus obtained to the toxophore.

According to a preferred embodiment of the present invention, the synthesis of the compounds according to the invention is partly carried out on a solid phase such as a polystyrene resin, particularly preferably a commercially available Wang polystyrene resin. The resin is in this case first swollen in a solvent such as dimethylformamide (DMF). The moiety of the formula (II), (III) or (IV) addressing αᵥβ₃ integrin receptors is then bonded to the resin via its carboxyl function by standard processes. For example, the bonding of the carboxylic acid to the resin can be carried out in the presence of a base such as pyridine and a reagent activating the carboxyl unit, such as an acid halide, for example dichlorobenzoyl chloride, in a solvent such as dimethylformamide (DMF). However, other reagents conventionally used for this purpose can also be employed. The reaction mixture is stirred at room temperature and normal pressure for at least 2 hours, preferably 12 hours, particularly preferably approximately 24 hours, the carboxylic acid being employed in an excess with respect to the loading of the solid phase, preferably in a two- to three-fold excess. All reactions described herein can then be carried out on the moiety of the formula (II), (III) or (IV) bound to the resin and addressing αᵥβ₃ integrin receptors, as described here.

According to a preferred embodiment of the present invention, the toxophore is camptothecin or a camptothecin derivative. The linkage of these toxophores to the linking unit can be carried out via the C20 OH group or other functional groups in the molecule..

The camptothecin unit used as a starting compound can be present in the 20(R) or in the 20(S) configuration or as a mixture of these two stereoisomeric forms. The 20(S) configuration is preferred.

After linkage of the first amino acid to camptothecin, diastereomer mixtures can be formed. Pure diastereomers of the compounds according to the invention can be prepared by the processes indicated above, for example, by separating the diastereomers in a suitable manner after coupling of the first amino acid unit to the camptothecin and subsequent protective group removal.

The radical of the formula (II) addressing αᵥβ₃ integrin receptors can be prepared from commercially obtainable starting compounds by the following steps:
a) reaction of a carboxylic acid derivative of the formula (IIa) 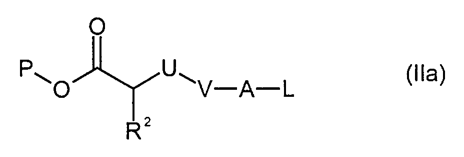 where
   - P: is a conventional protective group, a solid phase conventionally used for carrying out a solid-phase reaction or R¹ as defined above;
   - A: is a phenylene group optionally containing additional radicals, which is 1,3- or 1,4-substituted with respect to V and L;
   - L: is -H, -F, -Cl, -Br, -I, -SCN, -N₂⁺ or an organometallic radical;
   and the other radicals are as defined above;
   with a phenyl compound of the formula (IIb)

   M-B-W-D (IIb)

   where
   - M: is -H, -I, -N₂⁺, -COOOCOBNO₂ or an organometallic radical;
   - B: is a phenylene group optionally containing additional radicals, which is 1,3- or 1,4-substituted with respect to M and W-D;
   - W: is as defined in Claim 1;
   - D: is -NO₂, -NH₂ or -CHO;
   to give a biphenyl compound of the formula (IIc) 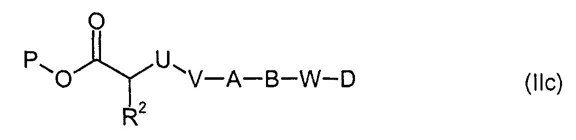 where the radicals are as defined above;
b) conversion of the radical D into the corresponding amino group, if D is not -NH₂; and
c) if appropriate, derivatization of nitrogen atoms present at preferred points of time in the preparation process and/or the conversion of the compound obtained into the free acid and/or the conversion of the compound obtained to one of its physiological salts by reaction with an inorganic or organic base or acid.

According to a preferred embodiment, in the process according to the invention all steps are carried out during the binding of the carboxylic acid derivative of the formula (IIa) to a solid phase.

Furthermore, according to an embodiment of the process which is preferred according to the invention, a carboxylic acid derivative of the formula (IIa), in which
- L: is -F, -Cl, -Br or -I
and the other radicals are as defined above,
is reacted with a phenyl compound of the formula (IIb), in which
- M: is an organometallic radical;
and the other radicals are as defined above,
in the presence of a palladium(II) compound and of triphenylphosphane.

Preferably, in the above process according to the invention a carboxylic acid derivative of the formula (IIa) is employed which contains a sulphonamide or carbamate group which was formed by reaction of an amino group of the corresponding precursor of the carboxylic acid derivative of the formula (IIa) with a sulphonyl halide or a carbamoyl halide.

It is furthermore preferred that in the above process according to the invention in the case in which the compound of the formula (IIc) D is equal to -NO₂, the conversion of D into an amino group is carried out in the presence of a tin(II) compound.

It is furthermore preferred that in the above process according to the invention in the case in which the compound of the formula (IIc) D is equal to -CHO, the conversion of D into an amino group is carried out by reaction of an amine under reducing conditions.

It is moreover preferred that the compound of the formula (IIc) in which D is an amino group is converted, by a reaction of this amino group with a carbonic acid derivative or thiocarbonic acid derivative and a reaction following this with an amine of the formula NHR⁴R⁶, into a urea or thiourea unit, where R⁴ and R⁶ are as defined above.

The essential steps of the preparation process for the radical of the formula (II) are the reaction of a carboxylic acid, whose carboxyl group is protected and which has at least one aryl group provided with a radical accessible to an aryl-aryl coupling reaction, with a phenyl compound having at least one radical accessible to an aryl-aryl coupling reaction, which furthermore has a radical D which is an amino group or can be converted into an amino group in a simple manner, and the conversion of the radical D into the corresponding amino group, if it is not already an amino group. Further process steps which can be included are the derivatization of nitrogen atoms present in the molecule at preferred points in time in the preparation process and/or the conversion of the compound thus obtained into the free acid and/or the conversion of the compound thus obtained into one of its physiologically acceptable salts by reaction with an inorganic or organic acid or base.

The carboxylic acids to be employed as starting compounds are either commercially accessible or accessible in a simple manner by chemical standard processes, such as are known to any person skilled in the art and are described in standard works such as Houben-Weyl, Methoden der organischen Chemie [Methods of Organic Chemistry], Georg Thieme-Verlag, Stuttgart.

According to a preferred embodiment, the process for the preparation of radicals of the formula (II) starts from the following carboxylic acid derivatives: 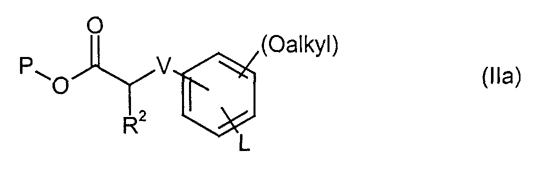 For the preparation process, the carboxyl group is in this case blocked by a conventional protective group P. Protective groups of this type are known to the person skilled in the art and do not have to be expressly mentioned here. The carboxyl group is particularly preferably esterified, P being a C₁₋₆-alkyl such as, for example, methyl, ethyl, propyl, isopropyl, butyl, isobutyl, t-butyl, pentyl, isopentyl, neopentyl, hexyl, a C₃₋₇-cycloalkyl such as, for example, cyclopropyl, cyclo-propylmethyl, cyclobutyl, cyclopentyl, cyclohexyl, an aryl such as, for example, phenyl, benzyl or tolyl or a substituted derivative thereof. Particularly preferably, however, the preparation process for the radicals of the formula (II) is carried out on a solid phase in order to achieve an implementation of the process which is as economical as possible. In this case, the carboxyl radical can be connected to any solid phase conventionally used for reactions of this type. According to the invention, a solid phase particularly preferably used is a polystyrene resin and, in particular, a commercially obtainable Wang polystyrene resin.

According to the present preferred embodiment, R² can be as described above and V can be an optionally substituted C₁₋₅-alkylene group. Thus the starting compounds of this preferred embodiment can be interpreted as derivatives of propanoic acid, butanoic acid, pentanoic acid, hexanoic acid or heptanoic acid. In the α-position to the carboxyl group, these carboxylic acid derivatives can have a substituent such as, for example, hydrogen, a C₁₋₆-alkyl such as, for example, methyl, ethyl, propyl, isopropyl, butyl, isobutyl, t-butyl, pentyl, isopentyl, neopentyl, hexyl, a C₃₋₇-cycloalkyl such as, for example, cyclopropyl, cyclobutyl, cyclopentyl, cyclohexyl, an aryl such as, for example, phenyl, benzyl or tolyl or a substituted derivative thereof, an optionally substituted alkenyl radical, an optionally substituted alkinyl radical, -NR²'SO₂R²', -NR²'COOR²', -NR²'COR²',-NR²'CONR²'₂ or -NR²'CSNR²'₂. The alkyl and cycloalkyl radicals and the benzyl radical can be introduced, for example, by reaction of the ester of the starting compounds with the appropriate alkyl, cycloalkyl or benzyl halides in basic medium if the corresponding derivatives are not commercially obtainable. The alkinyl radical can be introduced, for example, by reaction of the α-bromo ester of the present starting compound, which is accessible via the Reformatski reaction, with an appropriate acetylide anion. In the case of the phenyl radical, of the alkenyl radical and of the nitrogen-containing substituents, the corresponding α-phenyl- or α-aminocarboxylic acid derivatives are preferably used as starting materials and, if necessary, the other substituents on the α-C atom relative to the terminal carboxyl group are introduced by means of the corresponding alkyl halide. The above reactions and their implementation are well known to the person skilled in the art and are described in detail in standard works such as, for example, Houben-Weyl, Methoden der organischen Chemie [Methods of Organic Chemistry], Georg Thieme Verlag, Stuttgart.

For the introduction of a substituent in the β-position relative to the carboxyl group, it suggests itself, for example, to start from the corresponding α,β-unsaturated carboxylic acid derivatives and to react these with the respective alkyl, cycloalkyl or aryl cuprates in the sense of a Michael addition. It is then possible, if desired, to additionally introduce a substituent in the α-position relative to the carboxyl group as described above. These reactions and their implementation are also well known to the person skilled in the art and are described in detail in standard works such as, for example, Houben-Weyl, Methoden der organischen Chemie [Methods of Organic Chemistry], Georg Thieme Verlag, Stuttgart.

The radicals -NR²'SO₂R²', -NR²'COOR²', -NR²'COR²', -NR²'CONR²'₂ or -NR²'CSNR²'₂ preferably found in the α- or β-position relative to the carboxyl group are preferably prepared from the respective α- or β-amino acid. The α-amino acids used according to the invention are commercially obtainable, for example, from Novabiochem or Bachem. The β-amino acids can in some cases also be obtained from these companies or can be prepared according to the procedures of T.B. Johnson, Journal of the American Chemical Society, 1936, 58, or of V.A. Soloshonok, Tetrahedron Assymetry, 1995, 1601. These amino acids can be converted into the desired carboxyl-protected amino acid derivative, for example by protection of the amino group, subsequent protection of the carboxylic acid unit and subsequent deprotection of the amino group. Protective groups which can be used here for the amino group are all groups known for this purpose. Particularly preferred according to the invention is the use of a 9-fluorenylmethoxycarbonyl group (FMOC) as a protective group for the amino unit. The carboxylic acid group is protected or derivatized as described above. The carboxyl-protected α- or β-amino acids accessible in this way are reacted with a suitable sulphonating, carbamoylating or acylating reagent in order to obtain the corresponding sulphonamide, carbamate or amide derivatives. A sulphonating reagent used is preferably a sulphonyl chloride of the formula R²"-SO₂Cl or a carbamoyl chloride of the formula R²"-OCOCl, where R²" is a C₁₋₁₀-alkyl such as methyl, ethyl, propyl, isopropyl, butyl, isobutyl, t-butyl, pentyl, isopentyl, neopentyl, hexyl, heptyl, octyl, nonyl, decyl, cyclopropyl, cyclobutyl, cyclopentyl, cyclohexyl, or camphor-10-yl, an aryl such as phenyl, benzyl, tolyl, mesityl or substituted derivatives of these such as 2-chlorophenyl, 4-chlorophenyl, 2,5-dichlorophenyl, 4-trifluoromethylphenyl, 4-methoxyphenyl, 4-t-butylphenyl, 2,5-dimethylphenyl, 3-chlorophenyl, 3-aminophenyl, 4-aminophenyl, 2-methoxy-5-methylphenyl, 2,3,5,6-tetramethylphenyl, 2,3-dichlorophenyl, 2,6-dichlorophenyl, 2-naphthyl, 3-trifluoromethylphenyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline or 8-quinolinyl, or a heterocyclic analogue of the abovementioned cyclic radicals. Particularly preferably, R²" is a mesityl radical, a benzyl radical, a 2-chlorophenyl radical, a 4-chlorophenyl radical, a 2,5-dichlorophenyl radical, a 3-aminophenyl radical, a 4-aminophenyl radical, a 4-trifluoromethylphenyl radical or a camphor-10-yl radical. Instead of the abovementioned sulphonyl or carbamoyl chlorides, it is also possible to employ the corresponding fluorides, bromides or iodides. As acylating reagents, the appropriate carboxylic acid halides or carboxylic anhydrides are reacted with the amino group, the corresponding C₁₋₆-alkylcarbonyl chlorides such as the methyl-, ethyl-, propyl-, isopropyl-, butyl-, isobutyl-, t-butyl-, pentyl-, isopentyl-, neopentyl-, hexyl-, C₃₋₇-cycloalkyl- such as cyclopropyl-, cyclobutyl-, cyclopentyl-, cyclohexyl-, aryl- such as phenyl-, benzyl- or tolylcarboxylic acid chlorides or substituted derivatives thereof being preferred according to the invention. For the preparation of the urea or thiourea radicals, the amino group is preferably first reacted with a carbonic acid or thiocarbonic acid derivative such as a chloroformic acid ester or thiophosgene and then with a suitable amine NHR²'₂. The above reactions and their implementation are well known to the person skilled in the art and are described in detail in standard works such as, for example, Houben-Weyl, Methoden der organischen Chemie [Methods of Organic Chemistry], Georg Thieme Verlag, Stuttgart.

The starting compounds to be employed according to the above preferred embodiment have a terminal phenyl unit which must carry at least one substituent L. This substituent L must be substitutable by another phenyl group by means of one of the known aryl-aryl coupling processes. According to the present invention, L can be equal to -H, -F, -Cl, -Br, -I, -SCN, -N₂⁺ or an organometallic radical. Preferred organometallic radicals which may be mentioned are, for example, a magnesium, copper, boron, tin, lithium or lithium cuprate radical.

In addition to the radicals V and L, the terminal phenyl unit can have one or more further substituents, preferably one or more alkoxy radicals, particularly preferably one or more methoxy radicals.

If the appropriate starting compounds are not commercially obtainable, the terminal phenyl unit can be connected to the appropriate carboxylic acid derivative by standard processes such as, for example, a Friedel-Crafts alkylation, Friedel-Crafts acylation or by organometallic synthesis processes such as, for example, a palladium-assisted coupling, which are optionally followed by further derivatization steps which are known to the person skilled in the art and are described in detail in standard works such as, for example, Houben-Weyl, Methoden der organischen Chemie [Methods of Organic Chemistry], Georg Thieme Verlag, Stuttgart.

With respect to the radicals V and L, the terminal phenyl unit can be 1,3- or 1,4-substituted. Each of these isomers is, if not commercially obtainable, accessible in the manner known to the person skilled in the art.

According to a further preferred embodiment, the process for the preparation of compounds of the formula (II) starts from the following carboxylic acid derivatives:

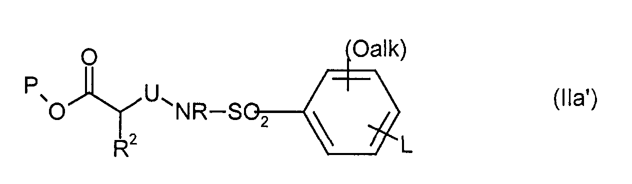

In this case, P and R² are as described above and can be introduced in the manner explained above if they are not already contained in the commercial starting compound. U represents an optionally substituted alkylene group and preferably an optionally substituted C₁₋₃-alkylene group. With respect to the possible substituents on U, reference is made to the above explanations for the compounds according to the invention.

In the case in which U is an optionally substituted methylene group, the preparation of the compound shown above starts from the optionally additionally substituted 3-aminopropanoic acid and this is reacted with an arylsulphonyl halide, preferably an arylsulphonyl chloride. The arylsulphonyl chloride is selected according to the desired presence and position of the radicals L and Oalk, L having the same meaning as described above and Oalk representing one or more alkoxy radicals, preferably one or more methoxy radicals. The preferred aryl sulphonyl halides are commercially obtainable or can be prepared by standard reactions familiar to the person skilled in the art. The above reactions and their implementation are well known to the person skilled in the art and described in detail in standard works such as, for example, Houben-Weyl, Methoden der organischen Chemie [Methods of Organic Chemistry], Georg Thieme Verlag, Stuttgart.

The biphenyl nucleus is produced in all embodiments according to the invention by an aryl-aryl coupling. Formally, in this connection the radical L on the terminal phenyl group of the carboxylic acid derivative serving as a starting compound is replaced by a phenyl compound of the following formula

M-B-W-D (IIb)

where
- M: is -H, -I, -N₂⁺, -COOOCOBNO₂ or an organometallic radical;
- B: is a phenylene group which is 1,3- or 1,4-substituted with respect to M and W-D and optionally contains additional radicals;
- W: is as defined above;
- D: is -NO₂, -NH₂ or -CHO;

Possible coupling reactions are, for example, the reaction of two unsubstituted phenyl groups (i.e. L and M are equal to hydrogen) in the presence of AlCl₃ and an acid (Scholl reaction), the coupling of two phenyl iodides in the presence of copper (Ullmann reaction), the reaction of the unsubstituted carboxylic acid derivative with a phenyldiazonium compound under basic conditions (Gomberg-Bachmann reaction) or coupling with involvement of organometallic reagents. In this connection, the coupling of two phenyl-Grignard compounds in the presence of thallium bromide, the coupling of two organoboron compounds in the presence of silver nitrate and sodium hydroxide, the reaction of a diphenyllithium cuprate in the presence of oxygen and palladium-assisted couplings of a phenyl halide by an organometallic phenyl compound are worthy of mention. The implementation of these reactions is described in detail in standard works such as Houben-Weyl, Methoden der organischen Chemie [Methods of Organic Chemistry], Thieme-Verlag, Stuttgart. The choice of the coupling reaction is directed by the presence of optionally interfering or sensitive substances in the reactants. For the preferred radicals of the formula (II) according to the invention, however, it has proven particularly advantageous to prepare the biphenyl nucleus by coupling of a phenyl halide with an organometallic phenyl compound in the presence of a palladium-(II) compound and triphenylphosphane.

The phenyl halide used here can be the corresponding phenyl fluoride, chloride, bromide or iodide, the corresponding bromide being particularly preferred. The organometallic phenyl compound used is preferably a substance in which a metallic element such as, for example, zinc, magnesium, boron, lithium, copper, tin or another element conventionally used for these purposes is bonded directly to the aryl ring. According to the invention, organoboron compounds are particularly preferred. Further substituents can additionally be bonded to the aryl ring in addition to the radical -W-D and the metallic element. Preferably, these substituents are one or more alkyl radicals, preferably a C₁₋₆-alkyl radical such as methyl, ethyl, propyl, isopropyl, butyl, isobutyl, t-butyl, pentyl, isopentyl, neopentyl, hexyl, a C₃₋₇-cycloalkyl radical such as cyclopropyl, cyclobutyl, cyclopentyl, cyclohexyl, and particularly preferably one or more methyl groups. If W is present, i.e. the radical D is bonded to the phenyl ring B via an optionally substituted alkylene group, the length of the main chain of this alkylene chain must be selected for the reasons described above such that in the resulting compound of the formula (IIc) not more than 6 atoms are present between the terminal carboxyl unit and the radical D in addition to the biphenyl nucleus.

Particularly preferred aryl reagents according to the invention are 3-nitrobenzeneboronic acid or 3-formylbenzeneboronic acid.

The radical D introduced into the compound is converted into an amino group, if it is not already an amino group. In the case in which D is a nitro group, this is reduced to the corresponding amino group by conventional reducing agents such as, for example, tin chloride. In the case in which D is an aldehyde group, the conversion into the amino group is carried out by reaction with an amine under reducing conditions, for example in the presence of an orthoester and of a reducing agent such as a metal hydride, for example a borohydride. The amino group thus formed can subsequently be derivatized, for example by reaction with, for example, alkyl or cycloalkyl halides. With respect to the preferred substituents which can be introduced in this way on the nitrogen atom, reference is made to the above description of the radicals of the formula (II) according to the invention.
According to a preferred embodiment of the present invention, the synthesis of the radicals of the formula (II) according to the invention is carried out on a solid phase such as a polystyrene resin, particularly preferably a commercially obtainable Wang polystyrene resin. In this connection, the resin is first swollen in a solvent such as dimethylformamide (DMF). The appropriate carboxylic acid serving as a starting compound is then bonded to the resin by standard processes. For example, the bonding of the carboxylic acid to the resin can be carried out in the presence of a base such as pyridine and a reagent activating the carboxyl unit, such as an acid halide, for example dichlorobenzoyl chloride, in a solvent such as dimethylformamide (DMF). However, other reagents conventionally used for this purpose can also be employed. The reaction mixture is stirred at room temperature and normal pressure for at least 2 hours, preferably 12 hours, particularly preferably approximately 24 hours, the carboxylic acid being employed in an excess, preferably in a two- to three-fold excess, with respect to the loading of the solid phase.

After removal of reagents which may be unreacted, if desired a derivatization of the carboxylic acid bonded to the resin can be carried out without this previously needing to be removed from the resin. According to a preferred embodiment according to the invention, for example, an amino acid as described above whose amino group is protected is bonded to the solid phase and then, after liberation of the amino group, a substituent is introduced into the latter. The amino group is preferably sulphonylated or carbamoylated. For this, the amino acid bonded to the solid phase is treated with an excess of a solution of the appropriate sulphonylating or carbamoylating agent, preferably a two- to four-fold excess, particularly preferably an approximately three-fold excess, in a solvent such as, for example, tetrahydrofuran (THF) in the presence of an auxiliary base such as diisopropylethylamine and the reaction mixture is stirred at room temperature and normal pressure for at least 2 hours, preferably 12 hours, particularly preferably approximately 24 hours. The sulphonamide or carbamate obtained does not have to be removed from the resin, but can immediately be reacted further after removal of unreacted reactants which are possibly present.

The aryl-aryl coupling is preferably carried out according to the invention by treating the optionally derivatized, for example sulphonylated or carbamoylated as described above, carboxylic acid bonded to the solid phase in aqueous medium in the presence of a base such as sodium carbonate with the appropriate aryl coupling reagent of the formula (IIb) and a catalyst conventionally used for this purpose, for example a palladium-(II) salt, preferably bis-(triphenylphosphane)-palladium-(II) chloride in combination with triphenylphosphane. In this connection, preferably an approximately 3- to 8-fold, preferably an approximately 4- to 6-fold, excess of the aryl coupling agent is employed, which according to the invention is in particular 3-nitrobenzeneboronic acid or 3-formylbenzeneboronic acid, and catalytically active amounts of the palladium compound, for example an approximately 10-fold excess with respect to the carboxylic acid, and the reaction mixture is heated after briefly stirring at room temperature, for example for 5 to 10 minutes, for approximately 2-24 hours, preferably 6-24 hours and particularly preferably 12-24 hours at a temperature in the range from 40 to 110°C, preferably 50 to 100°C and particularly preferably 60 to 90°C. The biphenyl compound obtained can immediately be reacted further without purification after unreacted reactants which may be present have been removed by washing with an acidic solution, for example a hydrochloric acid solution.

If the radical D is a nitro group, its conversion into an amino group according to the invention is preferably carried out by addition of a customary reducing agent such as tin-(II) chloride to the intermediate obtained as above bonded to the solid phase, if appropriate in the presence of solvents such as N-methylpyrrolidone (NMP) by stirring the reaction mixture at room temperature and normal pressure for at least 2 hours, preferably 12 hours, particularly preferably approximately 24 hours.

If the radical D is an aldehyde group, its conversion into an amino group is carried out by reductive amination. For this, the intermediate obtained as above and bonded to the solid phase is treated with an approximately 3- to 6-fold, preferably a 4- to 5-fold, excess of an amine in the presence of a neutralizing agent such as diisopropylethylamine and of an orthoester which is present in an approximately 6-to 10-fold excess. After stirring at room temperature for a number of hours, preferably 1 to 3 hours, an approximately 3- to 6-fold, preferably 4- to 5-fold, excess of an acidic solution of a metal hydride such as, for example, tetrabutylammonium borohydride is added to the reaction mixture and it is again stirred for a number of hours, preferably 12-24 hours, at room temperature.

The product obtained above can optionally be reacted further by derivatization of the radical D representing an amino group of the compound of the formula (IIc) or introduction of further substituents onto nitrogen atoms present in the molecule or directly removed from the resin. Removal from the resin is carried out in a conventional manner in an acidic medium. The product removed from the resin can be purified by known purification processes such as, for example, chromatographic processes after removal of solvents which may be present.

Furthermore, the radical D representing an amino group of the compound of the formula (IIc) can be converted into an amide group, urea group, thioamide group, thiourea group, amidine group or guanidine group. These structural units can be prepared by standard reactions familiar to the person skilled in the art, such as are described, for example, in Houben-Weyl, Methoden der organischen Chemie [Methods of Organic Chemistry], Georg Thieme Verlag, Stuttgart.

It is particularly preferred according to the invention to convert the radical D representing an amino group of the compound of the formula (IIc) into a urea or thiourea unit. For this, the above amino group of the carboxylic acid bonded to the solid phase is first preferably reacted with a 2- to 5-fold, preferably 3- to 4-fold, excess of a carbonic acid ester or thiocarbonic acid ester derivative in an inert solvent such as tetrahydrofuran (THF), dichloromethane or a mixture of both (preferably a 1:1 mixture) at room temperature and stirring for approximately 1 hour, preferably approximately 45 minutes. The carbonic acid ester or thiocarbonic acid ester derivative used can preferably be phosgene, triphosgene, thiophosgene or chloroformic acid esters, commercially available chloroformic acid esters being preferred for the preparation of the urea derivatives and thiophosgene being preferred for the preparation of the thiourea derivatives.

The carbamates or isothiocyanates formed in this way can be converted into the corresponding urea and thiourea derivatives by reaction with suitable amines. The amines used can be substances of the formula HNRR', where R and R' independently of one another or simultaneously can be hydrogen, a substituted or. unsubstituted alkyl or cycloalkyl radical, a substituted or unsubstituted aryl radical, a saturated or unsaturated, optionally substituted heterocyclic radical, an alkylamine radical, an alkylamide radical or can be connected to one another and together with the nitrogen atom can form an optionally substituted heterocyclic ring system which can be saturated or unsaturated and/or can contain further heteroatoms. With respect to the preferred radicals on the amine, reference is made to the above description of the radicals of the formula (II) according to the invention. According to the invention, the carbamate or isothiocyanate bonded to a solid phase is preferably reacted with a distinct excess of amine, preferably a 3- to 10-fold excess and particularly preferably a 5- to 10-fold excess, at room temperature with stirring for approximately 1 to 5 hours, preferably approximately 2 to 3 hours, in the presence of an auxiliary base such as diisopropylethylamine in an inert solvent such as dimethylformamide (DMF).

The radical of the formula (III) addressing αᵥβ₃ integrin receptors can be prepared from commercially obtainable starting compounds via the following steps:

The essential steps of the preparation process according to the invention are the reaction of a β-amino acid of the formula (IIIa) 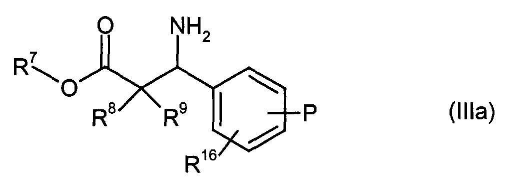 where
- P: is -(CH₂)ₘNO₂, -(CH₂)ₘO-C₁₋₆-alkyl, -(CH₂)ₘSO₂P', -(CH₂)ₘCOP', -(CH₂)ₘCH₂O-C₁₋₆-alkyl, where m is in each case an integer of 0 or 1;
- P': is -OH, -O-C₁₋₆-alkyl,
and the other radicals are as defined above, where R⁷ can additionally be a solid phase conventionally used for carrying out a solid-phase reaction;
with a compound R¹⁰-A to give a compound of the formula (IIIb) 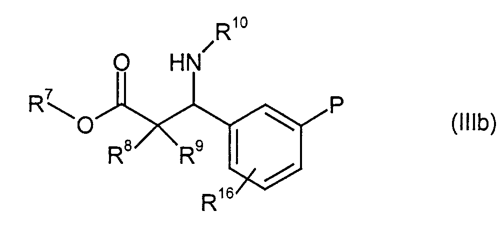 where
- R¹⁰: is -SO₂R¹⁰', -COOR¹⁰'' or -COR¹⁰';
- R¹⁰' and R¹⁰": are as defined above;
- A: is -Cl, -Br, -I, -O-triflyl, -O-tosyl, -O-C₁₋₆-alkyl, -O-CO-C₁₋₆-alkyl, -O-CO-O-C₁₋₆-alkyl, -OC(CH₃)=CH₂;
and the other radicals are as defined above;
the conversion of the radical P into the radical Q,
where
- Q: is -(CH₂)ₘNH₂, -(CH₂)ₘOH, -(CH₂)ₘCH₂OH, -(CH₂)ₘSO₂A, -(CH₂)ₘCOA,
- A: is as defined above;
- m: is an integer of 0 or 1;
the reaction of the compound (IIIb) obtained above with a compound of the formula (IIIc) 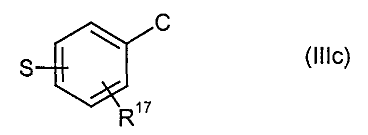 where
- S: is ASO₂(CH₂)ₙ-, NH₂(CH₂)ₙ-, ACO(CH₂)ₙ-, HOCH₂(CH₂)ₙ-, M(CH₂)ₙ-, MCH₂(CH₂)ₙ-, HSCH₂(CH₂)ₙ- or HS(CH₂)ₙ-,
where
n is an integer of 0 or 1;
M is a radical including Mg, Li, Cd or Sn;
A is as defined above; and
C is -NO₂ or 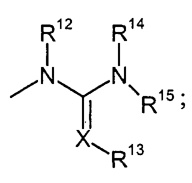 and
X, R¹², R¹³, R¹⁴ and R¹⁵ are as defined above;
to give a compound of the formula (IIId) 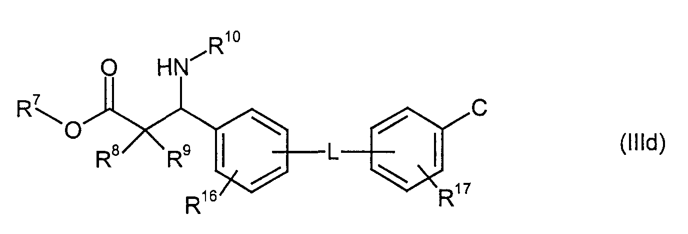 where the radicals are as defined above;
if appropriate the conversion of C, if C is a nitro group, into an optionally cyclic urea, thiourea or guanidine unit with retention of the radical (III); and
if appropriate the removal of protective groups and/or derivatization of nitrogen atoms present at preferred points of time in the preparation process and/or conversion of the compound obtained into the free acid and/or conversion of the compound obtained into one of its physiological salts by reaction with an inorganic or organic base or acid.

The β-amino acid derivatives of the formula (IIIa) are either commercially obtainable or are accessible in a simple manner by standard chemical processes, such as are known to any person skilled in the art and are described in standard works such as Houben-Weyl, Methoden der organischen Chemie [Methods of Organic Chemistry], Georg Thieme-Verlag, Stuttgart. In particular, reference is made to the preparation processes for β-amino acid derivatives described by Rodionow et al., J. Am. Chem. Soc. 51, 1929, 844-846, Kunz et al., Angew. Chem. 101, 1989, 1042-1043 and Ishihara et al., Bull. Chem. Soc. Jpn., 68, 6, 1995, 1721-1730.

According to a preferred embodiment of the present invention, the β-amino acid derivatives of the formula (IIIa) are obtained by reaction of malonic acid with a benzaldehyde derivative of the formula (IIIa') 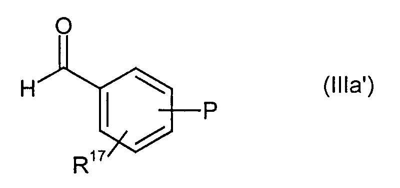 where R¹⁷ and P are as defined above, in the presence of ammonia, ammonium compounds or amines. Instead of malonic acid, an ester, if appropriate with addition of a base conventionally employed for these purposes, such as NaH or a sodium alkoxide, preferably sodium methoxide or sodium ethoxide, can also be used. Preferably, an ammonium compound such as, for example, ammonium acetate is employed as the nitrogen compound.

The benzaldehyde derivatives (IIIa') are either commercially obtainable or are accessible in a simple manner by standard chemical processes, such as are known to any person skilled in the art and are described in standard works such as Houben-Weyl, Methoden der organischen Chemie [Methods of Organic Chemistry], Georg Thieme-Verlag, Stuttgart.

According to a preferred embodiment of the present invention, a nitrobenzaldehyde derivative such as 3- or 4-nitrobenzaldehyde or an alkoxybenzaldehyde derivative such as 3- or 4-methoxybenzaldehyde is employed as the compound of the formula (IIIa').

According to a preferred embodiment of the present invention, the β-amino acid of the formula (IIIa) is obtained by reaction of approximately equimolar amounts of malonic acid, ammonium acetate and 3-nitrobenzaldehyde or 3-methoxybenzaldehyde in a solvent such as isopropanol with heating for a number of hours, preferably 2 to 6 hours, at 50 to 110°C, preferably with reflux of the solvent, in the surrounding atmosphere (i.e. in the air and under normal pressure).

For the following reaction steps, the carboxyl group is blocked by a conventional protective group P. Protective groups of this type are known to the person skilled in the art and do not have to be expressly mentioned here. The carboxyl group is particularly preferably esterified, where P is a C₁₋₆-alkyl such as, for example, methyl, ethyl, propyl, isopropyl, butyl, isobutyl, t-butyl, pentyl, isopentyl, neopentyl, hexyl, a C₃₋₇-cycloalkyl such as, for example, cyclopropyl, cyclopropylmethyl, cyclobutyl, cyclopentyl, cyclohexyl, an aryl such as, for example, phenyl, benzyl, tolyl or a substituted derivative thereof.

Furthermore, the preparation process according to the invention for the radicals of the formula (III) can be carried out on a solid phase as described above for radicals of the formula (II). In this case, the carboxyl radical can be connected to any solid phase conventionally used for reactions of this type, such as a polystyrene resin, for example a Wang polystyrene resin.

According to a preferred embodiment according to the invention, the carboxyl group of the above β-amino acid is esterified by reaction with an alcohol such as ethanol or a polymer conventionally used for carrying out a solid-phase reaction. This can be carried out under conditions known to the person skilled in the art, such as acid catalysis and, if appropriate, addition of a dehydrating agent such as dicyclohexylcarbodiimide. Preferably, however, the β-amino acid is suspended in the appropriate alcohol present in an excess, such as ethanol, HCl is passed through for a period of approximately 30 minutes to approximately 2 hours and the mixture is then heated in a surrounding atmosphere for a number of hours, preferably approximately 1 to 6 hours and particularly preferably approximately 3 to 5 hours, at approximately 50 to approximately 100°C, preferably under reflux of the alcohol.

The carboxyl-protected β-amino acids accessible in this way are reacted with a suitable sulphonating, carbamoylating or acylating reagent in order to obtain the corresponding sulphonamide, carbamate or amide derivatives. The sulphonating reagent used is preferably a sulphonyl chloride of the formula R¹⁰"-SO₂Cl or a carbamoyl chloride of the formula R¹⁰"-OCOCl, where R¹⁰" is a C₁₋₁₀-alkyl such as methyl, ethyl, propyl, isopropyl, butyl, isobutyl, t-butyl, pentyl, isopentyl, neopentyl, hexyl, heptyl, octyl, nonyl, decyl, cyclopropyl, cyclobutyl, cyclopentyl, cyclohexyl, or camphor-10-yl, an aryl such as phenyl, benzyl, tolyl, mesityl or substituted derivatives of these such as -C₆H₂(CH₃)₃, -C₆(CH₃)₅, -CH₂C₆H₂(CH₃)₃, 2-chlorophenyl, 3-chlorophenyl, 4-chlorophenyl, 3-aminophenyl, 4-aminophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 4-chlorophenylmethyl, 2,4-dichlorophenylmethyl, 2,6-dichlorophenylmethyl, 2-methoxycarbonylphenylmethyl, 3-trifluoromethylphenyl, 4-trifluoromethylphenyl, 3,5-bis(trifluoromethyl)phenyl, 4-trifluoromethoxyphenyl, phenylmethyl, 2-acetamido-4-methyl-thiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methyl-benzothiazol-2-yl, N-methoxycarbonyl-piperidin-3-yl, thiophen-2-yl, isoxazol-5-yl, 2-chloropyridin-3-yl, pyridin-3-yl, 5-methylisoxazol-3-yl, 1-adamantyl, 4-chlorophenoxymethyl, 2,2-dimethylethenyl, 2-chloropyridin-5-methyl, 5,7-dimethyl-1,3,4-triazaindolizin-2-yl, (S)-camphan-1-yl, (R)-camphan-1-yl, 8-quinolinyl, or a heterocyclic analogue of the abovementioned cyclic radicals. Instead of the abovementioned sulphonyl or carbamoyl chlorides, it is also possible to employ the corresponding fluorides, bromides or iodides. As acylating reagent, the appropriate carboxylic acid halides or carboxylic acid anhydrides are reacted with the amino group, the appropriate C₁₋₆-alkyl carboxylic acid chlorides such as methyl-, ethyl-, propyl-, isopropyl-, butyl-, isobutyl-, t-butyl-, pentyl-, isopentyl-, neopentyl-, hexyl-, C₃₋₇-cycloalkyl such as cyclopropyl-, cyclobutyl-, cyclopentyl-, cyclohexyl-, aryl such as phenyl-, benzyl-, tolylcarboxylic acid chlorides or substituted derivatives thereof being preferred according to the invention. For the preparation of the urea or thiourea radicals, the amino group is preferably first reacted with a carbonic acid or thiocarbonic acid derivative such as a chloroformic acid ester or thiophosgene and then with a desired amine. The above reactions and their implementation are well known to the person skilled in the art and are described in detail in standard works such as, for example, Houben-Weyl, Methoden der organischen Chemie [Methods of Organic Chemistry], Georg Thieme Verlag, Stuttgart.

According to a preferred embodiment of the invention, the carboxyl-protected β-amino acid of the formula (IIIa) is treated with an equimolar amount or a slight excess of the appropriate sulphonylating agent, for example phenylsulphonyl chloride, or acylating agent, for example mesitylacetyl chloride, with cooling, preferably at 0°C, in a solvent such as pyridine or dioxane in a surrounding atmosphere in the presence of a base such as an amine, preferably triethylamine or diisopropylethylamine, and the mixture is stirred at this temperature for a period of approximately 10 minutes to approximately 2 hours. In the case of sulphonylation, this is followed by stirring at room temperature for a number of hours, preferably approximately 2 to 6 hours.
Before the synthesis of the linker group L, the radical P of the compound of the formula (IIIb) must be converted into a group Q which can participate in a nucleophilic substitution either as a nucleophilic reagent or as a substrate. If P includes a nitro group, this will be reduced to the corresponding amino group, which according to the present invention can preferably be carried out by addition of tin(II) chloride to a solution of the compound of the formula (IIIb) in a solvent such as ethanol and subsequent heating to approximately 50 to 110°C, preferably under reflux of the solvent, for a number of hours, preferably approximately 1 to 4 hours, in a surrounding atmosphere. If P includes an ether group, the liberation of the corresponding hydroxyl group is preferably carried out by addition of a Lewis acid such as boron tribromide in a solvent such as dichloromethane with cooling, preferably at -78°C, and subsequent stirring for a number of hours, preferably 6 to 24 hours, at room temperature. If P includes a sulphonic acid or carboxylic acid group, a conversion into the corresponding sulphonyl or carboxylic acid halide is preferably carried out. This can be carried out in a manner known to the person skilled in the art, for example by reaction of the corresponding sulphonic or carboxylic acid with thionyl chloride.

The compound prepared in this way is then reacted with a compound of the formula (IIIc) 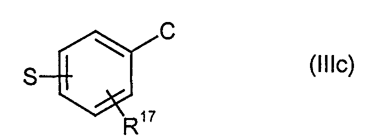 where
- S: is ASO₂(CH₂)ₙ-, NH₂(CH₂)ₙ-, ACO(CH₂)ₙ-, HOCH₂(CH₂)ₙ-, M(CH₂)ₙ-, MCH₂(CH₂)ₙ-, HSCH₂(CH₂)ₙ- or HS(CH₂)ₙ-,
where
n is an integer of 0 or 1;
M is a radical including Mg, Li, Cd or Sn;
A is as defined above; and
C is -NO₂ or 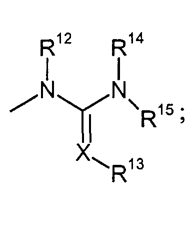 and
X, R¹², R¹³, R¹⁴ and R¹⁵ are as defined above;
to give a compound of the formula (IIId) 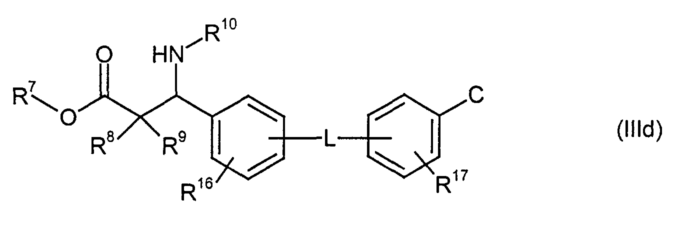 where the radicals are as defined above. This reaction formally represents the substitution of a leaving group in one of the starting compounds by a nucleophilic unit in the other starting compound in each case.

According to a preferred embodiment of the present invention, the reactants are mixed together in approximately equimolar amounts in the presence of a base such as pyridine or sodium hydride and, if appropriate, in a solvent such as, for example, tetrahydrofuran (THF) or dimethylformamide (DMF) in a surrounding atmosphere at room temperature or with cooling, preferably at approximately 0°C, and stirred for a number of hours, preferably approximately 1 h to approximately 24 hours, at room temperature or with cooling, for example at 0°C.

The compounds of the formula (IIId) thus obtained are converted into the radicals of the formula (III) according to the invention by conversion of the terminal nitro group into an open-chain or cyclic guanidine, urea or thiourea unit.

For this, the nitro group is first converted according to the invention into an amino group, preferably by addition of a customary reducing agent such as tin-(II) chloride, if appropriate in the presence of solvents such as ethanol, by stirring the reaction mixture with heating at approximately 50 to 110°C, preferably under reflux of the solvent, in a surrounding atmosphere for approximately 2 hours.

The amino group thus obtained is then converted into a guanidine, urea or thiourea unit. For this, the above amino group is first preferably reacted with a carbonic acid ester or thiocarbonic acid ester derivative in a solvent such as dimethylformamide (DMF) in the presence of mercury-(II) chloride with cooling, preferably at approximately 0°C, and stirring for approximately 10 minutes to approximately 3 hours with cooling, preferably at approximately 0°C, and if appropriate subsequently at room temperature. The carbonic acid ester or thiocarbonic acid ester derivative employed can preferably be phosgene, triphosgene, thiophosgene, chloroformic acid esters or thiopseudourea derivatives, commercially obtainable chloroformic acid esters being preferred for the preparation of the urea derivatives, thiophosgene being preferred for the preparation of the thiourea derivatives and thiopseudourea derivatives being preferred for the preparation of guanidine derivatives.

The carbamates or isothiocyanates formed in this way can be converted into the corresponding urea, thiourea and guanidine derivatives by reaction with appropriate amines. The amines used can be substances of the formula HNRR', where R and R' independently of one another or simultaneously can be hydrogen, a substituted or unsubstituted alkyl or cycloalkyl radical, a substituted or unsubstituted aryl radical, a saturated or unsaturated, optionally substituted heterocyclic radical, an alkylamine radical, an alkylamide radical or can be connected to one another and together with the nitrogen atom can form an optionally substituted heterocyclic ring system which can be saturated or unsaturated and/or can contain further heteroatoms. With respect to the preferred radicals on the amine, reference is made to the above description of the compounds according to the invention. According to the invention, the carbamate or isothiocyanate is preferably reacted with an amine at room temperature with stirring for approximately 1 to 5 hours, preferably approximately 2 to 3 hours, in the presence of an auxiliary base such as diisopropylethylamine in a solvent such as dimethylformamide (DMF). In the case of the preparation of cyclic guanidine derivatives, the corresponding isothiocyanate is preferably first heated in ethanol for a number of hours, preferably approximately 12 to 24 hours, and then heated with a diamine such as diaminoethane in a solvent such as toluene, dimethylformamide (DMF) or a mixture of both.

According to a further preferred embodiment of the present invention, it is also possible to generate the above guanidine, urea or thiourea group on the compound of the formula (IIIc) in the above manner and then to react the compound of the formula (IIIc) thus obtained with the compound of the formula (IIIb) in the manner described above.

The compounds obtained according to the process explained above can furthermore be derivatized by removal of protective groups which may be present, further substitution of nitrogen atoms present at preferred positions in the preparation process and/or conversion of the compound obtained into the free acid and/or its physiologically acceptable salts. For example, the t-butoxymethoxycarbonyl groups conventionally used as protective groups for nitrogen atoms are removed in an acidic medium, for example by addition of trifluoroacetic acid. Suitable alkylating agents for derivatization of nitrogen atoms are reagents conventionally used for this purpose in this step, to which, for example, a substituted or unsubstituted alkyl or cycloalkyl radical, a substituted or unsubstituted aryl radical or a saturated or unsaturated, optionally substituted heterocyclic radical can be bonded to the corresponding nitrogen atom. With respect to the substituents preferably bonded to the respective nitrogen atoms, reference is made to the above description of the compounds according to the invention. The above reactions and their implementation are well known to the person skilled in the art and are described in detail in standard works such as, for example, Houben-Weyl, Methoden der organischen Chemie [Methods of Organic Chemistry], Georg Thieme Verlag, Stuttgart.

The ester derivatives according to the invention can be converted into the corresponding free carboxylic acids in a conventional manner, such as, for example, by basic ester hydrolysis.

If desired, the compounds according to the invention can be converted into their physiologically acceptable salts. This can be carried out either by reaction with an organic or inorganic base such as, for example, an alkali metal hydroxide or alkaline earth metal hydroxide such as KOH, NaOH, LiOH, Mg(OH)₂ or Ca(OH)₂, whereby the terminal carboxyl group is deprotonated and the corresponding carboxylate is formed, or by reaction with an organic or inorganic acid such as, for example, hydrochloric acid, sulphuric acid, phosphoric acid, mandelic acid, oleic acid, linoleic acid or p-toluenesulphonic acid, whereby one or more of the above nitrogen atoms are protonated.

The radical of the formula (IV) addressing αᵥβ₃ integrin receptors can be prepared from commercially obtainable starting compounds.

The conjugates according to the invention can be used as active compound components for the production of medicaments against carcinomatous disorders. For this, they can be converted into the customary formulations such as tablets, coated tablets, aerosols, pills, granules, syrups, emulsions, suspensions and solutions in a known manner using inert, non-toxic, pharmaceutically suitable excipients or solvents. Preferably, the compounds according to the invention are used here in an amount such that their concentration in the total mixture is approximately 0.5 to approximately 90% by weight, the concentration, inter alia, being dependent on the corresponding indication of the medicament.

The abovementioned formulations are produced, for example, by extending the active compounds with solvents and/or excipients having the above properties, where, if appropriate, additionally emulsifiers or dispersants and, in the case of water as the solvent, alternatively an organic solvent, have to be added.

The medicaments according to the invention can be administered in a customary manner.

The present invention is illustrated below with the aid of non-restricting examples and comparison examples.

### Examples

In the examples below, all quantitative data, if not stated otherwise, relate to percentages by weight. The mass determinations were carried out by high-performance liquid chromatography-mass spectrometry (HPLC-MS) using the electron spray ionization (ESI) method or by FAB or MALDI mass spectroscopy.

### List of the abbreviations used

- HPLC: - high-performance liquid chromatography
- RP: - reverse phase
- ACN: - acetonitrile
- DMF: - dimethylformamide
- DCM: - dichloromethane
- THF: - tetrahydrofuran
- DIEA: - diisopropylethylamine (Hünig's base)
- NMP: - N-methylpyrrolidone
- TFA: - trifluoroacetic acid
- Fmoc: - fluorenyl-9-methoxycarbonyl
- RT: - room temperature
- MTBE: - methyl tert-butyl ether
- Boc: - tert-butoxycarbonyl
- TLC: - thin-layer chromatography
- DMAP: - dimethylaminopyridine
- DMSO: - dimethyl sulphoxide
- Abu: - γ-amino butyric acid

### I. Synthesis of starting materials

### I.1 20-O-L-Valyl-camptothecin trifluoroacetate

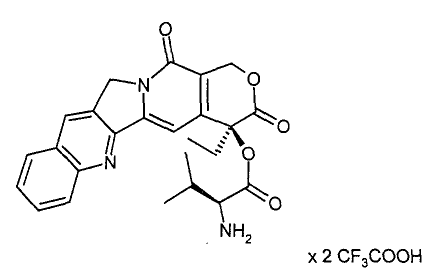

A suspension of 10 g (28.7 mmol) of 20(S)-camptothecin in 500 ml of absolute dichloromethane is treated with stirring with 14 g (2 eq.) of N-(tert-butoxycarbonyl)-valine-N-carboxyanhydride and 1 g of 4-(N,N-dimethylamino)-pyridine. After heating under reflux for 4 days, the mixture is concentrated in vacuo. The residue is stirred with 100 ml of MTBE for 20 min. 200 ml of petroleum ether are then added and the mixture is filtered. 14.9 g of the Boc-protected intermediate compound are obtained, which can contain small amounts of D-valine epimer which, however, can be removed without problems after removal of the protective group.

11.65 g of this Boc-protected intermediate compound are then stirred at 5°C for 1 h in a mixture of 300 ml of dichloromethane and 70 ml of anhydrous trifluoroacetic acid. After concentrating in vacuo to a small volume, the product is precipitated with diethyl ether and thoroughly washed with diethyl ether. The product is again precipitated from dichloromethane/methanol using diethyl ether. If appropriate, the crude product is again taken up in 40 ml of methanol, and the solution is treated with 120 ml of MTBE and cooled to 0°C. The precipitate is filtered off and 9.4 g (80%) of 20-O-(valyl)-camptothecin trifluoroacetate are obtained after drying.
[TLC: acetonitrile/water (20:1); R_{f}= 0.39].

### I.2 20-O-[L-Histidyl-L-valyl]-camptothecin bis-trifluoroacetate

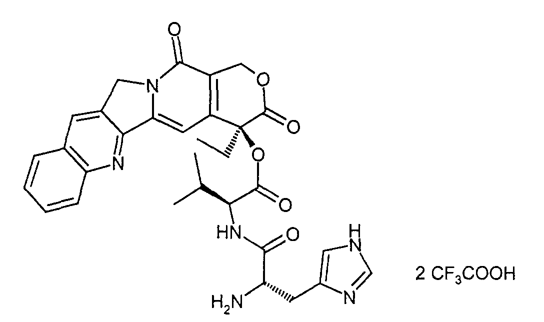

2 g (7.8 mmol) of benzyl N-tert-butoxycarbonyl-histidine are dissolved in 100 ml of DMF and cooled down to 0°C. 1.59 g (1.5 eq) of hydroxybenzotriazole and 1.8 g (1.2 eq) of N-(3-dimethylaminopropyl)-N'-ethylcarbodiimide hydrochloride are added and the mixture is stirrred at 0°C for 30 min. 3.65 g (6.5 mmol) of the compound from Example I.1 and 2.7 ml of Hünig's base are added. The coupling reaction is complete after 16 h. The reaction mixture is poured into 600 ml of MTBE. The precipitating product is collected and it is taken up in dichloromethane. The mixture is extracted twice with water and then the organic phase is dried and concentrated. MTBE is added and the precipitating product is filtered and dried in vacuo. Yield: 4.45g = quantitative; TLC: acetonitrile/water (10:1) R_{f}= 0.33.

4.45 g (6.5 mmol) of this Boc-protected intermediate compound are then stirred at 5°C for 1 hin a mixture of 60 ml of dichloromethane and 30 ml of anhydrous trifluoroacetic acid. After concentrating in vacuo, the product is taken up in dichloromethane/methanol, precipitated with MTBE and thoroughly washed with MTBE. The product is again precipitated from dichloromethane/methanol using MTBE. The precipitate is filtered off and 4.75 g (91%) of the target compound are obtained after drying.
[TLC: acetonitrile/water/glacial acetic acid 5/1/0.2 R_{f}= 0.3].

### I.3 N-tert-butoxycarbonyl-L-prolyl-L-leucyl-glycyl-L-leucin

Commercially available educts N-tert.Butoxycarbonyl-proline hydroxysuccinimide ester (687 mg; 2.2 mmol) and L-Leucyl-glycyl-L-leucin (602.8 mg; 2 mmol) are dissolved in 10 ml DMF, 1035 µl Ethyl-diisopropylamine are added and the mixture is sonificated for 18h. Subsequently, the solvent is removed in vacuo and the residue is dissolved in dichloromethane and filtered. The crude product is purified by flash chromatography at silicagel using acetonitrile/water 20:1 as eluent. Relevant fractions are collected and concentrated. The remaining residue is dissolved in dichloromethane and washed with citric acid. The organic layer is dried upon sodium sulfate and the solvent is removed. The product is obtained in a yield of 420mg (42%).
[ESI-MS: m/e = 499 = (M+H)⁺ ]

### I.4 N-tert-butoxycarbonyl-L-prolyl-L-leucyl-glycyl-L-leucyl-L-asparagine

This compound is synthesized by coupling of 1.3 with an asparagine derivative or via alternative routes following standard procedures as described in Houben Weyl; Methoden der Organischen Chemie; Vierte Auflage; Band XV Teil 1 und 2; Georg Thieme Verlag Stuttgart 1974, or in Hans-Dieter Jakubke and Hans Jeschkeit: Aminosäuren, Peptide, Proteine; Verlag Chemie, Weinheim 1982.
[TLC: acetonitrile/water/glacial acetic acid 5/1/0.2 R_{f}= 0.68].

### I.5 N-tert-butoxycarbonyl-L-prolyl-L-leucyl-glycyl-L-leucin-L-histidine

This compound is synthesized following standard procedures.
[TLC: acetonitrile/water/glacial acetic acid 5/1/0.2 R_{f}= 0.28].

### I.6 N-fluorenyl-9-methoxycarbonyl-L-prolyl-L-leucyl-glycyl-L-leucin-L-asparagine-L-glycine

This compound is synthesized following standard procedures.
[TLC: acetonitrile/water/glacial acetic acid 5/1/0.2 R_{f}= 0.55].

### II. Preparation of camptothecin petide conjugates

### II.1 20-O-[L-Prolyl-L-leucyl-glycyl-L-leucyl-L-histidyl-L-valyl]-camptothecin bis-trifluoroacetate

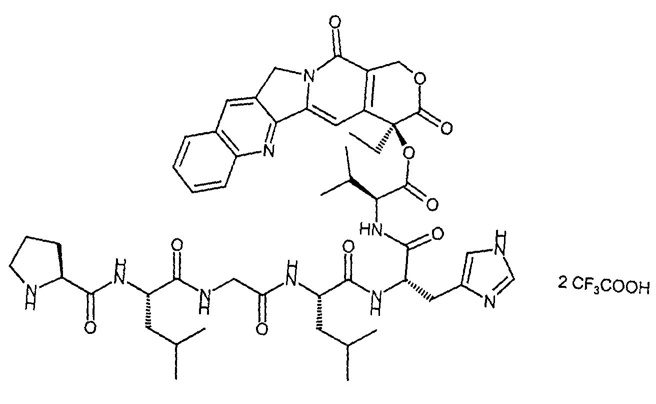

277.4 mg (0.57 mmol) of the compound I.3 are dissolved in 80 ml of DMF. 116 mg (0.86 mmol) of 1-hydroxy-1H-benzotriazole and 131.7 mg (0.69 mmol) of N-(3-dimethylaminopropyl)-N'-ethylcarbodiimide hydrochloride are added and furthermore 400 mg (0.57 mmol) of the compound from Example 1.2 and 222 mg of Hünig's base. The coupling reaction is complete after 2 h. The reation mixture is concentrated and the residue is treated with water. The crude product is purified by flash chromatography at silicagel using dichloromethane/-methanol/ammonia 17% 15/1/0.1 as eluent. Relevant fractions are collected and concentrated. The product is obtained in a yield of 472 mg (77%) [TLC: Acetonitrile/water 10/1 R_{f}= 0.2].

470 mg (0.44 mmol) of this Boc-protected intermediate compound are stirred at 5°C for 1 h in a mixture of 50 ml of dichloromethane and 10 ml of anhydrous trifluoroacetic acid. After concentrating in vacuo, the product is taken up in dichloromethane/methanol, precipitated with diethyl ether and filtered off. The residue is again precipitated from dichloromethane/methanol using diethyl ether. The precipitate is filtered off and, after drying, 432 mg (91%) of the target compound are obtained.
[TLC: acetonitrile/water/glacial acetic acid 5/1/0.2 R_{f}= 0.15].

In analogy to I.1, further camptothecin amino acid conjugates were prepared by reaction of camptothecin with partially protected amino derivatives. Subsequently, the peptide chain is elongated either by attachment of single amino acid derivatives and subsequent deprotection of the amino terminus or by fragment condensation as exemplified in II.1 or in a combination of both. If appropriate, protective groups are removed. Coupling-, protection- and deprotection-steps are performed according to known methods as reported in: Houben Weyl; Methoden der Organischen Chemie; Vierte Auflage; Band XV Teil 1 und 2; Georg Thieme Verlag Stuttgart 1974; Hans-Dieter Jakubke and Hans Jeschkeit: Aminosäuren, Peptide, Proteine; Verlag Chemie, Weinheim 1982. The conjugates prepared according to this method are shown below:

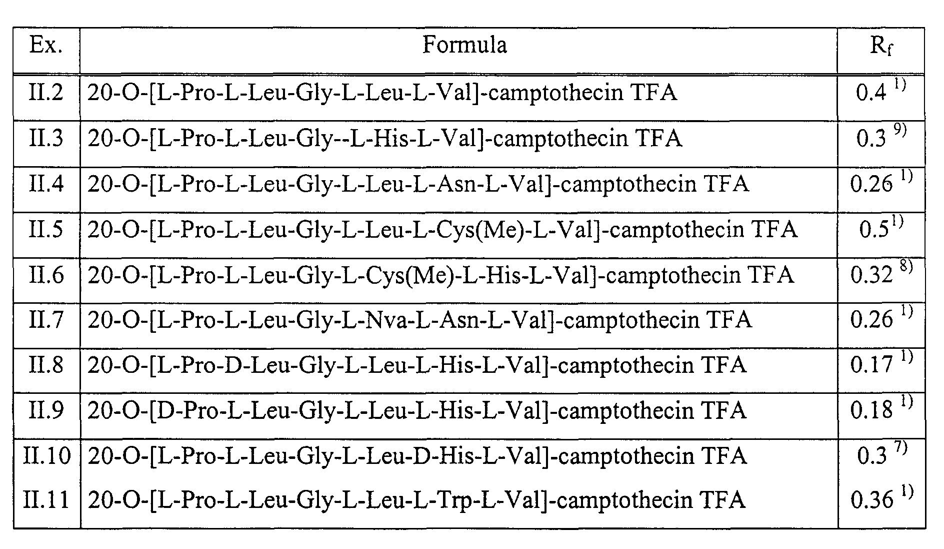

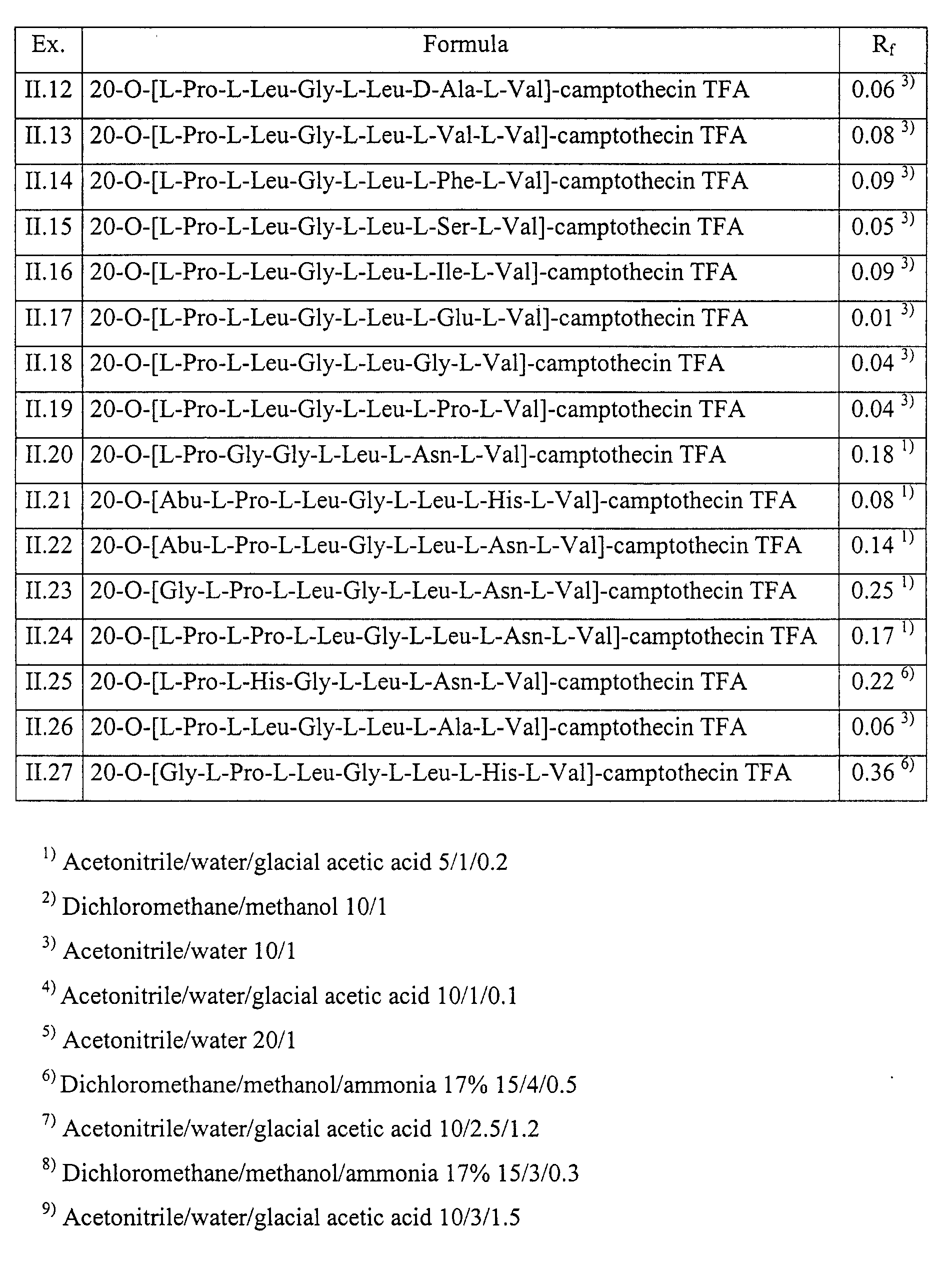

### III. Preparation of integrin ligands

### Example III. 1

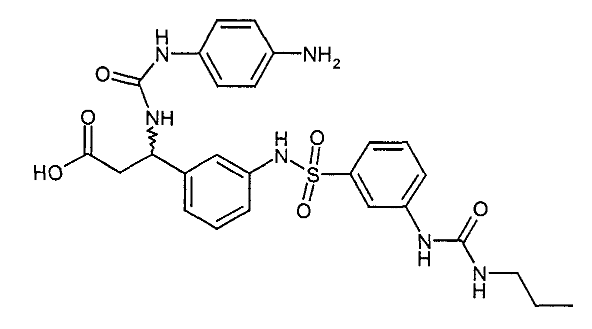

### III.1-a: 3-Amino-3-[3-(propylaminocarbonylamino-phenyl-sulphonylamino)-phenyl]-propionic acid

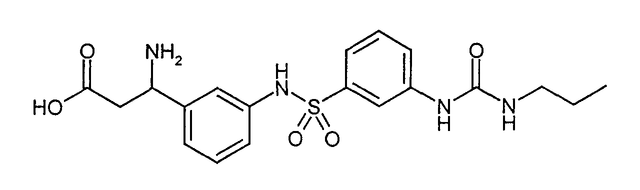

1.2 g of polystyrene Wang resin (loading 1.08 mmol/g) are swollen in DMF. The solvent is filtered off with suction and a solution of 841 mg of (3R,S)-3-(9-fluorenylmethoxycarbonylamino)-3-(3-nitrophenyl)-propionic acid (amino acid reagent) in 15 ml of DMF are added. After shaking at room temperature for 15 min, the suspension is treated with 350 µl of pyridine and 540 mg of 2,6-dichlorobenzoyl chloride. It is shaken overnight at room temperature. The resin is then washed with DMF, MeOH and DCM.

The resin is treated with a solution of 5400 mg of tin(II) chloride dihydrate in 12 ml of NMP and shaken overnight at room temperature. The resin is then washed with NMP, MeOH, THF and DCM.

The resin is treated with a solution of 450 µl of DIEA in 500 µl of THF and a solution of 430 mg of 3-nitrobenzenesulphonyl chloride in 500 µl of THF. It is shaken overnight at room temperature. The resin is then washed with DMF, MeOH and THF.

The resin is treated with a solution of 5400 mg of tin(II) chloride dihydrate in 12 ml of NMP and shaken overnight at room temperature. The resin is then treated with NMP, MeOH, THF and DCM.

The resin is treated with a solution of 500 µl of DIEA in 12 ml of THF/DCM 1:1 and a solution of 2757 mg of 4-nitrophenylchloroformic acid ester in 12 ml of THF/DCM 1:1. After shaking at room temperature for 45 min, it is washed with THF and DMF and a solution of 943 mg of propylamine and 2780 µl of DIEA in 20 ml of NMP is added. After shaking for 10 h, the resin is washed with DMF, MeOH, THF and DCM.

For the removal of the product, the resin is shaken for 1 h with 12 ml of TFA/DCM and filtered off, and the filtrate is concentrated in vacuo.

### III.1: 3-(4-Aminophenylaminocarbonylamino)-3-[3-(3-propylaminocarbonylaminophenyl-sulphonylamino)-phenyl]-propionic acid

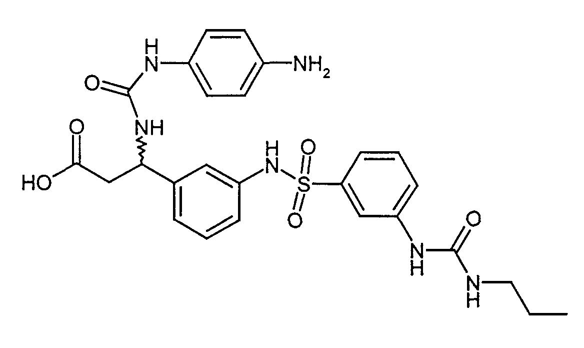

70 mg (0.166 mmol) of the compound III.1-a are stirred with 54 mg (2 eq) of 4-nitrophenyl isocyanate for 1 h in 10 ml of DMF. The mixture is concentrated and the residue is purified by flash chromatography on silica gel using dichloromethane/methanol/ammonia 17% strength (15:2:0.2). After precipitation from dichloromethane/methanol using ether, the intermediate a (29 mg; 30%) is obtained.

This is dissolved in methanol and hydrogenated over palladium/carbon. The catalyst is separated off, the solution is concentrated and the residue is lyophilized from dioxane/water. 18 mg (74%) of the target compound are obtained.

### III.2: Enantiomer A of III.1 (3-(4-Aminophenylaminocarbonylamino)-3-[3-(3-propylaminocarbonylamino-phenyl-sulphonylamino)-phenyl]-propionic acid)

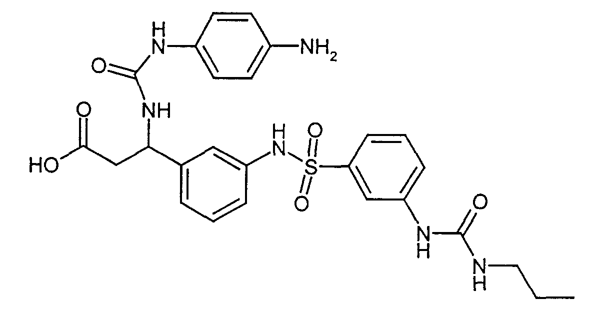

### III.2.a: 3-(Amino)-3-(3-nitrophenyl)propionic acid hydrochloride

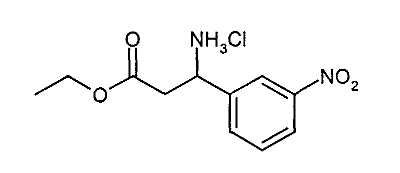

151 g of 3-nitrobenzaldehyde, 94 g of ammonium acetate and 127 g of malonic acid were heated under reflux for 5 h in 1 12-propanol. The solution was filtered and the precipitate was washed with 0.7 1 of hot 2-propanol. The crude product was dried in vacuo, suspended in 1.5 1 of water, treated with 1 N hydrochloric acid and filtered. The filtrate was concentrated to yield 146 g.
¹H-NMR (400 MHz, D₄-MeOH): 3.09 (m, 2 H), 4.88 (m, 1 H), 7.74 (t, 1 H), 7.90 (d, 1 H), 8.33 (d, 1 H), 8.43 (s, 1 H).

### III.2.b: Ethyl 3-(amino)-3-(3-nitrophenyl)propanoate hydrochloride

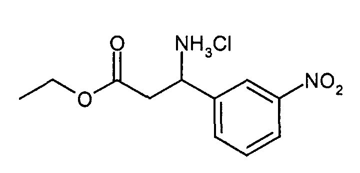

60 g 3-amino-3-(3-nitrophenyl)-propionic acid hydrochloride were suspended in 660 ml of ethanol and gaseous HCl was passed in the mixture for 1 h. The reaction mixture was then heated under reflux for 4 h and then cooled and concentrated. 62 g of a white solid were obtained.
¹H-NMR (400 MHz, D₄-MeOH): 1.22 (t, 3 H), 3.12 (dd, 1 H), 3.20 (dd, 1 H), 4.18 (q, 2 H), 4.95 (t, 1 H), 7.77 (t, 1 H), 7.94 (d, 1 H), 8.35 (d, 1 H), 8.43 (s, 1 H).

### III.2.c: Ethyl 3-{[(allyloxy)carbonyl]amino}-3-(3-nitrophenyl)propanoate

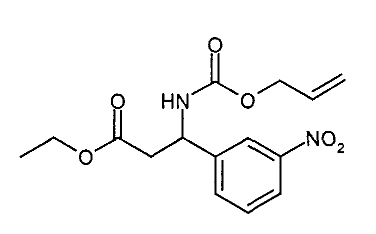

To 41.2 g ethyl 3-amino-3-(3-nitrophenyl)-propioniate hydrochloride in 350 ml dichloromethane 48.8 g diisopropylethylamine and 24.8 g allyloxycarbonyl chloride in 150 ml dichloromethane were added at 0°C. After stirring for 30 min the mixture was washed with water, dried over MgSO₄ and concentrated to give a white solid (yield: 56.4 g).
¹H-NMR (400 MHz, CDCl₃): 1.18 (t, 3 H), 2.90 (d, 2 H), 4.11 (q, 2 H), 4.58 (m, 2 H), 5.15-5.40 (m, 3 H), 5.90 (m, 1 H), 6.05 (m, 1 H), 7.55 (t, 1 H), 7.70 (d, 1 H), 8.12 (d, 1H), 8.19 (s, 1H).

### III. 2. d: Ethyl 3-{[(allyloxy)carbonyl]amino}-3-(3-aminophenyl)propanoate

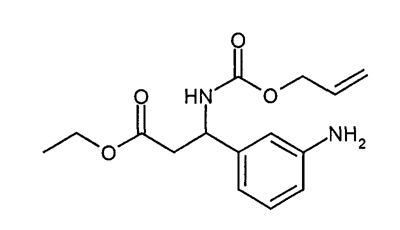

64.6 g tin-(II) chloride were added to a solution of 18.8 g ethyl 3-{[(allyloxy)-carbonyl]amino}-3-(3-nitrophenyl)propanoate_in 245 ml ethanol, and the mixture was heated to reflux for 2 h. The reaction mixture was hydrolized with 300 ml of 2N NaOH solution, then filtered through silica gel and washed with dichloromethane. The organic phase was dried over MgSO₄ and concentrated to yield 13.1 g of a white solid.
¹H-NMR (400 MHz, CDCl₃): 1.19 (t, 3 H), 2.80 (m, 2 H), 4.06 (q, 2 H), 4.57 (m, 2 H), 5.07 (m, 1 H), 5.20 (d, 1 H), 5.29 (d, 1 H), 5.70 (m, 1 H), 5.89 (m, 1 H), 6.57 (d, 1 H), 6.62 (s, 1 H), 6.68 (d, 1 H), 7.11 (t, 1 H).

### III.2.e: Ethyl-3-{[(allyloxy)carbonyl]amino}-3-(3-{[(3-nitrophenyl)sulfonyl]amino}phenyl)propanoate

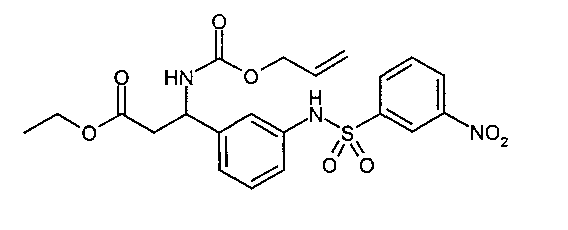

3-Nitrophenylsulphonyl chloride was added at 0°C to a solution of 11.6 g ethyl 3-{[(allyloxy)carbonyl]amino}-3-(3-aminophenyl)propanoate in 110 ml pyridine. After a reaction time of 2 h, the mixture was concentrated, treated with 1 N HCl and extracted with dichloromethane. After drying over MgSO₄, the solvent was removed and the crude product was purified by chromatography on silica gel (dichloromethane/methanol = 40:1) to give 17.8 g of a solid.

### III.2.f: Ethyl-3-{[(allyloxy)carbonyl]amino}-3-(3-{[(3-aminophenyl)sulfonyl]amino}phenyl)propanoate

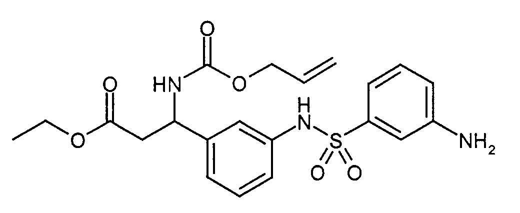

43.5 g tin-(II) chloride were added to a solution of 17.8 g ethyl 3-{[(allyloxy)-carbonyl]amino}-3-(3-{[(3-nitrophenyl)sulfonyl]amino}phenyl)propanoate in 165 ml ethanol, and the mixture was heated to reflux for 2 h. The reaction mixture was hydrolized with 200 ml of 2N NaOH solution, then filtered through silica gel and washed with dichloromethane. The organic phase was dried over MgSO₄ and concentrated to yield 9.2 g of a solid.

The material was separated into ist enantiomers by chiral chromatography with the selector Bayer-CSP (N-methacryloyl-L-valin-3-pentylamide) using THF as solvent. Similar selectors have been described in the literature (Angew. Chem. Int. Ed. Engl. 30 (1991), 1662-1664.). This separation yielded in the two products fraction 1 and fraction 2.
¹H-NMR (400 MHz, CDCl₃): 1.18 (t, 3 H), 2.80 (m, 2 H), 3.95 (br.s, 2 H), 4.08 (q, 2 H), 4.54 (m, 2 H), 5.08 (m, 1 H), 5.22 (d, 1 H), 5.30 (d, 1 H), 5.78 (m, 1 H), 5.90 (m, 1 H), 6.58 (s, 1 H), 6.75 (d, 1 H), 6.88 (d, 1 H), 6.95 (s, 1 H), 7.05 (d, 1 H), 7.11 (d, 1 H), 7.15-7.22 (m, 3 H).

### III.2.g: Ethyl-3-{[(allyloxy)carbonyl]amino}-3-(3-{[(3-{[(propylamino)carbonyl] amino}phenyl)sulfonyl]amino}phenyl)propanoate

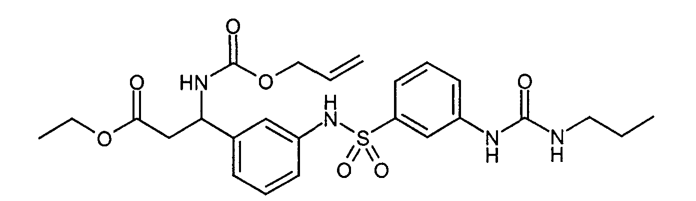

9.6 g propylisocyanate was added to a solution of 50 g of enantiopure ethyl 3-{[(allyloxy)carbonyl]amino}-3-(3-{[(3-aminophenyl)sulfonyl]amino}phenyl)propanoate from fraction 1 in 500 ml dioxane and the mixture was stirred for 18 h at 50°C. After evaporation of the solvent 1.5 1 hydrochloric acid (1 M) was added and the solution was extracted with dichloromethane. The organic phase was dried over MgSO₄ and concentrated. Chromatographie on silica gel (dichloromethane/methanol = 30:1) yielded 19.1 g of the compound.
¹H-NMR (400 MHz, CDCl₃): 0.90 (t, 3 H), 1.19 (t, 3 H), 1,52 (q, 2 H), 2.83 (m, 2 H), 3.18 (m, 2 H), 4.10 (q, 2 H), 4.56 (m, 2 H), 5.05-5.35 (m, 3 H), 5.90 (m, 1 H), 6.18 (m, 1 H), 6.77 (m, 1 H), 6.88 (s, 1 H), 7.00-7-45 (m, 8 H), 7.98 (m, 1 H).

### III.2.h: Ethyl 3-amino-3-(3{[(3-{[(propylamino)carbonyl]amino}phenyl)sulfonyl]-amino}phenyl)propanoate

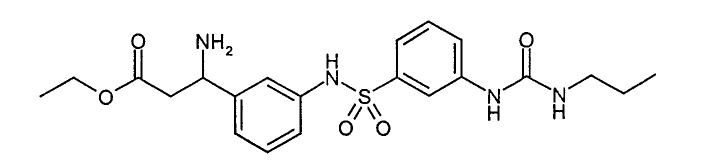

2.57 ml acetic acid, 5.32 ml tributyltinhydride and 177 mg bistriphenylphosphine palladium(II) chloride were added to a solution of 9.58 g ethyl 3-{[(allyloxycarbonyl]amino}-3-(3-{[(3-{[(propylamino)carbonyl]amino}phenyl)sulfonyl]-amino}phenyl)propanoate in 245 ml dichloromethane and stirred at room temperature for 22 h. A solution of NaHCO₃ was added and the mixture was extracted with dichloromethane. After drying over MgSO₄, the solvent was removed and the crude product was purified by chromatographie on silica gel (dichloromethane/methanol = 10:1) to give 2.30 g of the desired compound.
¹H-NMR (400 MHz, CDCl₃): 0.88 (t, 3 H), 1.20 (t, 3 H), 1,50 (q, 2 H), 2.60 (dd, 1 H), 2.67 (dd, 1 H), 3.17 (m, 2 H), 4.11 (q, 2 H), 4.33 (m, 1 H), 5.21 (m, 1 H), 6.89 (d, 1 H), 7.07 (d, 1 H), 7.16 (t, 1 H), 7.22-7.34 (m, 5 H), 7.38 (s, 1 H), 7.49 (s, 1 H).

### III.2: 3-(4-Aminophenylaminocarbonylamino)-3-[3-(propylaminocarbonylaminophenyl-sulphonylamino)-phenyl]-propionic acid, diastereoisomer A

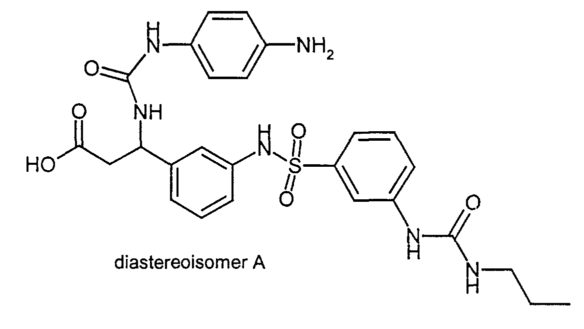

700 mg (1.56 mmol) of the compound III.2.h are stirred with 510 mg (2 eq) of 4-nitrophenyl isocyanate for 1 h in 100 ml of DMF. The mixture is concentrated and the residue is purified by flash chromatography on silica gel using dichloromethane/methanol/ammonia 17% strength (15:2:0.2). After precipitation from dichloromethane/methanol using ether, the intermediate a (290 mg; 30%) is obtained.

This is dissolved in methanol and hydrogenated upon palladium/carbon. The catalyst is separated off, the solution is concentrated and the residue is lyophilized from dioxane/water. 204 mg (74%) of intermediate b are obtained.

200 mg (0.34 mmol) of intermediate b are dissolved in methanol and treated with 1 ml of a 2M lithium hydroxide solution. After 6h additional 300 µl of lithium hydroxide are added and the mixture is stirred until the de-esterification is complete. The solution is concentrated and precipitated from dichloromethane using ether. 141 mg (75%) of compound III.2 are obtained [TLC: (acetonitrile/water/glacial acetiic acid 10/1/0.1 R_{f}=0.6].
ESI-MS: m/e = 555 (M+H)⁺].

### III.3: Enantiomer B of 3-(4-Aminophenylaminocarbonylamino)-3-[3-(propyaminocarbonylamino-phenyl-sulphonylamino)-phenyl]-propionic acid

The same protocol as for the synthesis of enantiomer A was used except that fraction 2 of ethyl 3-{[(allyloxy)carbonyl]amino}-3-(3-{[(3-aminophenyl)sulfonyl]amino}phenyl)propanoate was was used after the seperation of the enantiomers.

### III.4: 3-[3-(3-(Benzimidazol-2-yl-aminocarbonylamino)-phenyl)-phenylsulphonamido]-3-(3-amino-phenyl)-propionic acid

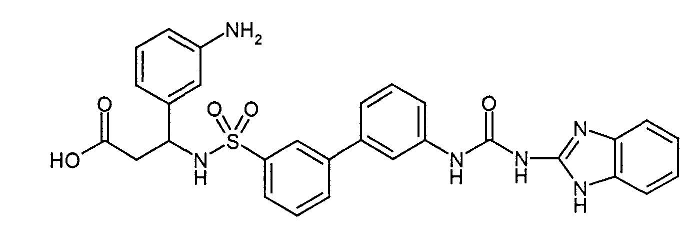

1.2 g of polystyrene Wang resin (loading 1.08 mmol/g) are swollen in DMF. The solvent is filtered off with suction and a solution of 841 mg of (3R,S)-3-fluorenylmethoxycarbonylamino-3-(3-nitrophenyl)-propionic acid (amino acid reagent) in 15 ml of DMF are added. After shaking at room temperature for 15 min, the suspension is treated with 350 µl of pyridine and 540 mg of 2,6-dichlorobenzoyl chloride. It is shaken overnight at room temperature. The resin is then washed with DMF, MeOH and DCM.

The resin is treated with 15 ml of a 20% strength piperidine solution in DMF and shaken at room temperature for 10 min. It is then washed 3 times with DMF and 15 ml of a 20% strength piperidine solution in DMF are added again. After shaking for 20 min, it is washed with DMF and THF. The resin is treated with a solution of 450 µl of DIEA in 500 µl of THF and a solution of 430 mg of 3-bromobenzenesulphonyl chloride (sulphonylating reagent) in 500 µl of THF. It is shaken overnight at room temperature. The resin is then washed with DMF, MeOH and THF.

The resin is suspended in 9000 µl of xylene, treated with 1250 mg of 3-aminobenzeneboronic acid monohydrate and a solution of 1940 mg of sodium carbonate in 9000 µl of water and shaken at room temperature for 5 min. 200 mg of bis(triphenylphosphine)-palladium(II) chloride and 150 mg of triphenylphosphine are then added and the mixture is stirred at 85°C overnight. The resin is then washed with THF/water 1:1, 0.25 M aqueous hydrochloric acid, water, DMF, MeOH, THF and DCM.

The resin is treated with a solution of 500 µl of DIEA in 12 ml of THF/DCM 1:1 and a solution of 2757 mg of 4-nitrophenylchloroformic acid ester in 12 ml of THF/DCM 1:1. After shaking at room temperature for 45 min, it is washed with THF and DMF and a solution of 2125 mg of 2-aminobenzimidazole (amine reagent) and 2780 µl of DIEA in 20 ml of NMP are added. After shaking for 10 h, the resin is washed with DMF, MeOH, THF and DCM. For the removal of the product, the resin is shaken with 12 ml of TFA/DCM for 1 h and filtered off, and the filtrate is concentrated in vacuo.

The crude product is taken up in methanol and reduced to the target product using hydrogen over palladium on active carbon. Chromatographic purification is carried out on silica gel using dichloromethane/methanol/ammonia (17% strength) (15:4:0.4). [TLC: (acetonitrile/water/glacial acetic acid 10:1:0.1), R_{f}= 0.5]. [MALDI-MS: m/e = 571 (M+H)⁺].

### III.5: 3-[4-(3-(Benzimidazol-2-yl-aminocarbonylamino)-phenyl)-phenylsulphonamido]-3-(3-amino-phenyl)-propionic acid

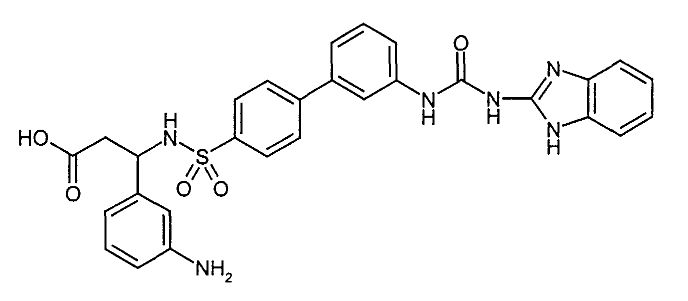

The preparation is carried out analogously to III.4 using 4-bromosulphonyl chloride as a sulphonylating reagent. Chromatographic purification of the target product is carried out on silica gel using dichloromethane/methanol/ammonia (17% strength) (15:2:0.2).
[TLC: (dichloromethane/methanol/ammonia (17% strength) (15:6:0.6); R_{f}= 0.33]. [FAB-MS: m/e=571 (M+H)⁺].

### IV Preparation of conjugates of integrin ligands with cytotoxic agents

### General procedure A (thiourea linkage)

0.09 mmol of an integrin ligand from series III are dissolved in 10 ml of dioxane/water (1:1) and treated with 9.6 µl (0.13 mmol) of thiophosgene. After stirring at room temperature for 15 min 94 µl (0.54 mmol) of Hünig's base are added, the mixture is stirred for a further 10 min and then concentrated. The residue is taken up in dichloromethane and precipitated using ether. The obtained isothiocyanates are reacted in the next step without further purification.

0.09 mmol of the isothiocyanate is dissolved in 15 ml of DMF and then treated with 0.08 mmol of one of the peptide conjugates in series II in the presence of 43 µl of Hünig's base. After stirring at room temperature for 30 min, the mixture is concentrated and the residue is stirred with water. The residue is separated and dissolved in methanol/dichloromethane. The mixture is precipitated using ether.

If neccessary, further purification is done by flash chromatography at silica gel. Appropriate eluent systems are:
Dichloromethane/methanol/ammonia 17% 15/3/0.3
Dichloromethane/methanol/ammonia 17% 16/2/0.2

The relevant fractions are collected, concentrated and the target products are isolated by precipitation from methanol/dichloromethane using ether.

### General procedure B (urea linkage)

0.07 mmol 4-Nitrophenyl chloroformic acid ester are dissolved in 10 ml THF and 16 µl Hünig's base are added. Subsequently, 0.05 mmol of one of the integrin ligands from series III dissolved in a mixture of 5 ml THF and 0.5 ml DMF are added in small portions and the mixture is stirred at room temperature for 10 min. 0.04 mmol of a peptide conjugate from series II dissolved in 2 ml of DMF and 24 µl Hünig's base are added and the mixture is stirred for an additional hour at room temperature. The solvent is removed and the residue is purified by flash chromatography at silica gel. Appropriate eluent mixtures are:
Dichloromethane/methanol/ammonia 17% 15/3/0.3
Dichloromethane/methanol/ammonia 17% 16/2/0.2

The relevant fractions are collected, concentrated and the target products are isolated by precipitation from methanol/dichloromethane using ether.

### Example 1

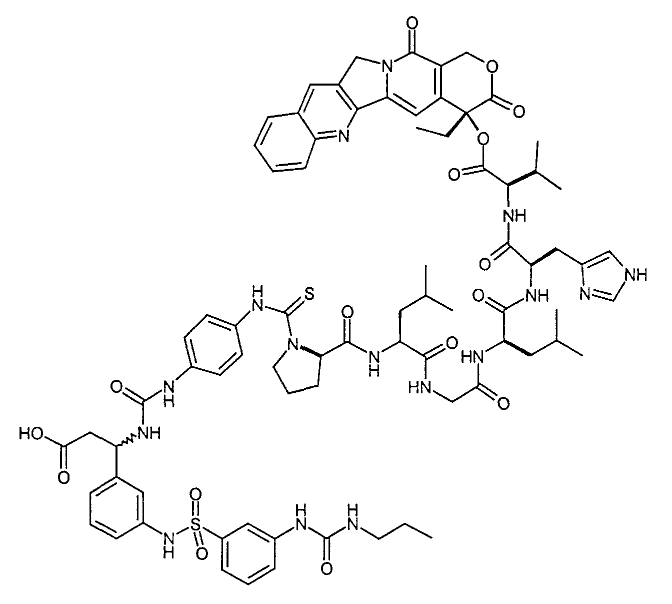

| | | |
|---|---|---|
| Educts: II.1, III.1 | Procedure: A | |
| Yield: 71% | R_{f}= 0.28 ⁶⁾ | [ESI-MS: m/e = 1561 = (M+H)⁺] |

### Example 2: Diastereoisomer A of compound from Example 1

| | | |
|---|---|---|
| Educts: II.1, III.2 | Procedure: A | |
| Yield: 32% | R_{f}= 0.28 ⁶⁾ | [ESI-MS: m/e = 1561 = (M+H)⁺] |

### Example 3: Diastereoisomer B of compound from Example 1

| | | |
|---|---|---|
| Educts: II.1, III.3 | Procedure: A | |
| Yield: 13% | R_{f}= 0.5 ¹⁾ | [ESI-MS: m/e = 1561 = (M+H)⁺] |

### Example 4

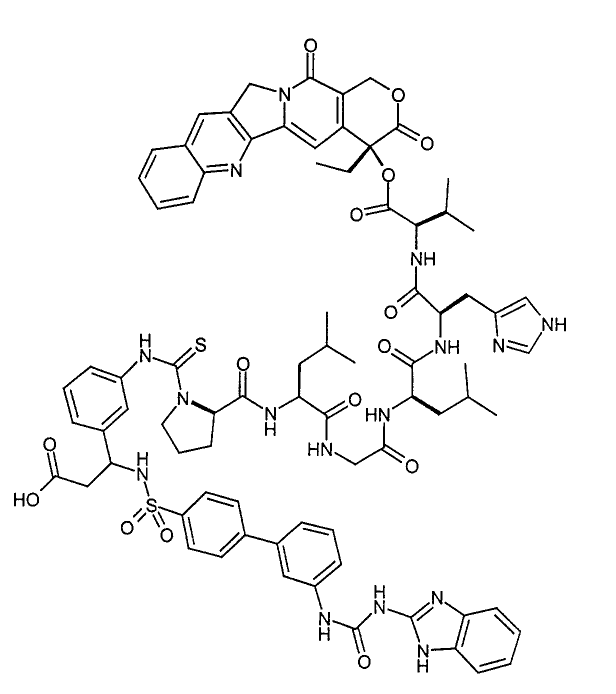

| | | |
|---|---|---|
| Educts: II.1, III.5 | Procedure: A | |
| Yield: 20% | R_{f}= 0.54 ¹⁾ | [ESI-MS: m/e = 1578 = (M+H)⁺] |

### Example 5

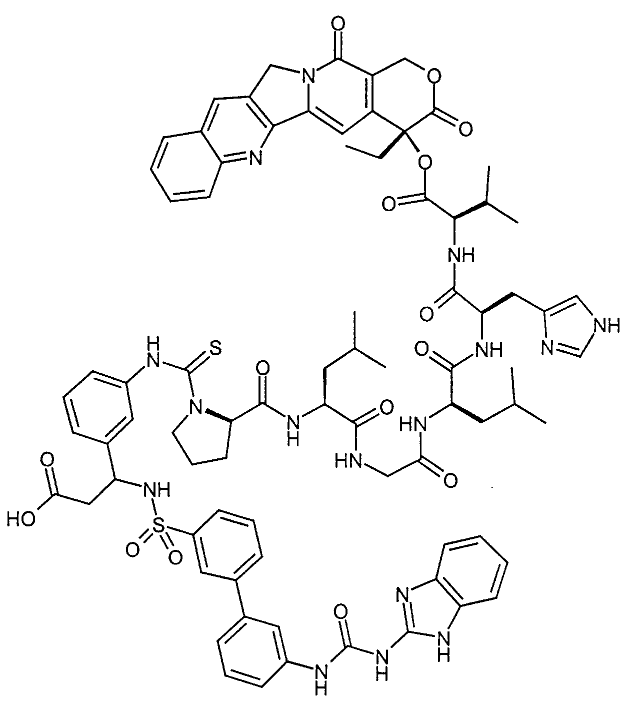

| | | |
|---|---|---|
| Educts: II.1, III.4 | Procedure: A | |
| Yield: 64% | R_{f}= 0.22 ⁸⁾ | [ESI-MS: m/e = 1578 = (M+H)⁺] |

### Example 6

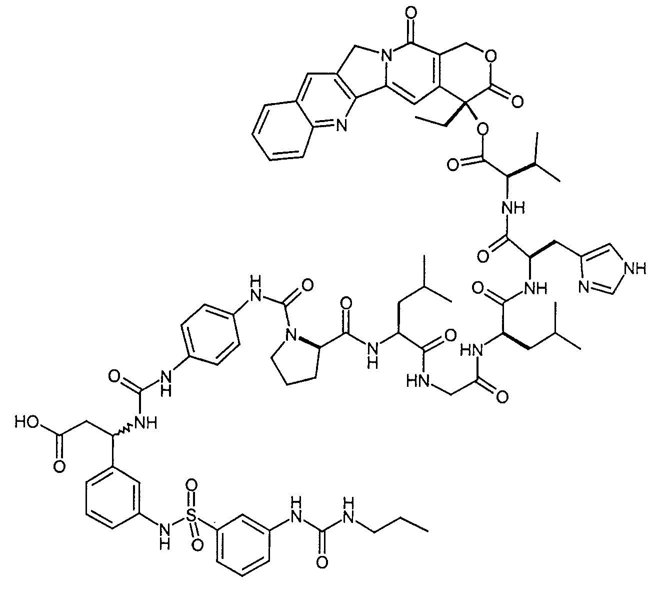

| | | |
|---|---|---|
| Educts: II.1, III.1 | Procedure: B | |
| Yield: 9% | R_{f}= 0.44 ⁶⁾ | [ESI-MS: m/e = 1545 = (M+H)⁺] |

### Example 7

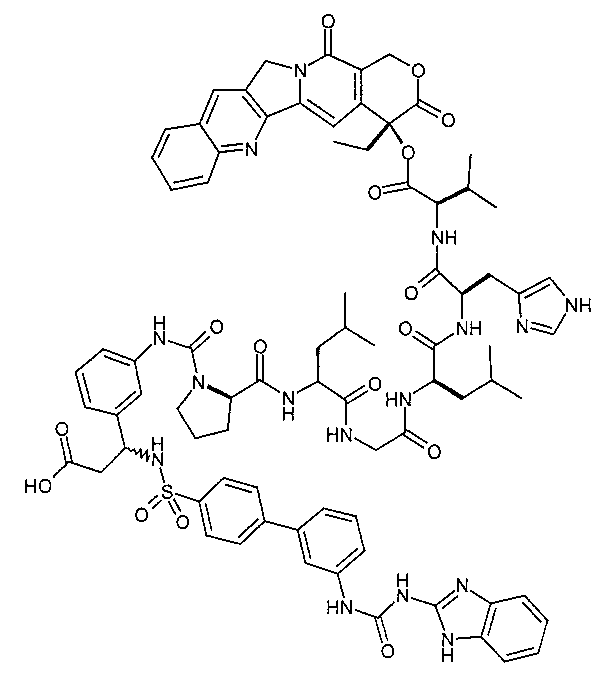

| | | |
|---|---|---|
| Educts: II.1, III.5 | Procedure: B | |
| Yield: 6% | R_{f}=0.44⁶⁾ | [ESI-MS: m/e = 1561 = (M+H)⁺] |

### Example 8

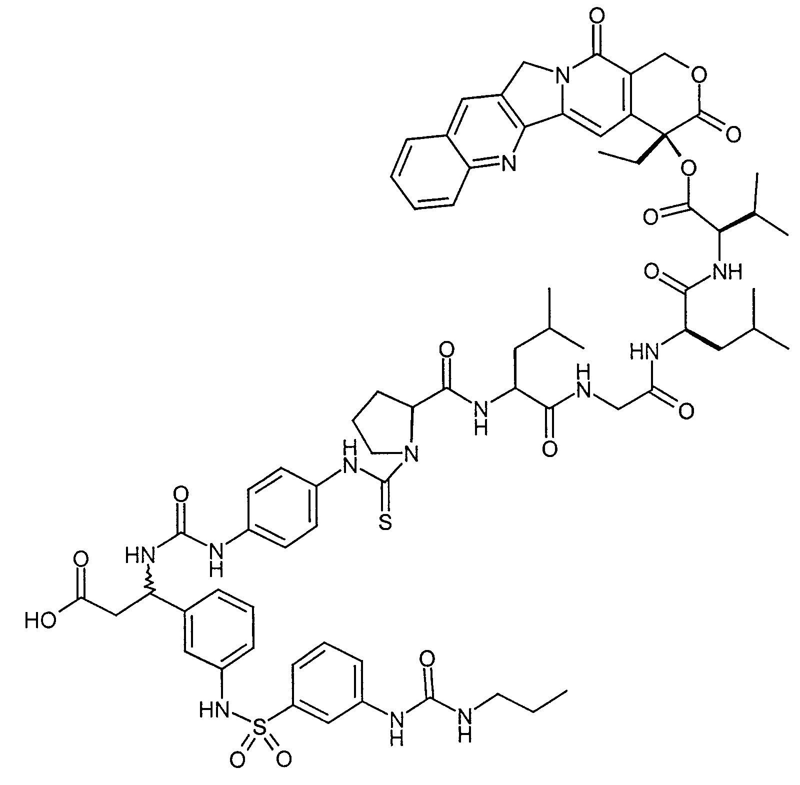

| | | |
|---|---|---|
| Educts: II.2, III.1 | Procedure: A | |
| Yield: 85% | R_{f}= 0.42 ³⁾ | [ESI-MS: m/e = 1424 = (M+H)⁺] |

### Example 9

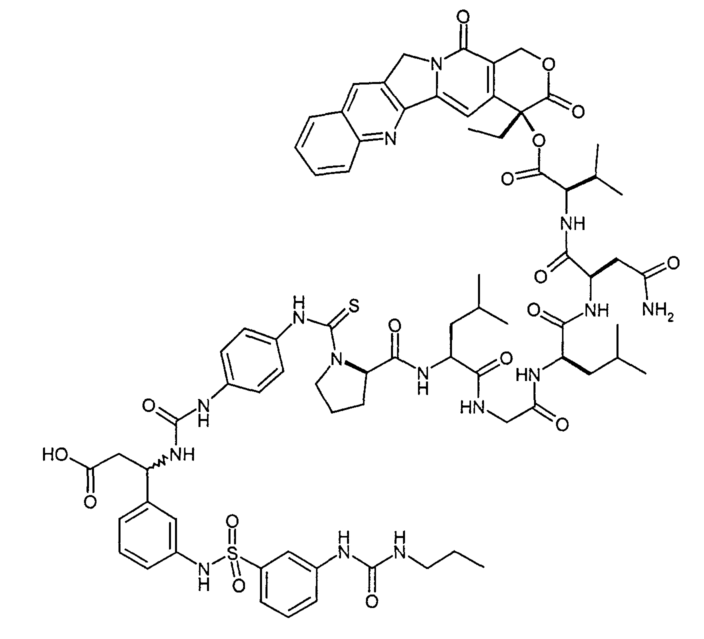

| | | |
|---|---|---|
| Educts: II.4, III.1 | Procedure: A | |
| Yield: 61% | R_{f}= 0.4 ³⁾ | [ESI-MS: m/e = 1538 = (M+H)⁺] |

### Example 10: Diastereoisomer A of compound from Example 9

| | | |
|---|---|---|
| Educts: II.4, III.2 | Procedure: A | |
| Yield: 27% | R_{f}=0.4 ³⁾ | [ESI-MS: m/e = 1538 = (M+H)⁺] |

### Example 11: Diastereoisomer B of compound from Example 9

| | | |
|---|---|---|
| Educts: II.4, III.3 | Procedure: A | |
| Yield: 11% | R_{f}=0.3 ³⁾ | [ESI-MS: m/e = 1538 = (M+H)⁺] |

### Example 12

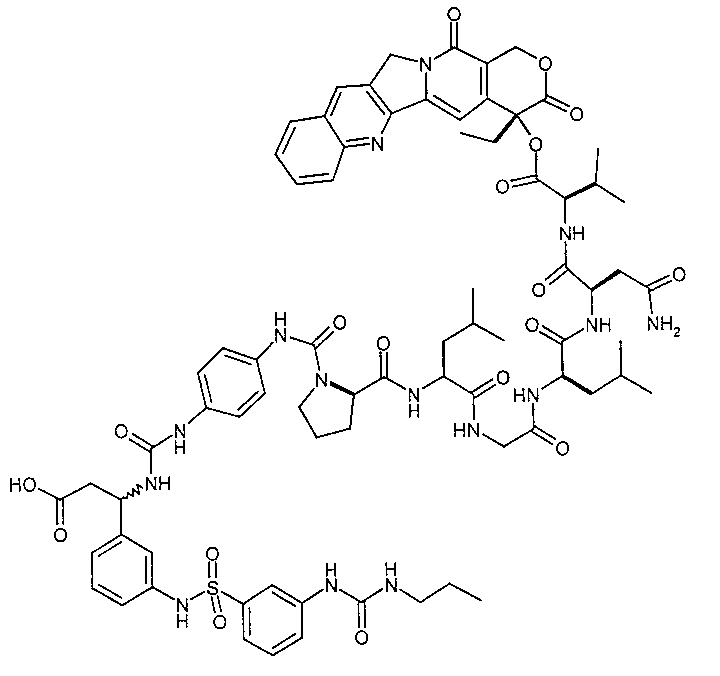

| | | |
|---|---|---|
| Educts: II.4, III.1 | Procedure: B | |
| Yield: 30% | R_{f}= 0.4 ³⁾ | [ESI-MS: m/e = 1522 (M+H)⁺] |

### Example 13

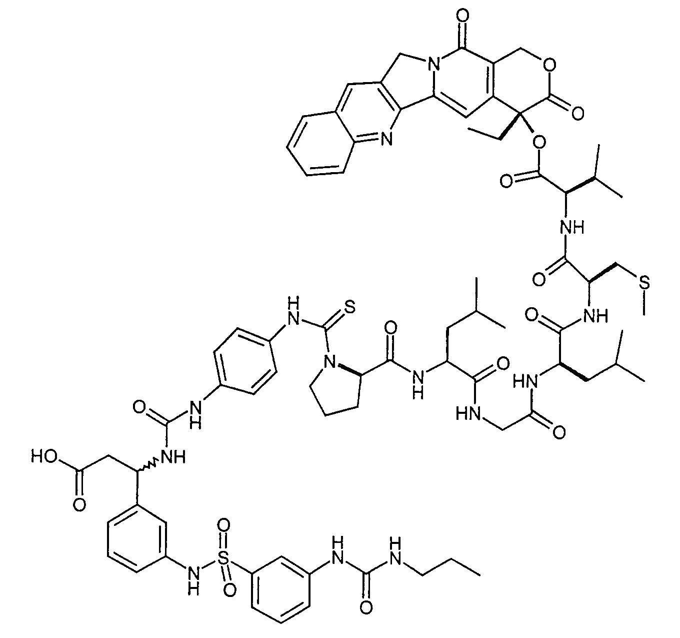

| | | |
|---|---|---|
| Educts: II.5, III.1 | Procedure: A | |
| Yield: 34% | R_{f}= 0.37 ⁶⁾ | [ESI-MS: m/e = 1541 (M+H)⁺] |

### Example 14

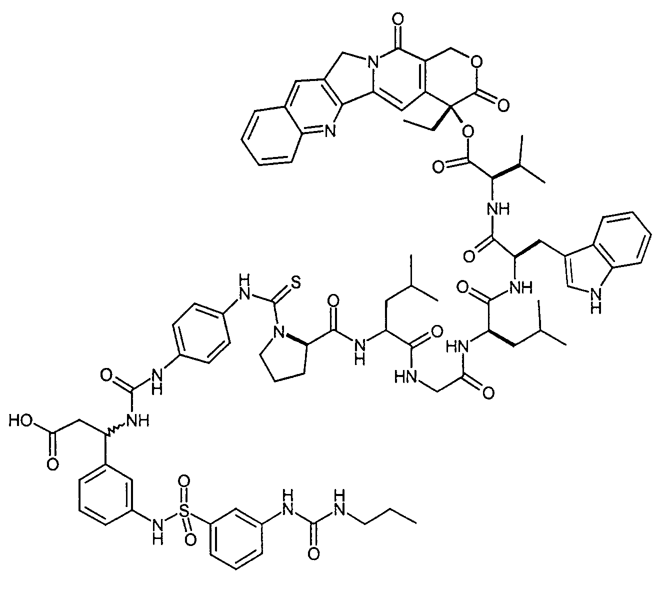

| | | |
|---|---|---|
| Educts: II.11, III.1 | Procedure: A | |
| Yield: 41% | R_{f}= 0.48 ³⁾ | [ESI-MS: m/e = 1610 (M+H)⁺] |

### Example 15

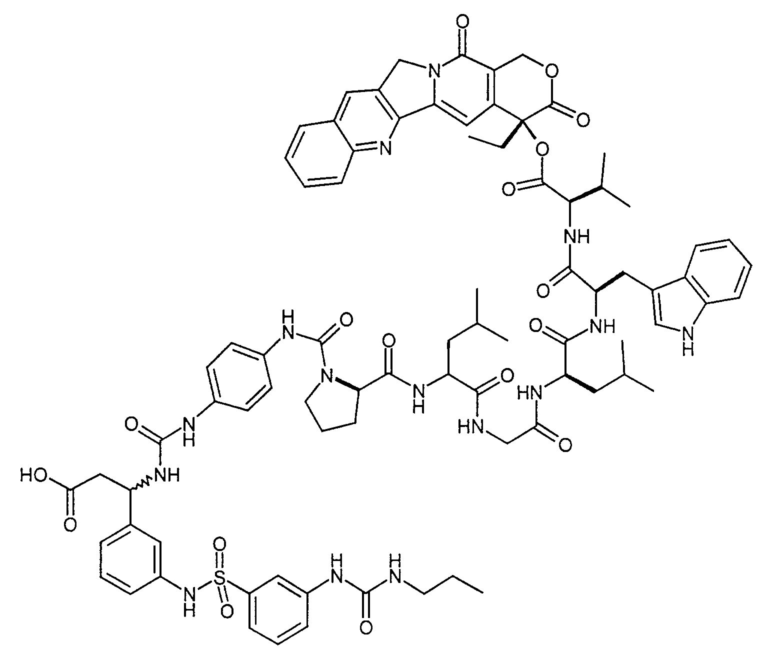

| | |
|---|---|
| Educts: II.11, III.1 | Procedure: B |
| Yield: 9% | R_{f}=0.42 ⁶⁾ |

### Example 16

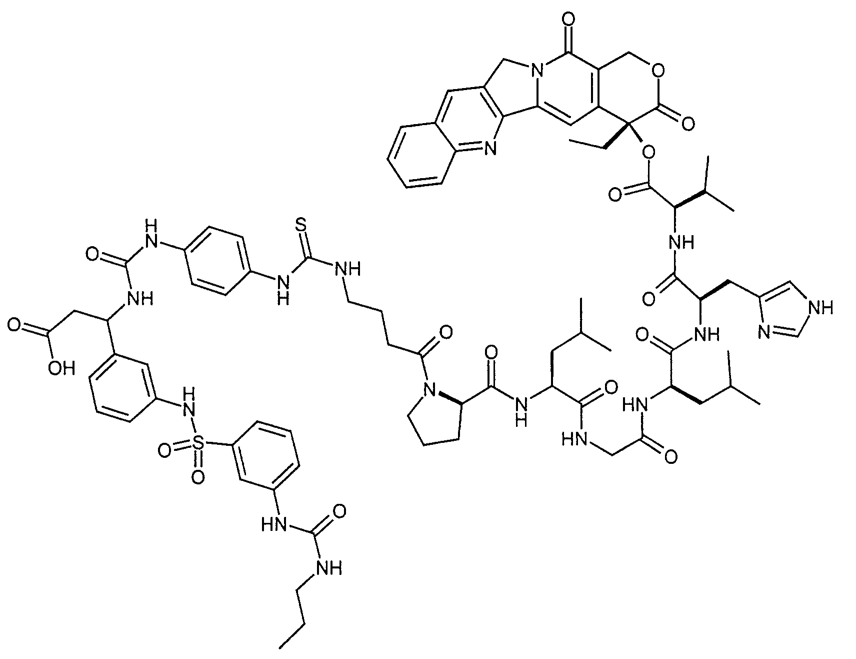

| | | |
|---|---|---|
| Educts: II.21, III.1 | Procedure: A | |
| Yield: 15% | R_{f}= 0.53 ⁶⁾ | [ESI-MS: m/e = 1646 (M+H)⁺] |

### Example 17

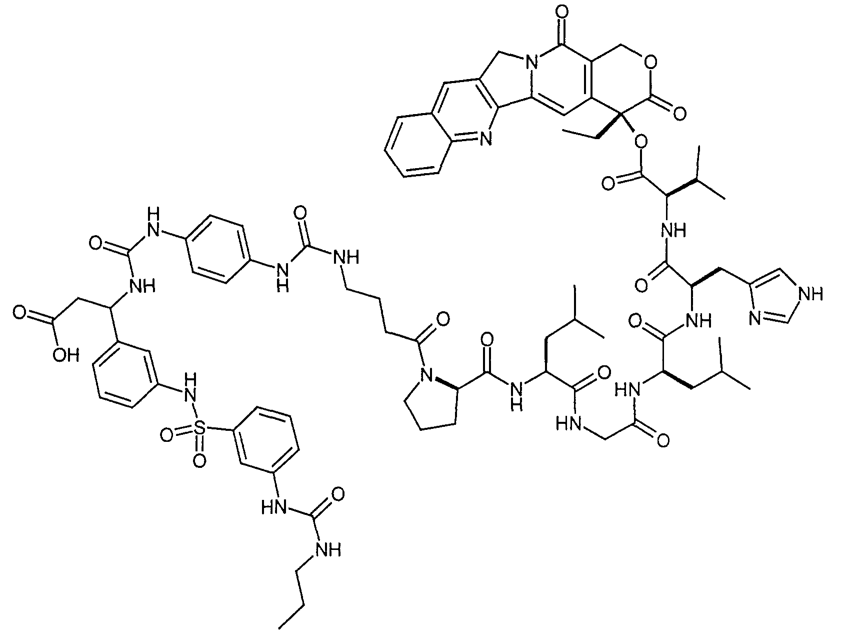

| | | |
|---|---|---|
| Educts: II.21, III.1 | Procedure: B | |
| Yield: 11% | R_{f}=0.07³⁾ | [ESI-MS: m/e = 1630 (M+H)⁺] |

### Example 18

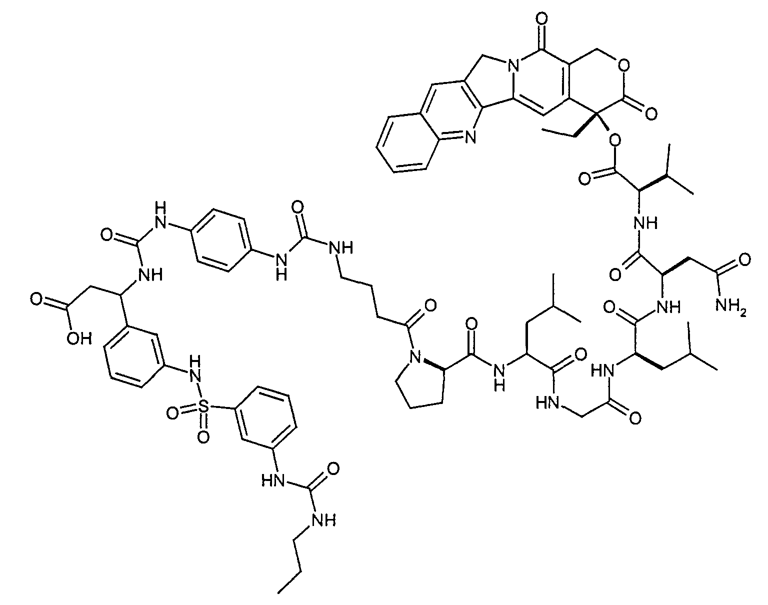

| | | |
|---|---|---|
| Educts: II.22, III.1 | Procedure: B | |
| Yield: 8% | R_{f}= 0.31 ⁶⁾ | [ESI-MS: m/e = 1608 (M+H)⁺] |

### Example 19

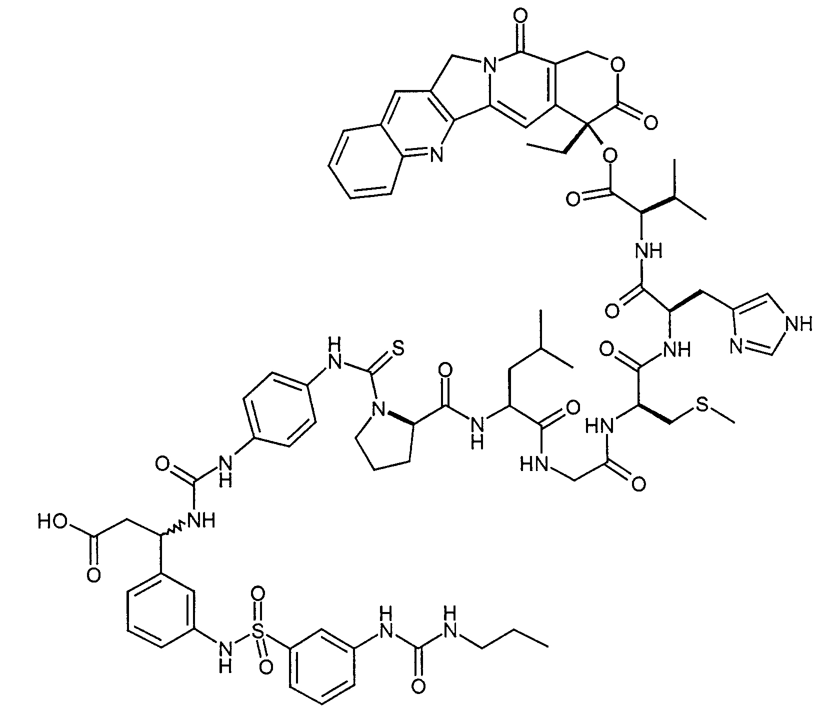

| | | |
|---|---|---|
| Educts: II.6, III.1 | Procedure: A | |
| Yield: 38% | R_{f}= 0.31 ⁶⁾ | [ESI-MS: m/e = 1566 (M+H)⁺] |

### Example 20

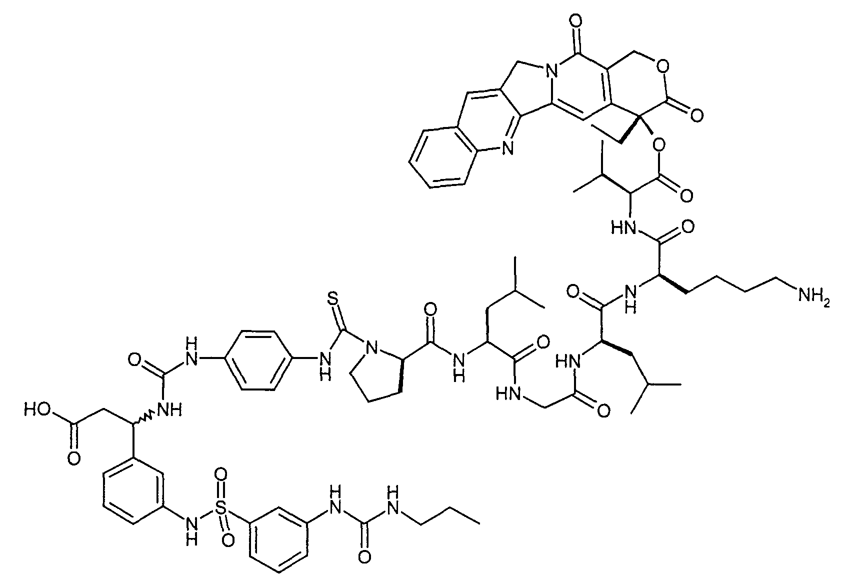

| | | |
|---|---|---|
| Educts: II, III.1 | Procedure: A, subsequent Fmoc cleavage. | |
| Yield: 11% over 2 steps | R_{f}=0.5 ¹⁾ | [ESI-MS: m/e = 1552 (M+H)⁺] |

### Example 21

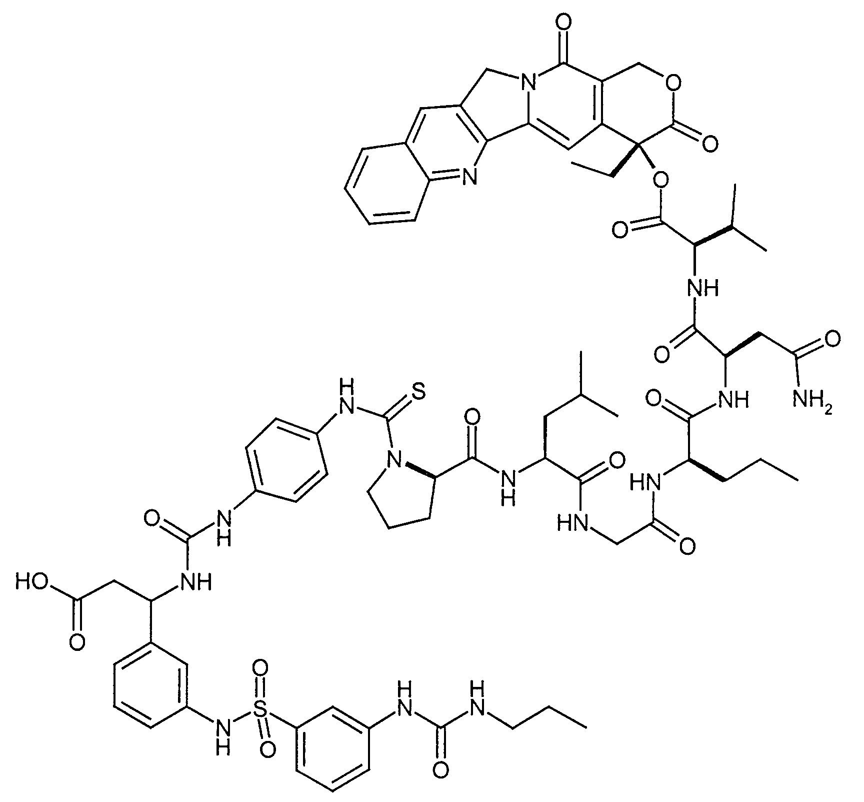

| | | |
|---|---|---|
| Diastereoisomer A | | |
| Educts: II.7, III.2 | Procedure: A | |
| Yield: 45% | R_{f}= 0.3 ⁶⁾ | [ESI-MS: m/e = 1524 (M+H)⁺] |

### Example 22

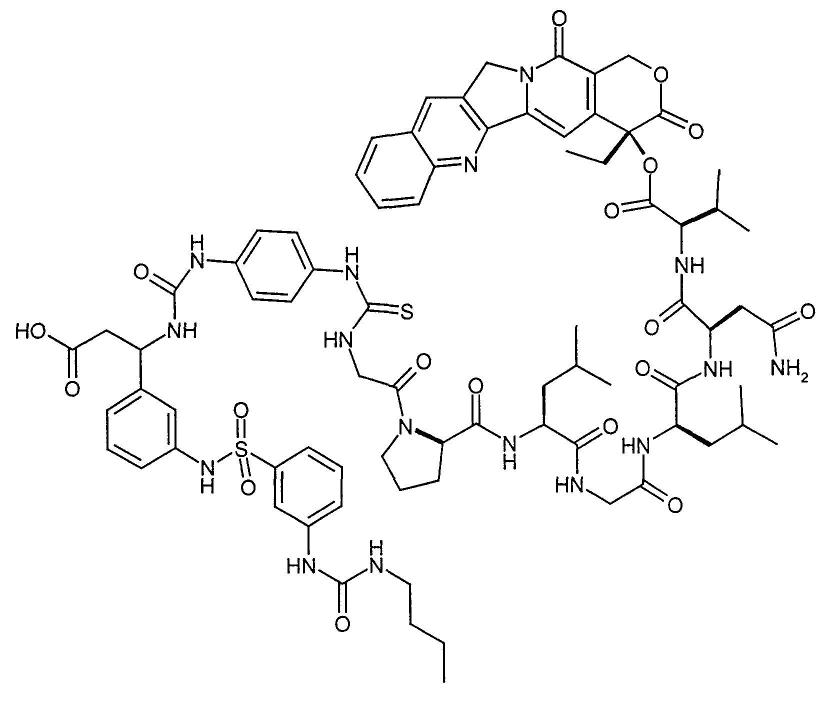

| | | |
|---|---|---|
| Diastereoisomer A | | |
| Educts: II.23, III.2 | Procedure: A | |
| Yield: 11% | R_{f}= 0.27 ⁶⁾ | [ESI-MS: m/e = 1596 (M+H)⁺] |

### Example 23

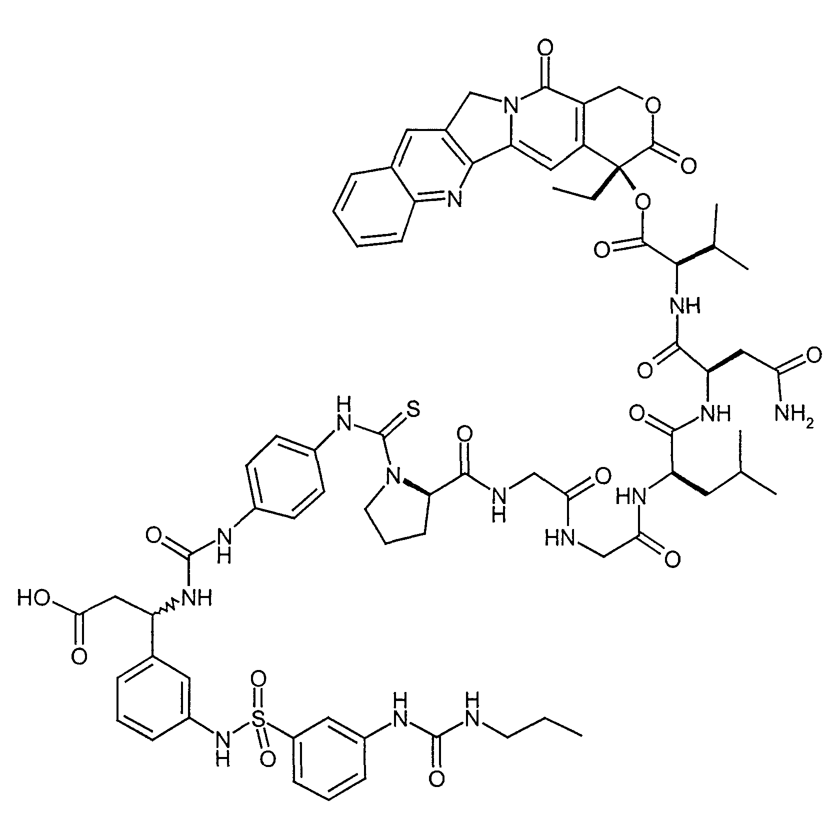

| | | |
|---|---|---|
| Educts: II.20, III.1 | Procedure: A | |
| Yield: 74% | R_{f}= 0.25 ⁶⁾ | [ESI-MS: m/e = 1482 (M+H)⁺] |

### Example 24

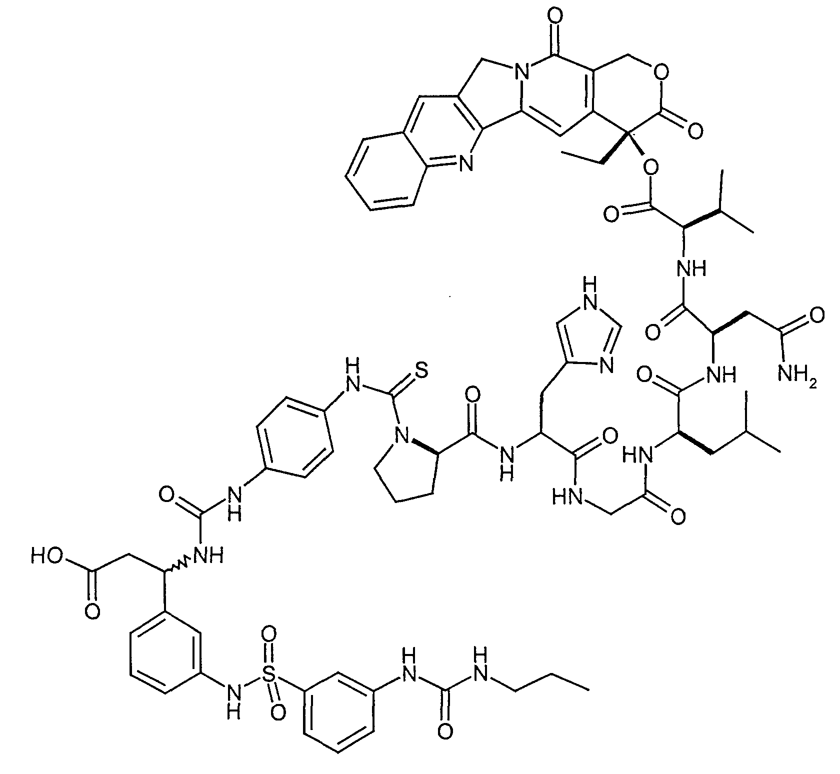

| | | |
|---|---|---|
| Educts: II.25, III.1 | Procedure: A | |
| Yield: 44% | R_{f}=0.38 ¹⁾ | [ESI-MS: m/e = 1563 (M+H)⁺] |

### Example 25 (diastereoisomer A of compound from example 19)

| | | |
|---|---|---|
| Educts: II.6, III.2 | Procedure: A | |
| Yield: 36% | R_{f}=0.14³⁾ | [ESI-MS: m/e = 1566 (M+H)⁺] |

### Example 26

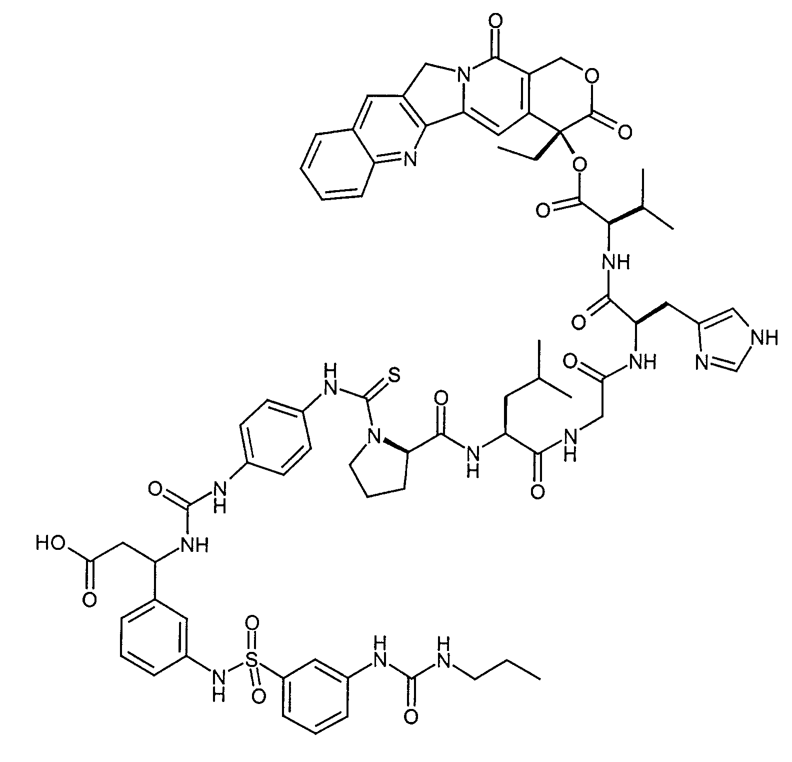

| | | |
|---|---|---|
| Diastereoisomer A | | |
| Educts: II.3, III.2 | Procedure: A | |
| Yield: 21% | R_{f}= 0.34 ⁶⁾ | [ESI-MS: m/e = 1448 (M+H)⁺] |

### Example 27

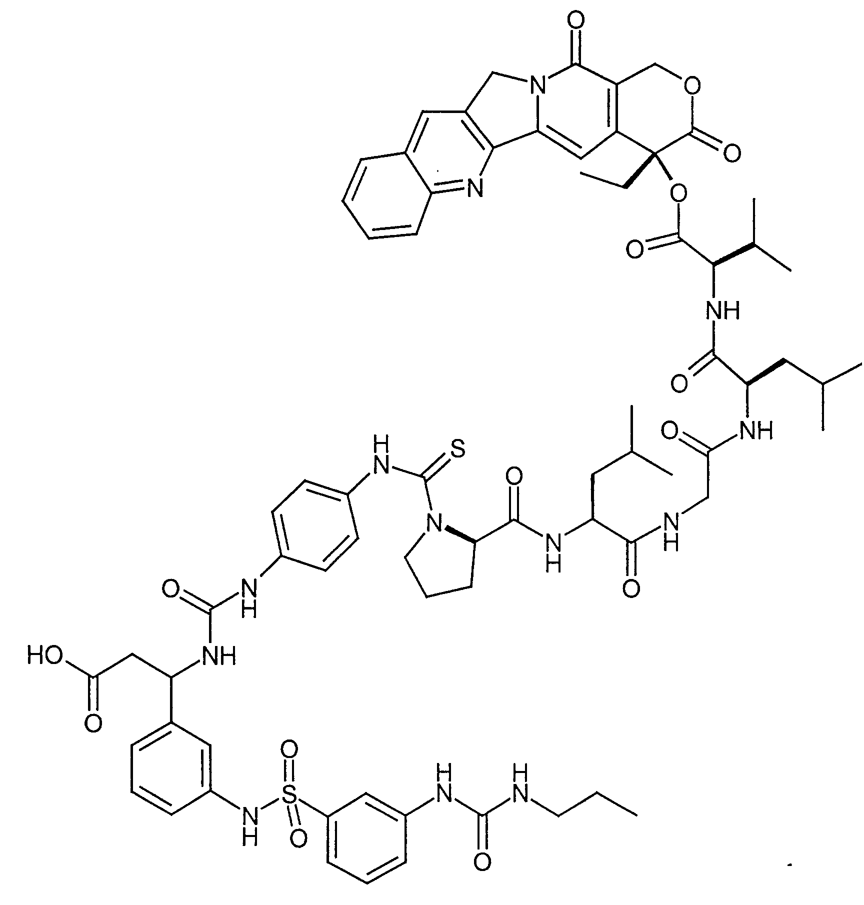

| | | |
|---|---|---|
| Diastereoisomer A | | |
| Educts: II.2, III.2 | Procedure: A | |
| Yield: 66% | R_{f}= 0.48 ³⁾ | [ESI-MS: m/e = 1425 = (M+H)⁺] |

### Example 28

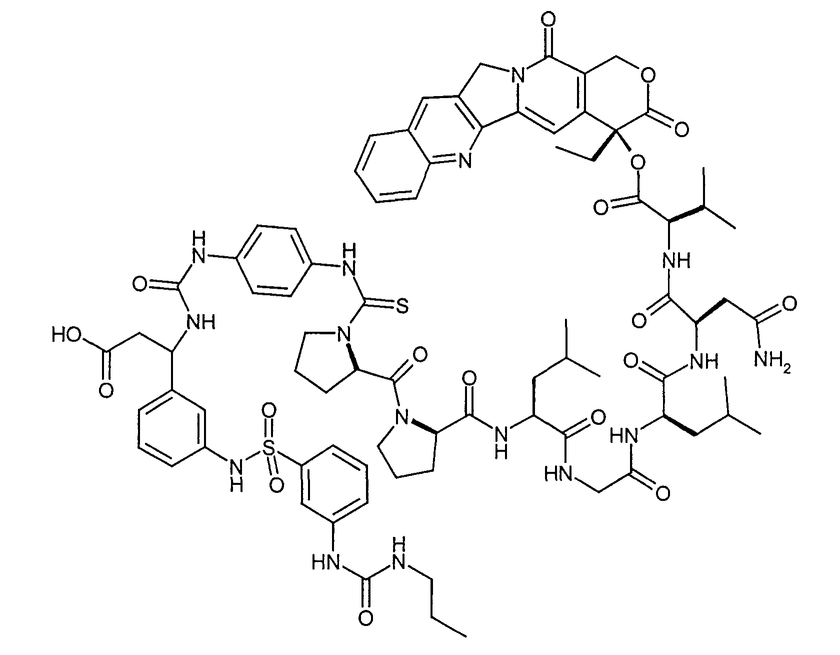

| | | |
|---|---|---|
| Diastereoisomer A | | |
| Educts: II.24, III.2 | Procedure: A | |
| Yield: 35% | R_{f}= 0.5 ⁴⁾ | [ESI-MS: m/e = 1636 = (M+H)⁺] |

### Example 29

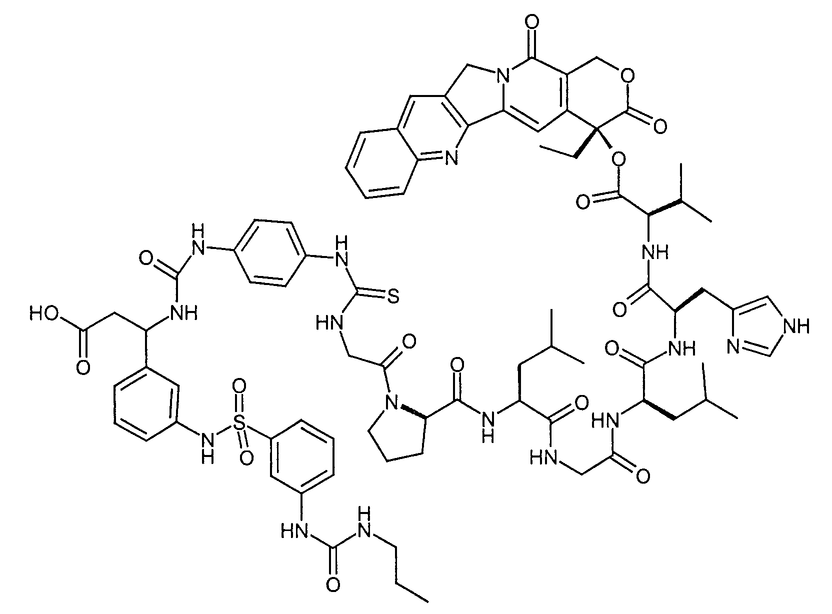

| | | |
|---|---|---|
| Diastereoisomer A | | |
| Educts: II.27, III.2 | Procedure: A | |
| Yield: 13% | R_{f}=0.6 ⁶⁾ | [ESI-MS: m/e = 1619 = (M+H)⁺] |

### Example 30

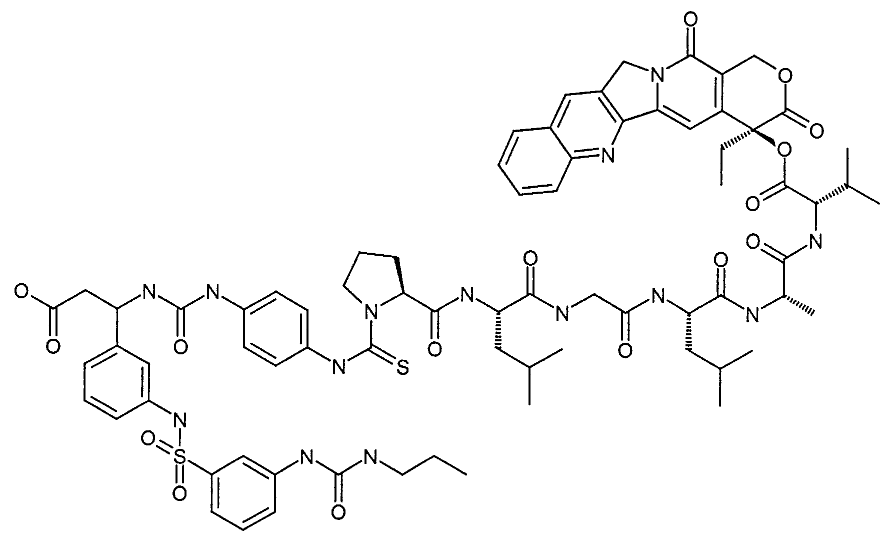

| | | |
|---|---|---|
| Educts: II.26, III.2 | Procedure: A | |
| Yield: 67% | R_{f}=0.48⁶⁾ | [ESI-MS: m/e = 1495.3 (M+H)⁺] |

### Example 31

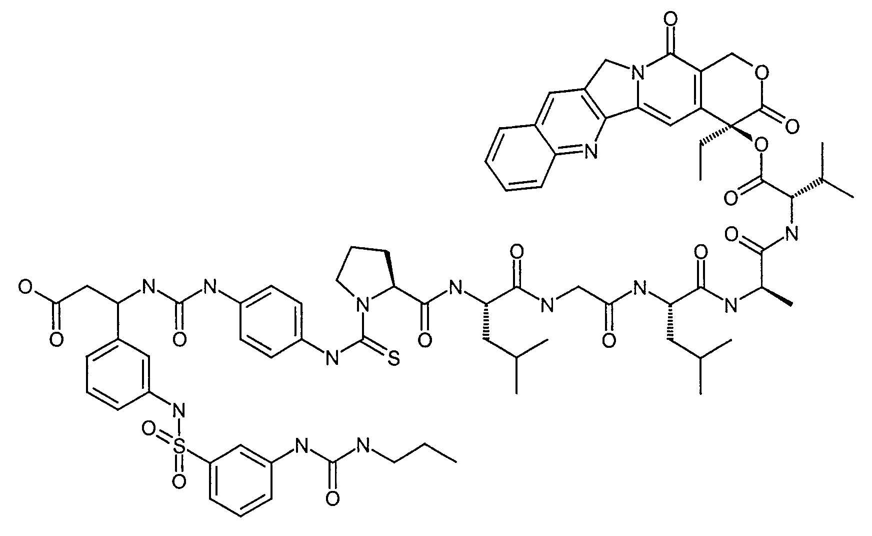

| | | |
|---|---|---|
| Educts: II.12, III.2 | Procedure: A | |
| Yield: 56% | R_{f}=0.46⁶⁾ | [ESI-MS: m/e = 1495.3 (M+H)⁺] |

### Example 32

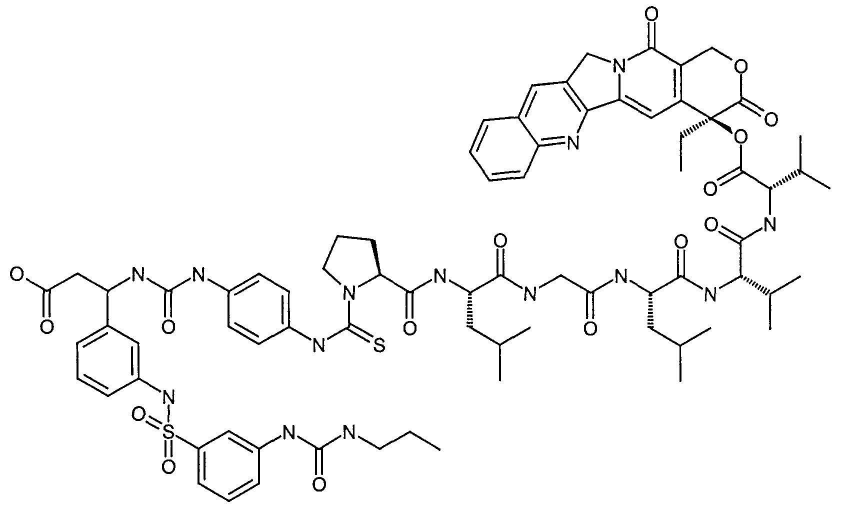

| | | |
|---|---|---|
| Educts: II.13, III.2 | Procedure: A | |
| Yield: 72% | R_{f}=0.62⁶⁾ | [ESI-MS: m/e = 1523.2 (M+H)⁺] |

### Example 33

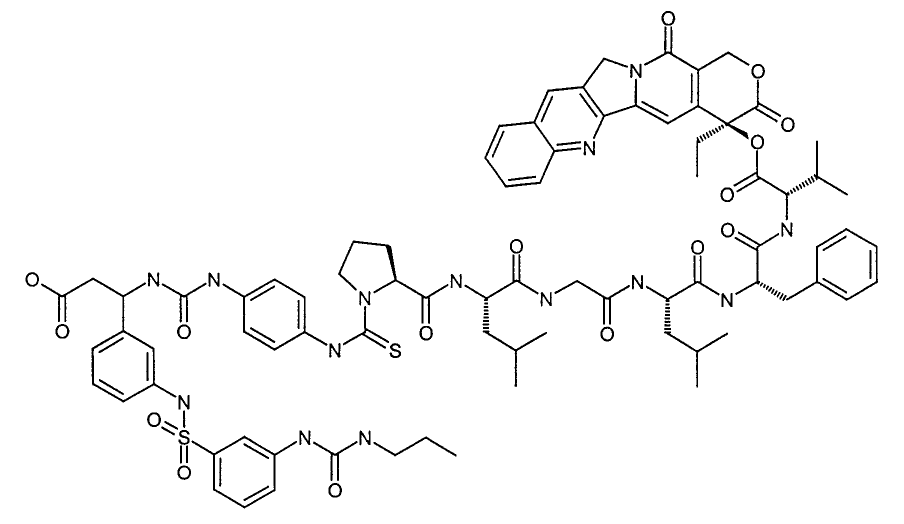

| | | |
|---|---|---|
| Educts: II.14, III.2 | Procedure: A | |
| Yield: 69% | R_{f}=0.59⁶⁾ | [ESI-MS: m/e = 1571.3 (M+H)⁺] |

### Example 34

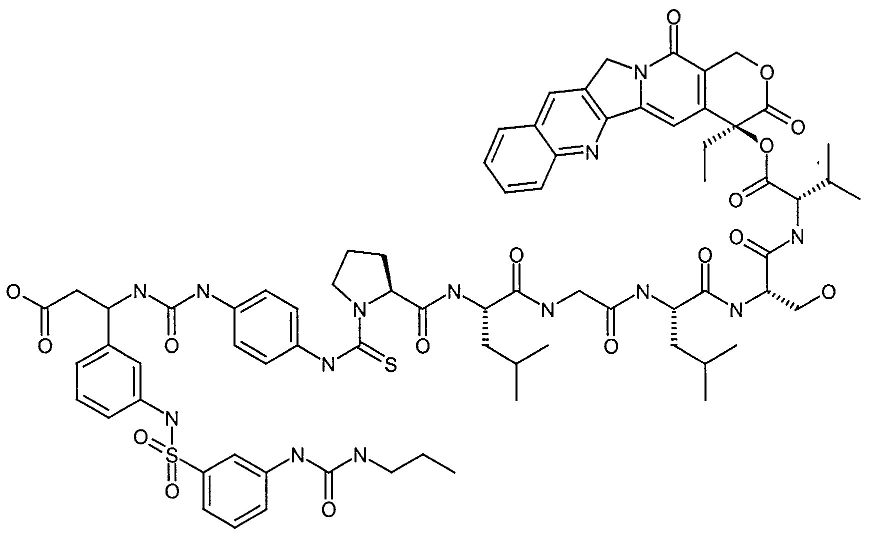

| | | |
|---|---|---|
| Educts: II.15, III.2 | Procedure: A | |
| Yield: 70% | R_{f}=0.26¹⁰⁾ | [ESI-MS: m/e = 1511.4 (M+H)⁺] |

### Example 35

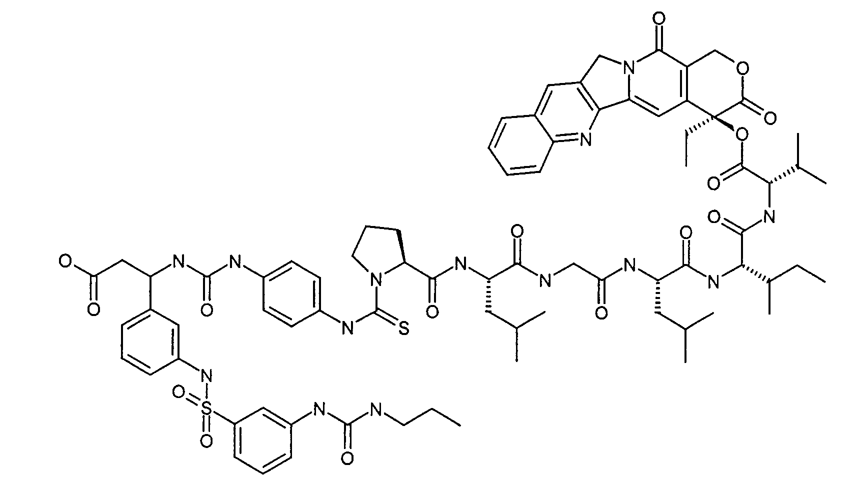

| | | |
|---|---|---|
| Educts: II.16, III.2 | Procedure: A | |
| Yield: 81% | R_{f}= 0.33 ¹⁰⁾ | [ESI-MS: m/e = 1537.4 (M+H)⁺] |

### Example 36

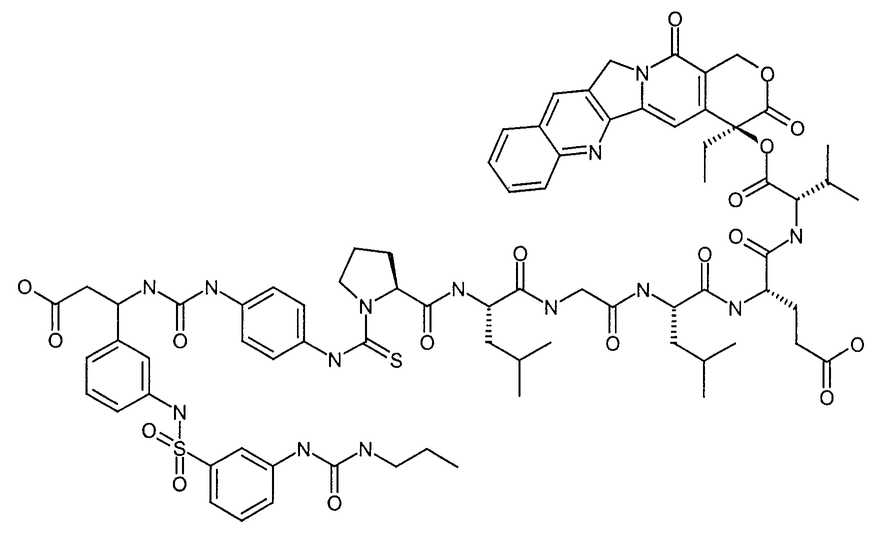

| | | |
|---|---|---|
| Educts: II.17, III.2 | Procedure: A | |
| Yield: 73% | R_{f}=0.48⁶⁾ | [ESI-MS: m/e = 1553.3 (M+H)⁺] |

### Example 37

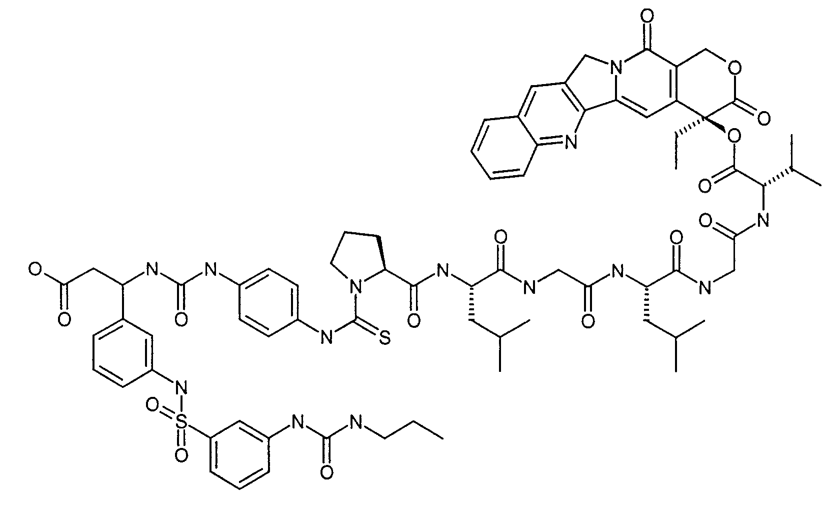

| | | |
|---|---|---|
| Educts: II.18, III.2 | Procedure: A | |
| Yield: 54% | R_{f}=0.31¹⁰⁾ | [ESI-MS: m/e = 1481.3 (M+H)⁺] |

### Example 38

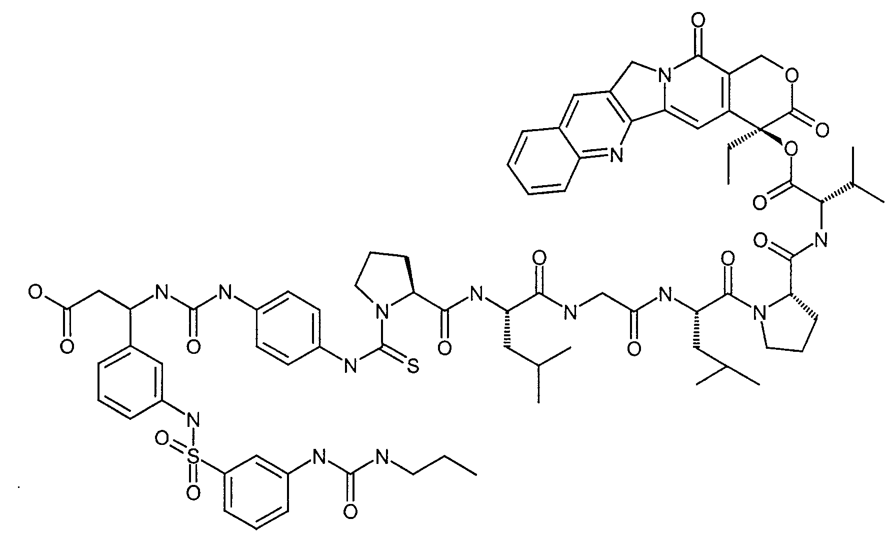

| | | |
|---|---|---|
| Educts: II.19, III.2 | Procedure: A | |
| Yield: 51% | R_{f}=0.42¹⁰⁾ | [ESI-MS: m/e = 1521.3 (M+H)⁺] |

### Biological Tests

### A: αᵥβ₃ Binding test

αᵥβ₃ from human A375 cells was purified analogously to a procedure which was described by Wong et al. (Molecular Pharmacology, 50, 529-537 (1996)). In each case, 10 µl of αᵥβ₃ (5 ng) in TBS pH 7.6, 2 mM CaCl₂, 1 mM MgCl₂, 1% n-octylglucopyranoside (Sigma); 10 µl of test substance in TBS pH 7.6, 0.1% DMSO and 45 µl of TBS pH 7.6, 2 mM CaCl₂, 1 mM MgCl₂, 1mM MnCl₂ were incubated at room temperature for 1 h. In each case, 25 µl of WGA SPA beads (Amersham, 4 mg/ml) and 10 µl of echistatin (0.1 µCi, Amersham, chloramine-T labelled) were then added. After 16 hours at room temperature, the samples were measured in a scintillation measuring apparatus (Wallac 1450). The test results are shown in Table 1 below.

**Table 1:**

| IC₅₀ values of the binding to the αᵥβ₃ receptor [nM] | |
|---|---|
| Example | IC₅₀ [nM] |
| 1 | 40 |
| 2 | 25 |
| 3 | 45 |
| 4 | 150 |
| 5 | 6000 |
| 6 | 60 |
| 7 | 700 |
| 8 | 30 |
| 9 | 30 |
| 10 | 20 |
| 11 | 300 |
| 12 | 300 |
| 13 | 50 |
| 14 | 70 |
| 15 | 500 |
| 16 | nix |
| 17 | 50 |
| 18 | 20 |
| 19 | 20 |
| 20 | 50 |

### B: Growth inhibition test for the determination of the cytotoxic properties on various tumour cell lines

The human large intestine cell lines SW 480 and HT29 (ATCC No. CCL 228 and HTB38 and the mouse melanoma cell line B16F10 (CRL 6475) were grown to confluence in Roux dishes in RPMI 1640 medium with addition of 10% FCS. They were then trypsinized and taken up in RPMI plus 10% FCS to a cell count of 50,000 cells or, for B16F10, 20,000 cells per ml. 100 µl of cell suspension/well were added to a 96 microwell plate and incubated at 37°C for 1 day in a CO₂ incubator. A further 100 µl of RPMI medium and 1 µl of DMSO were then added with the test substances. The growth was checked after day 6. For this, 25 µl of MTT solution (3-(4,5-dimethylthiazol-2-yl)-2,5-diphenyltetrazolium bromide) was added to each well at a starting concentration of 5 mg/ml of H₂O. The plate was incubated at 37°C for 5 hours in a CO₂ incubator. The medium was then aspirated and 100 µl of i-propanol/well were added. After shaking with 100 µl of H₂O for 30 min, the extinction was measured at 595 nm using a Multiplate Reader (BIO-RAD 3550-UV).

The cytostatic action is indicated in Table 2 as an IC₅₀ value, in each case for the individual cell lines.

**Table 2:**

| IC₅₀ values of the cytotoxic action on tumour cell lines [nM] | | | |
|---|---|---|---|
| | IC50 [nM] | | |
| Example | SW480 | HT29 | B16F10 |
| 1 | 70 | 50 | 250 |
| 2 | 100 | 180 | 500 |
| 3 | 150 | 200 | 600 |
| 4 | 70 | 90 | 400 |
| 5 | 80 | 100 | 350 |
| 6 | 50 | 40 | 150 |
| 7 | 150 | 150 | 600 |
| 8 | 200 | 150 | 350 |
| 9 | 60 | 80 | 400 |
| 10 | 70 | 100 | 600 |
| 11 | 150 | 200 | 400 |
| 12 | 40 | 30 | 150 |
| 13 | 80 | 100 | 400 |
| 14 | 50 | 60 | 400 |
| 15 | 1500 | 500 | 8000 |
| 16 | 40 | 25 | 50 |
| 17 | 70 | 100 | 600 |
| 18 | 35 | 25 | 70 |
| 19 | 60 | 80 | 300 |
| 20 | 50 | 70 | 300 |
| 21 | 60 | 30 | 200 |
| 22 | 30 | 15 | 90 |
| 23 | 400 | 150 | 1500 |
| 24 | 400 | 150 | 900 |
| 25 | 60 | 80 | 250 |
| 26 | 90 | 50 | 300 |
| 27 | 200 | 150 | 800 |
| 28 | 30 | 30 | 250 |
| 29 | 100 | 80 | 400 |
| 30 | 500 | 400 | |
| 31 | 300 | 250 | |
| 32 | 120 | 100 | 200 |
| 33 | 400 | 300 | 1000 |
| 34 | 100 | 50 | 800 |
| 35 | 200 | 150 | 800 |
| 36 | 150 | 100 | 300 |
| 37 | 500 | 300 | |
| 38 | 200 | 150 | 1000 |

### C. In-vivo inhibition or tumour growth using a nude mouse model

### Material

In all in-vivo experiments for investigating the inhibition of tumour growth, athymic nude mice (NMRI nu/nu strain) were used. The tumour was developed by serial passage in nude mice. The human origin of the tumour was confirmed by isoenzymatic and immunohistochemical methods.

### Experimental set-up

The tumour was implanted subcutaneously in both flanks of nu/nu nude mice 6 to 8 weeks old. The treatment was started, depending on the doubling time, as soon as the tumours had reached a diameter of 5 - 7 mm. The mice were assigned to the treatment group or the control group (5 mice per group having 8 - 10 assessable tumours) by randomization. The individual tumours of the control group all grew progressively.

The size of the tumours was measured in two dimensions by means of a slide gauge. The tumour volume, which correlated well with the cell count, was then used for all assessments. The volume was calculated according to the formula "length x breadth x breadth / 2" ([a x b²] / 2, a and b represent two diameters arranged at right angles).

The values of the relative tumour volume (RTV) were calculated for each individual tumour by dividing the tumour size on day X with the tumour size on day 0 (at the time of randomization). The average values of the RTV were then used for the further assessment.

The inhibition of the increase of the tumour volume (tumour volume of the test group/control group, T/C, in per cent) was the final measured value.

### Treatment

The compounds can be administered with a daily or an intermittent therapy schedule through a couple of days either by intraperitoneal, intravenious, oral or subcutaneous route.

In a subcutaneously growing melanoma xenograft model (MEXF 989) several compounds effected inhibitions of the tumor growth (eg. compound of example 2, 10 and 25). The compounds are dissolved in PEG400/water 2:1 and administered intravenously or intraperitoneally from day 1-3 and day 15-17. The optimal calculated T/C values are given in table 3.

**Table 3**

| Example | dose | lethality | optimal T/C in % |
|---|---|---|---|
| 2 | 6mg/kg/day | 0/5 | 17.7 |
| 2 | 12mg/kg/day | 1/5 (MTD) | 7.4 |
| 10 | 6mg/kg/day | 0/5 (MTD) | 11.4 |
| 25 | 6mg/kg/day | 1/5 (MTD) | 19.6 |

### D. CSF-induced proliferation of hemopoietic stem cells

Bone marrow cells are flushed out of the femur of mice. 10⁵ cells are incubated in McCoy 5A medium (0.3% agar) together with recombinant murine GM-CSF (Genzyme; parent cell colony formation) and the substances (10⁻⁴ to 100 µg/ml) at 37°C and 7% CO₂. 7 days later, the colonies (<50 cells) and clusters (17-50 cells) are counted.

A series of compounds exhibits a drastically reduced toxicity against stem cells in vitro compared to camptothecin (cf. Table 4).

**Table 4:**

| IC₅₀ values of the inhibition of colony formation of hemopoietic stem cells [ng/ml] | |
|---|---|
| Example | IC₅₀[ng/ml] |
| 1 | 30 |
| 16 | 15 |
| Camptothecin | 0.25 |

### E. Cleavage of conjugates by MMP-2 in buffer

2.5 µl MMP-2 (Calbiochem) with a specific activity of 60µU/µl are incubated with 10nM of the conjugates exemplified in the example series 1-25 in 1 ml of a medium consisting of 50 mM Tris-HCl pH 7.5, 0.2 M NaCl, 10 mM CaCL₂*2H₂O and 0,05% Brij 35. The enzyme mediated cleavage of the conjugates is detected by HPLC analysis using an RP18 (5µM) column with 70% HClO₄ /water (0.4% v/v) as eluent A and acetonitrile as eluent B (UV detection at 356nM). The efficiency of the cleavage is assessed by comparing the peak areas of the intact conjugate (starting material) and the cleavage product (tripeptide conjuagte of camptothecin) after 6 h and 24 h incubation (cf. Table 5).

**Table 5:**

| Ratio of peak areas of cleavage product/starting material after 6 h incubation with MMP-2 | |
|---|---|
| Example | MMP-2 mediated cleavage Peak area product/educt x100 |
| 1 | 17 |
| 2 | 18 |
| 3 | 25 |
| 4 | 6 |
| 5 | 5 |
| 6 | 33 |
| 10 | 16 |
| 11 | 20 |
| 12 | 70 |
| 14 | 5 |
| 17 | 16 |
| 18 | 51 |
| 25 | 9 |

## Claims

1. Conjugate, **characterized by** the formula (I)
CT-LI-Sp-IA (I)
in which
CT denotes a cytotoxic radical or a radical of a cytostatic or of a cytostatic derivative, which can additionally carry a hydroxyl, carboxyl or amino group,
LI is a linker group comprising 5 to 8 amino acid residues in the D or L configuration, which can each optionally carry protective groups ,
Sp is absent or a carbonyl or a thiocarbonyl radical,
IA is a non-peptide radical addressing an αᵥβ₃ integrin receptor, which is selected from the group consisting of
A) a radical of the formula (II) 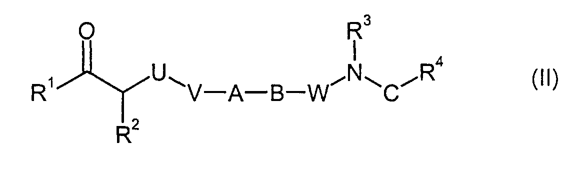 in which
R¹ is OH, a substituted or unsubstituted alkoxy or cycloalkoxy radical, a substituted or unsubstituted aryloxy radical or a saturated or unsaturated, optionally substituted heterocyclyloxy radical, or optionally represents a direct bond or an atom from the group consisting of N, O and S, via which the radical of the formula (II) is bonded to the rest of the conjugate;
R² is hydrogen, a substituted or unsubstituted alkyl or cycloalkyl radical, a substituted or unsubstituted aryl radical, a saturated or unsaturated, optionally substituted heterocyclic radical, an optionally substituted alkenyl radical or an optionally substituted alkinyl radical, via which the radical of the formula (II) is optionally bonded to the rest of the conjugate, or is -NR²'₂, -NR²'SO₂R²", -NR²'COOR²", -NR²'COR²', -NR²'CONR²'₂ or -NR²'CSNR²'₂;
in which
R^{2'} independently of one another is hydrogen, a substituted or unsubstituted alkyl or cycloalkyl radical, a substituted or unsubstituted aryl radical or a saturated or unsaturated, optionally substituted heterocyclic radical, or optionally represents a direct bond, via which the radical of the formula (II) is bonded to the rest of the conjugate;
R^{2"} is a substituted or unsubstituted alkyl or cycloalkyl radical, a substituted or unsubstituted aryl radical or a saturated or unsaturated, optionally substituted heterocyclic radical, via which the radical of the formula (II) is optionally bonded to the rest of the conjugate;
U is a direct bond or a substituted or unsubstituted alkylene group, via which the radical of the formula (II) is optionally bonded to the rest of the conjugate;
V is a substituted or unsubstituted alkylene group, -NR²'CO- or -NR²'SO₂ -, via which the radical of the formula (II) is optionally bonded to the rest of the conjugate;
A and B each independently of one another is a 1,3- or 1,4-bridged, optionally additionally substituted phenylene group;
W is a direct bond or a substituted or unsubstituted alkylene group;
C is absent or is 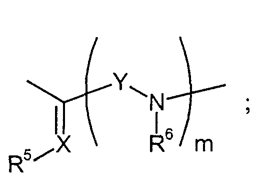
R³ is hydrogen, a substituted or unsubstituted alkyl or cycloalkyl radical, a substituted or unsubstituted aryl radical, a saturated or unsaturated, optionally substituted heterocyclic radical, an alkylamine radical, an alkylamide radical or is bonded to one of R⁴, Y, R⁵ or R⁶, if present, with formation of an optionally substituted heterocyclic ring system, which includes the nitrogen atom to which R³ is bonded and can be saturated or unsaturated and/or can contain further heteroatoms;
R⁴ is hydrogen, a substituted or unsubstituted alkyl or cycloalkyl radical, a substituted or unsubstituted aryl radical, a saturated or unsaturated, optionally substituted heterocyclic radical, an alkylamine radical, an alkylamide radical or is bonded to one of R³, Y, R⁵ or R⁶, if present, with formation of an optionally substituted heterocyclic ring system which includes the nitrogen atom to which R⁴ is bonded and can be saturated or unsaturated and/or can contain further heteroatoms, or optionally represents a direct bond, via which the radical of the formula (II) is bonded to the rest of the conjugate;
X is O, N or S;
m is 0 or 1;
Y is a direct bond or an optionally substituted alkylene or alkine group;
R⁵ is absent, -NO₂, -CN, -COR⁵', -COOR⁵', or is bonded to one of R³, Y, R⁴ or R⁶, if present, with formation of an optionally substituted carbocyclic or heterocyclic ring system which includes X and can be saturated or unsaturated and/or can contain further heteroatoms;
R^{5'} is hydrogen, a substituted or unsubstituted alkyl or cycloalkyl radical, a substituted or unsubstituted aryl radical or a saturated or unsaturated, optionally substituted heterocyclic radical which can be saturated or unsaturated and/or can contain further heteroatoms;
R⁶ is hydrogen, a substituted or unsubstituted alkyl or cycloalkyl radical, a substituted or unsubstituted aryl radical, a saturated or unsaturated, optionally substituted heterocyclic radical, an alkylamine radical, an alkylamide radical or is bonded to one of R³, R⁴, Y or R⁵, if present, with formation of an optionally substituted heterocyclic ring system which includes the nitrogen atom to which R⁶ is bonded and can be saturated or unsaturated and/or can contain further heteroatoms;
or
B) a radical of the formula (III) 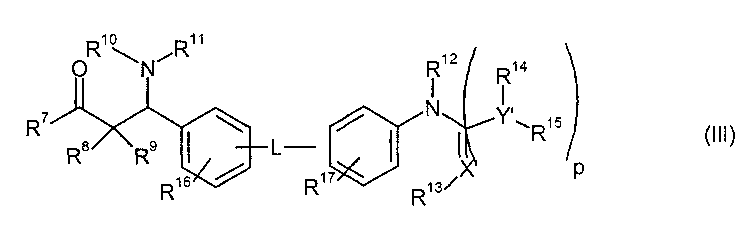 in which
R⁷ is OH, a substituted or unsubstituted alkoxy or cycloalkoxy radical, a substituted or unsubstituted aryloxy radical or a saturated or unsaturated, optionally substituted heterocyclyloxy radical, or optionally represents a direct bond or an atom from the group consisting of N, O and S, via which the radical of the formula (III) is bonded to the rest of the conjugate;
R⁸ is hydrogen, a substituted or unsubstituted alkyl or cycloalkyl radical, a substituted or unsubstituted aryl radical, a saturated or unsaturated, optionally substituted heterocyclic radical, an optionally substituted alkenyl radical, an optionally substituted alkinyl radical, a hydroxyl radical or an alkoxy radical or is bonded to R⁹ with formation of an optionally substituted carbocyclic or heterocyclic ring system which includes the carbon atom to which R⁸ is bonded and can optionally contain heteroatoms;
R⁹ is hydrogen, a substituted or unsubstituted alkyl or cycloalkyl radical, a substituted or unsubstituted aryl radical, a saturated or unsaturated, optionally substituted heterocyclic radical, an optionally substituted alkenyl radical, an optionally substituted alkinyl radical, a hydroxyl radical or an alkoxy radical or is bonded to R⁸ with formation of an optionally substituted carbocyclic or heterocyclic ring system which includes the carbon atom to which R⁹ is bonded and can optionally contain heteroatoms;
R¹⁰ is -SO₂R¹⁰', -COOR¹⁰", -COR¹⁰', -CONR¹⁰'₂ or -CS-NR¹⁰'₂, or represents a direct bond via which the radical of the formula (III) is optionally bonded to the rest of the conjugate;
R^{10'} independently of one another is hydrogen, a substituted or unsubstituted alkyl or cycloalkyl radical, a substituted or unsubstituted aryl radical or a saturated or unsaturated, optionally substituted heterocyclic radical, via which the radical of the formula (III) is optionally bonded to the rest of the conjugate;
R^{10"} is a substituted or unsubstituted alkyl or cycloalkyl radical, a substituted or unsubstituted aryl radical or a saturated or unsaturated, optionally substituted heterocyclic radical, via which the radical of the formula (III) is optionally bonded to the rest of the conjugate;
R¹¹ is hydrogen, a substituted or unsubstituted alkyl or cycloalkyl radical or a substituted or unsubstituted aryl radical,
R¹⁶ is hydrogen, CN, a substituted or unsubstituted alkyl or cycloalkyl radical, a substituted or unsubstituted alkoxy radical or a halogen atom;
R¹⁷ is hydrogen, CN, a substituted or unsubstituted alkyl or cycloalkyl radical, a substituted or unsubstituted alkoxy radical or a halogen atom;
L is -(CH₂)ₙNHSO₂(CH₂)ₒ-, -(CH₂)ₙSO₂NH(CH₂)ₒ-, -(CH₂)ₙNH-CO(CH₂)ₒ-, -(CH₂)ₙCONH(CH₂)ₒ-, -(CH₂)ₙOCH₂(CH₂)ₒ-, -(CH₂)ₙCH₂O(CH₂)ₒ-, -(CH₂)ₙCOO(CH₂)ₒ-, -(CH₂)ₙOOC-(CH₂)ₒ-, -(CH₂)ₙCH₂CO(CH₂)ₒ-, -CH₂)ₙCOCH₂(CH₂)ₒ-, -NHCONH-, -(CH₂)ₙSCH₂(CH₂)ₒ-, -(CH₂)ₙCH₂S(CH₂)ₒ-, -(CH₂)ₙCH₂SO(CH₂)ₒ-, -(CH₂)ₙSOCH₂(CH₂)ₒ-, -(CH₂)ₙCH₂SO₂(CH₂)ₒ- or -(CH₂)ₙSO₂CH₂(CH₂)ₒ-,
where n and o each is an integer of 0 or 1 and n + o ≤ 1;
R¹² is hydrogen, a substituted or unsubstituted alkyl or cycloalkyl radical, a substituted or unsubstituted aryl radical, a saturated or unsaturated, optionally substituted heterocyclic radical or is bonded to one of R¹³, R¹⁴ or R¹⁵, if present, with formation of an optionally substituted heterocyclic ring system which includes the nitrogen atom, to which R¹² is bonded and can be saturated or unsaturated and/or can contain further heteroatoms;
X' is N, O or S;
p is 0 or 1;
R¹³ is absent, is -H, a substituted or unsubstituted alkyl or cycloalkyl radical, -NO₂, -CN, -COR¹³', -COOR¹³', or is bonded to one of R¹², R¹⁴ or R¹⁵ with formation of an optionally substituted heterocyclic ring system which includes X' and can be saturated or unsaturated and/or can contain further heteroatoms;
R^{13'} is hydrogen, a substituted or unsubstituted alkyl or cycloalkyl radical, a substituted or unsubstituted aryl radical or a saturated or unsaturated, optionally substituted heterocyclic radical which can be saturated or unsaturated and/or can contain further heteroatoms;
Y' is N or S;
R¹⁴ is absent, hydrogen, a substituted or unsubstituted alkyl or cycloalkyl radical, a substituted or unsubstituted aryl radical, a saturated or unsaturated, optionally substituted heterocyclic radical or is bonded to one of R¹², R¹³ or R¹⁵, if present, with formation of an optionally substituted heterocyclic ring system which includes the nitrogen atom to which R¹⁴ is bonded and can be saturated or unsaturated and/or can contain further heteroatoms;
R¹⁵ is hydrogen, a substituted or unsubstituted alkyl or cycloalkyl radical, a substituted or unsubstituted aryl radical, a saturated or unsaturated, optionally substituted heterocyclic radical or is bonded to one of R¹², R¹³ or R¹⁴, if present, with formation of an optionally substituted heterocyclic ring system which includes the nitrogen atom to which R¹⁵ is bonded and can be saturated or unsaturated and/or can contain further heteroatoms, or optionally represents a direct bond via which the radical of the formula (III) is bonded to the rest of the conjugate;
or
C) a radical of the formula (IV) 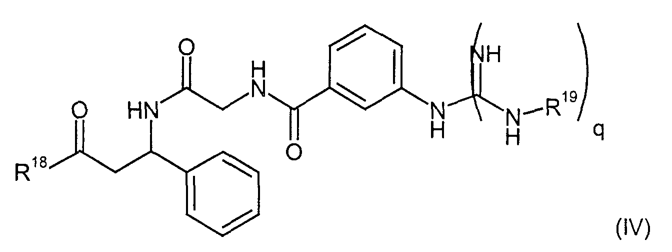 in which
R¹⁸ is OH, a substituted or unsubstituted alkoxy or cycloalkoxy radical, a substituted or unsubstituted aryloxy radical or a saturated or unsaturated, optionally substituted heterocyclyloxy radical, or optionally represents a direct bond or an atom from the group consisting of N, O and S, via which the radical of the formula (IV) is bonded to the rest of the conjugate;
q is 0 or 1;
R¹⁹ is hydrogen, a substituted or unsubstituted alkyl or cycloalkyl radical, a substituted or unsubstituted aryl radical, a saturated or unsaturated, optionally substituted heterocyclic radical, an alkylamine radical, an alkylamide radical, or optionally represents a direct bond, via which the radical of the formula (IV) is bonded to the rest of the conjugate;
and their physiologically acceptable salts and stereoisomers.

2. Conjugate according to Claim 1, **characterized in that**
LI is a linker group having the formula
-AA1-AA2-AA3-AA4-AA5-AA6-AA7-AA8-
wherein at least 5 of the radicals AA1 to AA8 are present, AA1 is bonded to the radical CT and
AA1 is a naturally occurring amino acid in the D or L configuration, which is selected from the group consisting of glycine, alanine, valine, leucine, isoleucine, histidine, glutamate, aspartate, serine, lysine, ornithine and phenylalanine;
AA2 is absent or is a naturally occurring amino acid in the D or L configuration, which is selected from the group consisting of alanine, valine, phenylalanine, tyrosine, threonine, serine, isoleucine, lysine, glutamate, histidine, glycine, arginine, asparagine, glutamine, S-methyl-cysteine, methionine, arginine, aspartate, tryptophane, proline, ornithine and leucine, and can optionally carry protective groups,
AA3 is absent or is a naturally occurring amino acid in the D or L configuration, which is selected from the group consisting of alanine, valine, phenylalanine, tyrosine, serine, isoleucine, lysine, glutamate, histidine, glycine, arginine, aspartate, tryptophane, proline, ornithine, methionine, S-methyl-cysteine, norvaline and leucine, and can optionally carry protective groups,
AA4 is absent or is a naturally occurring amino acid in the D or L configuration, which is selected from the group consisting of glycine, alanine, valine, leucine, isoleucine, cysteine and norvaline;
AA5 is absent or is a naturally occurring amino acid in the D or L configuration, which is selected from the group consisting of glycine, alanine, valine, leucine, isoleucine, histidine, tyrosine, glutamine, asparagine, proline, methionine, phenylalanine and cysteine;
AA6 is absent or is a naturally occurring amino acid in the D or L configuration, which is selected from the group consisting of glycine, alanine, valine, leucine, isoleucine, histidine, glutamine, asparagine, aspartate and proline;
AA7 is absent or is a naturally occurring amino acid in the D or L configuration, which is selected from the group consisting of glycine, alanine, valine, leucine, isoleucine, histidine, γ-aminobutyric acid, aspartate, glutamate, lysine and proline;
AA8 is absent or is a naturally occurring amino acid in the D or L configuration, which is selected from the group consisting of glycine, alanine, valine, leucine, isoleucine, histidine, lysine, proline and γ-aminobutyric acid;
and the other radicals CT, Sp and IA are as defined in claim 1.

3. Conjugate according to claim 2, **characterized in that**
LI is a linker group having the formula
-AA1-AA2-AA3-AA4-AA5-AA6-AA7-AA8-
wherein 5 to 7 of the radicals AA1 to AA8 are present, AA1 is bonded to the radical CT and
AA1 is valine, glycine, leucine, histidine;
AA2 is absent or is a naturally occurring amino acid in the D or L configuration, which is selected from the group consisting of alanine, phenylalanine, serine, isoleucine, glutamate, asparagine, glutamine, histidine, glycine, aspartate, tryptophane, proline, and leucine, and can optionally carry protective groups,
AA3 is absent or is a naturally occurring amino acid in the D or L configuration, which is selected from the group consisting of alanine, phenylalanine, serine, isoleucine, norvaline, S-methylcysteine, methionine, glutamate, histidine, glycine, aspartate, tryptophane, and leucine, and can optinally carry protective groups,
AA4 is absent or is a naturally occurring amino acid in the D or L configuration, which is selected from the group consisting of glycine, leucine, cysteine and norvaline, and can optionally carry protective groups,
AA5 is absent or is a naturally occurring amino acid in the D or L configuration, which is selected from the group consisting of glycine, alanine, valine, leucine, histidine, glutamine, phenylalanine, isoleucine, and methionine,
AA6 is absent or is a naturally occurring amino acid in the D or L configuration, which is selected from the group consisting of glycine, proline, glutamine, methionine, and leucine;
AA7 is absent or is a naturally occurring amino acid in the D or L configuration, which is selected from the group consisting of glycine, leucine, aspartate, histidine, γ-aminobutyric acid and proline;
AA8 is absent or is a naturally occurring amino acid in the D or L configuration, which is selected from the group consisting of glycine, proline and γ-aminobutyric acid;
and the other radicals CT, Sp and IA are as defined in claim 1.

4. Conjugate according to claim 2 or 3, **characterized in that**
CT is camptothecin or a camptothecin derivative, which can be bonded to the rest of the conjugate via the C20-OH group, or doxorubicine, or quinolone a;
LI is as defined in claim2 or 3;
Sp is absent, or is a carbonyl or a thiocarbonyl radical,
IA denotes a non-peptide radical of the formula (II) addressing an αᵥβ₃ integrin receptor,
in which
R¹ is OH, methoxy, ethoxy, propoxy, isopropoxy, butoxy, isobutoxy, t-butoxy, pentoxy, isopentoxy, neopentoxy, hexoxy, cyclopropoxy, cyclopropylmethoxy, cyclobutoxy, cyclopentoxy, cyclohexoxy, phenoxy, benzyloxy, tolyloxy or a substituted derivative thereof, or optionally represents a direct bond or an atom from the group consisting of N, O and S, via which the radical of the formula (II) is bonded to the rest of the conjugate;
R² is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, phenyl, benzyl, tolyl or a substituted derivative thereof, an optionally substituted alkenyl radical or an optionally substituted alkinyl radical, via which the radical of the formula (II) is optionally bonded to the rest of the conjugate, or is -NR²'₂, -NR²'SO₂R²", -NR²'COOR²", -NR^{2'}COR^{2'},-NR^{2'}CONR^{2'}₂ or -NR^{2'}CSNR^{2'}₂,
in which
R^{2'} independently of one another is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, phenyl, benzyl, tolyl or a substituted derivative thereof, or optionally represents a direct bond via which the radical of the formula (II) is bonded to the rest of the conjugate;
R2" is methyl, ethyl, propyl, isopropyl, butyl, isobutyl, t-butyl, pentyl, isopentyl, neopentyl, hexyl, cyclopropyl, cyclobutyl, cyclopentyl, cyclohexyl, phenyl, benzyl, tolyl or a substituted derivative thereof, -C₆H₂(CH₃)₃, 3-aminophenyl, 4-aminophenyl, 2-chlorophenyl, 4-chlorophenyl, 4-methoxyphenyl, 2,5-dichlorophenyl, 4-trifluoromethylphenyl, camphor-10-yl, 4-t-butylphenyl, 2,5-dimethylphenyl, 3-chlorophenyl, 2-methoxy-5-methylphenyl, 2,3,5,6-tetramethylphenyl, 2,3-dichlorophenyl, 2,6-dichlorophenyl, 2-naphthyl, 3-trifluoromethylphenyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline or 8-quinolinyl, via which the radical of the formula (II) is optionally bonded to the rest of the conjugate;
U is a direct bond,
V is an optionally substituted C₁₋₅-alkylene group, via which the radical of the formula (II) is optionally bonded to the rest of the conjugate;
A is a 1,3- or 1,4-bridged phenylene group which is unsubstituted or contains at least one alkoxy radical;
B is a 1,3- or 1,4-bridged phenylene group which is unsubstituted or contains at least one alkyl radical;
W is a direct bond or an optionally substituted C₁₋₄-alkylene group;
C is a direct bond or 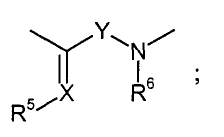
R³ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, 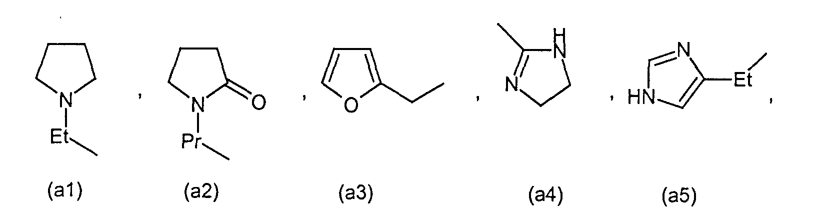 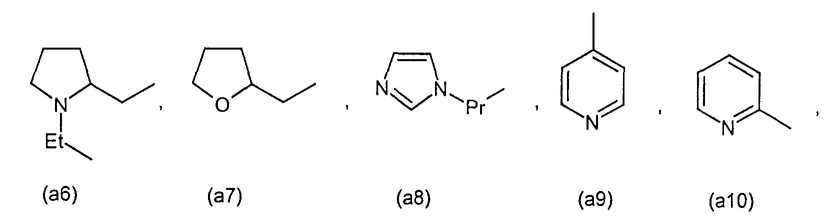 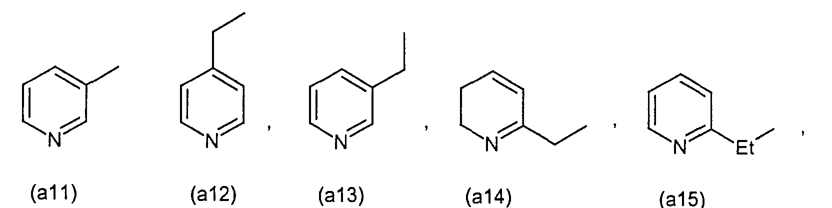 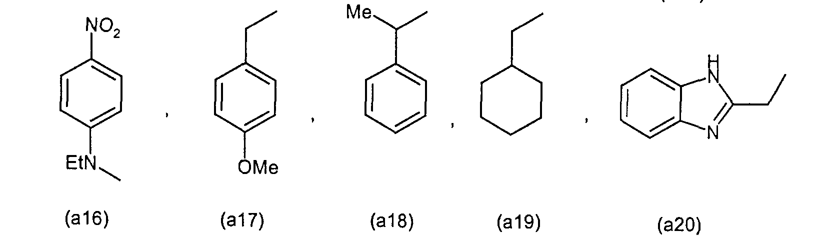 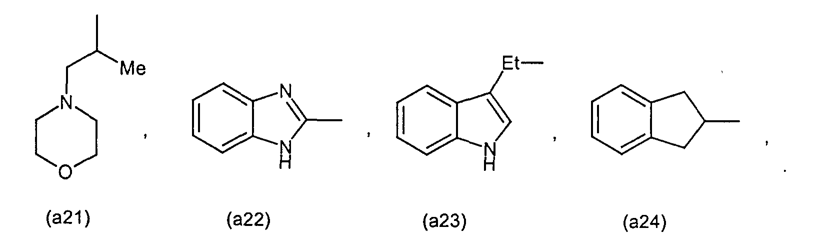 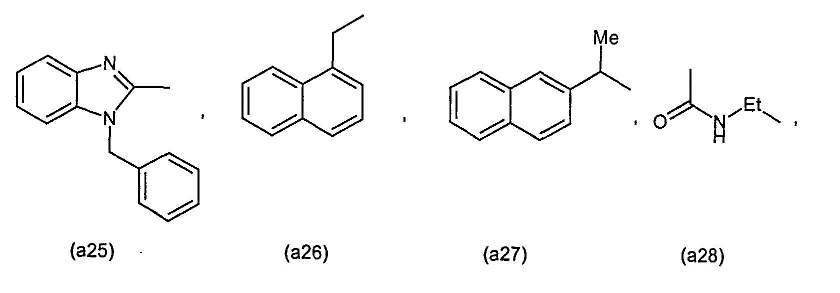 or is bonded to one of R⁴, Y, R⁵ or R⁶, if present, with formation of an optionally substituted heterocyclic 4- to 6-membered ring system, which includes the nitrogen atom to which R³ is bonded and can be saturated or unsaturated and/or can contain further heteroatoms;
R⁴ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28) or is bonded to one of R³, Y, R⁵ or R⁶, if present, with formation of an optionally substituted heterocyclic 4- to 6-membered ring system which includes the nitrogen atom to which R⁴ is bonded and can be saturated or unsaturated and/or can contain further heteroatoms, or optionally represents a direct bond via which the radical of the formula (II) is bonded to the rest of the conjugate;
X is O, N or S;
Y is a direct bond or a substituted or unsubstituted methylene or methine group;
R⁵ is absent, is -NO₂, -CN, -COR⁵', -COOR⁵' or is bonded to one of R³, Y, R⁴ or R⁶, if present, with formation of an optionally substituted carbocyclic or heterocyclic 4- to 6-membered ring system which includes X and which can be saturated or unsaturated and/or can contain further heteroatoms;
R^{5'} is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, phenyl, benzyl, tolyl or a substituted derivative thereof;
R⁶ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28) or is bonded to one of R³, Y, R⁴ or R⁵, if present, with formation of an optionally substituted heterocyclic 4-to 6-membered ring system which includes the nitrogen atom to which R⁶ is bonded and can be saturated or unsaturated and/or can contain further heteroatoms.

5. Conjugate according to Claim 4, **characterized in that**
R¹ represents a direct bond or an atom from the group consisting of N, O and S, via which the radical of the formula (II) is bonded to the rest of the conjugate;
and the other radicals of the formula (II) are as defined in Claim 4.

6. Conjugate according to Claim 4, **characterized in that**
R⁴ represents a direct bond, via which the radical of the formula (II) is bonded to the rest of the conjugate;
and the other radicals of the formula (II) are as defined in Claim 4.

7. Conjugate according to Claim 4, **characterized in that** the radical of the formula (II) is linked to the rest of the conjugate via a radical in the α- or β-position relative to the carboxyl group,
and the other radicals of the formula (II) are as defined in Claim 4.

8. Conjugate according to Claim 2 or 3, **characterized in that**
CT is camptothecin or a camptothecin derivative, which can be linked to the rest of the conjugate via the C20-OH group, or doxorubicine or quinolone a;
LI is as defined in claim 2 or 3;
Sp is absent, or a carbonyl or a thiocarbonyl radical,
IA is a non-peptide radical of the formula (II) addressing an αᵥβ₃ integrin receptor,
in which
R¹ is OH, methoxy, ethoxy, propoxy, isopropoxy, butoxy, isobutoxy, t-butoxy, pentoxy, isopentoxy, neopentoxy, hexoxy, cyclopropoxy, cyclopropylmethoxy, cyclobutoxy, cyclopentoxy, cyclohexoxy, phenoxy, benzyloxy, tolyloxy or a substituted derivative thereof, or optionally represents a direct bond or an atom from the group consisting of N, O and S, via which the radical of the formula (II) is bonded to the rest of the conjugate;
R² is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, phenyl, benzyl, 4-aminobenzyl, tolyl, phenylethyl, a substituted derivative such as 4-aminobenzyl or a saturated or unsaturated, optionally substituted heterocyclic analogue thereof, an optionally substituted alkenyl radical, an optionally substituted alkinyl radical, via which the radical of the formula (II) is optionally bonded to the rest of the conjugate;
U is a direct bond or an optionally substituted C₁₋₃-alkylene group such as -CH(C₆H₄-3-NH)- or -CH(C₆H₄-4-NH)-, via which the radical of the formula (II) is optionally bonded to the rest of the conjugate;
V is -NR²⁰CO- or -NR²⁰SO₂-;
R²⁰ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, phenyl, benzyl, tolyl, phenylethyl, phenylpropyl, phenoxyethyl or a substituted derivative thereof;
A is a 1,3- or 1,4-bridged phenylene group which is unsubstituted or contains at least one alkoxy radical;
B is a 1,3- or 1,4-bridged phenylene group which is unsubstituted or contains at least one alkyl radical;
W is a direct bond or an optionally substituted C₁₋₃-alkylene group;
C is 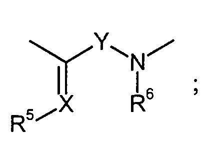
R³ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28) or is bonded to one of R⁴, Y or R⁶, if present, with formation of an optionally substituted heterocyclic 4- to 6-membered ring system, which includes the nitrogen atom to which R³ is bonded, and can be saturated or unsaturated and/or can contain further heteroatoms;
R⁴ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28) or is bonded to one of R³, Y or R⁶, if present, with formation of an optionally substituted heterocyclic 4- to 6-membered ring system, which includes the nitrogen atom to which R⁴ is bonded and can be saturated or unsaturated and/or can contain further heteroatoms, or optionally represents a direct bond via which the radical of the formula (II) is bonded to the rest of the conjugate;
X is O or S;
Y is a direct bond or a substituted or unsubstituted methylene or methine group;
R⁵ is absent;
R⁶ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28) or is bonded to one of R³, Y or R⁴, if present, with formation of an optionally substituted heterocyclic 4- to 6-membered ring system which includes the nitrogen atom to which R⁶ is bonded, and can be saturated or unsaturated and/or can contain further heteroatoms.

9. Conjugate according to Claim 8, **characterized in that**
R¹ represents a direct bond or an atom from the group consisting of N, O and S, via which the radical of the formula (II) is bonded to the rest of the conjugate;
and the other radicals of the formula (II) are as defined in Claim 8.

10. Conjugate according to Claim 8, **characterized in that**
R⁴ represents a direct bond, via which the radical of the formula (II) is bonded to the rest of the conjugate;
and the other radicals of the formula (II) are as defined in Claim 8.

11. Conjugate according to Claim 8, **characterized in that**
the radical of the formula (II) is linked to the rest of the conjugate via a radical in the α- or β-position relative to the carboxyl group;
and the other radicals of the formula (II) are as defined in Claim 8.

12. Conjugate according to Claim 2 or 3, **characterized in that**
CT is camptothecin, which can be linked to the rest of the conjugate via the C20-OH group;
LI is as defined in claim 2 or 3;
Sp is absent, or a carbonyl or a thiocarbonyl radical,
IA is a non-peptide radical of the formula (III) addressing an αᵥβ₃ integrin receptor,
in which
R⁷ is OH, methoxy, ethoxy, propoxy, isopropoxy, butoxy, isobutoxy, t-butoxy, pentoxy, isopentoxy, neopentoxy, hexoxy, cyclopropoxy, cyclopropylmethoxy, cyclobutoxy, cyclopentoxy, cyclohexoxy, phenoxy, benzyloxy, tolyloxy or a substituted derivative thereof, or optionally represents a direct bond or an atom from the group consisting of N, O and S, via which the radical of the formula (III) is bonded to the rest of the conjugate;
R⁸ is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -OH, methoxy, ethoxy, propoxy, butoxy, pentoxy, hexoxy, benzyloxy or is bonded to R⁹ with formation of an optionally substituted 3- to 6-membered carbocyclic or heterocyclic ring system, which includes the carbon atom to which R⁸ is bonded and can optionally contain heteroatoms;
R⁹ is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -OH, methoxy, ethoxy, propoxy, butoxy, pentoxy, hexoxy or is bonded to R⁸ with formation of an optionally substituted 3- to 6-membered carbocyclic or heterocyclic ring system which includes the carbon atom to which R⁹ is bonded and can optionally contain heteroatoms;
R¹⁰ is SO₂R¹⁰', -COOR¹⁰'', -COR¹⁰', -CONR¹⁰'₂ or -CSNR¹⁰'₂ or represents a direct bond, via which the radical of the formula (III) is optionally bonded to the rest of the conjugate;
R¹⁰' is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -C₆H₂(CH₃)₃, -C₆(CH₃)₅, -CH₂C₆H₂(CH₃)₃, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 4-chlorophenylmethyl, 2,4-dichloro-phenylmethyl, 2,6-dichlorophenylmethyl, 3-aminophenyl, 4-aminophenyl, 2-methoxycarbonylphenylmethyl, 3-trifluoromethylphenyl, 4-trifluoromethylphenyl, 3,5-bis(trifluoromethyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluoro-phenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methylbenzothiazol-2-yl, N-meth-oxycarbonylpiperidin-3-yl, thiophen-2-yl, isoxazol-5-yl, ethoxy, 2-chloropyridin-3-yl, pyridin-3-yl, benzyloxy, 5-methylisoxazol-3-yl, 1-adamantyl, 4-chlorophenoxymethyl, 2,2-dimethylethenyl, 2-chloropyridine-5-methyl, 5,7-dimethyl-l,3,4-triazaindolizin-2-yl, (S)-camphan-1-yl, (R)-camphan-1-yl or 8-quinolinyl;
R¹⁰" is a C₁₋₆-alkyl radical, a C₃₋₇-cycloalkyl radical, a substituted or unsubstituted aryl radical or a saturated or unsaturated, optionally substituted heterocyclic radical, via which the radical of the formula (III) is optionally bonded to the rest of the conjugate;
R¹¹ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, dialkylamino-C₁₋₄-alkyl, amino-C₁₋₄-alkyl, C₁₋₄-alkyloxy-C₁₋₄-alkyl or 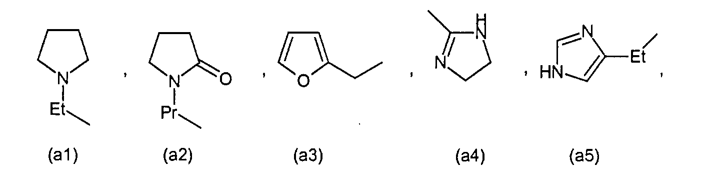 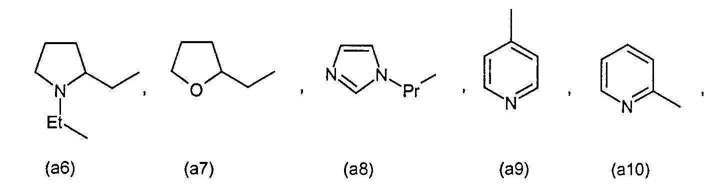 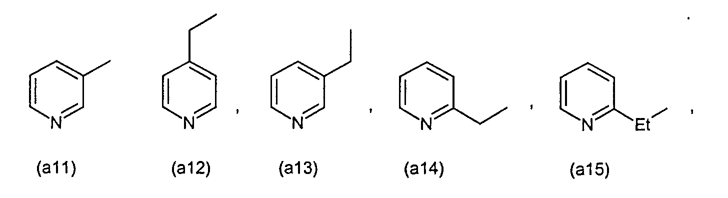 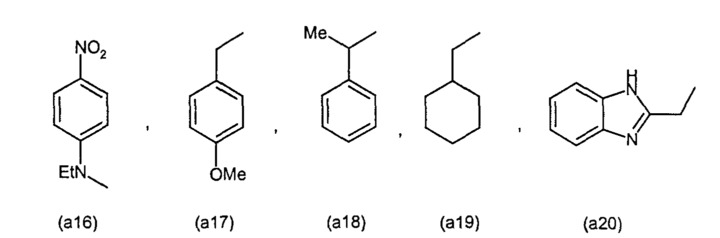 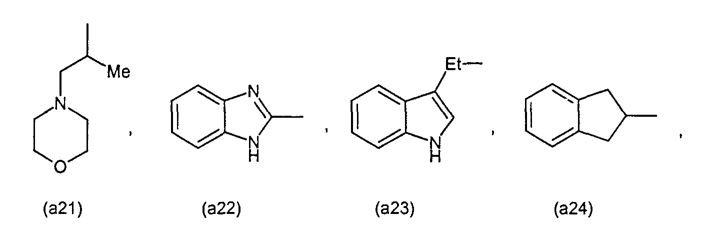 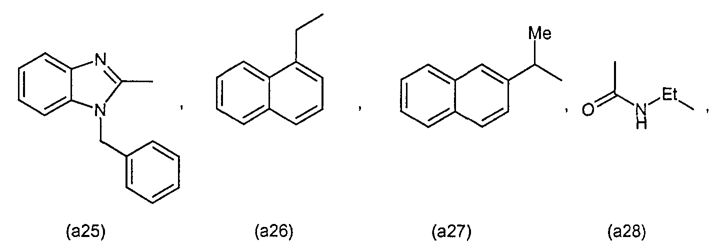
R¹⁶ is hydrogen, CN, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, methoxy, trifluoromethoxy, ethoxy, propoxy, butoxy, pentoxy or hexoxy, fluorine, chlorine, bromine or iodine;
R¹⁷ is hydrogen, CN, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, methoxy, trifluoromethoxy, ethoxy, propoxy, butoxy, pentoxy or hexoxy, fluorine, chlorine, bromine or iodine;
L is -NHSO₂-, -CH₂NHSO₂-, -NHSO₂CH₂-, -SO₂NH-, -CH₂SO₂NH-, -SO₂NHCH₂-, -NHCO-, -CH₂NHCO-, -NHCOCH₂-, -CONH-, -CH₂CONH-, -CONHCH₂-, -OCH₂-, -CH₂OCH₂, -OCH₂CH₂-, -CH₂O- or -CH₂CH₂O-;
R¹² is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28) or is bonded to one of R¹³, R¹⁴ or R¹⁵, if present, with formation of an optionally substituted heterocyclic 4- to 6-membered ring system which includes the nitrogen atom to which R¹² is bonded and can be saturated or unsaturated and/or can contain further heteroatoms;
X' is N, O or S;
p is 0 or 1;
R¹³ is absent, is -H, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, -NO₂, -CN, -COR⁷', -COOR⁷', or is connected to one of R¹², R¹⁴ or R¹⁵ with formation of an optionally substituted carbocyclic or heterocyclic 4- to 6-membered ring system which includes X' and can be saturated or unsaturated and/or can contain further heteroatoms;
R^{13'} is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof;
Y' is N or S;
R¹⁴ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28), or is bonded to one of R¹², R¹³ or R¹⁵, if present, with formation of an optionally substituted heterocyclic 4- to 6-membered ring system which includes the nitrogen atom to which R¹⁴ is bonded and can be saturated or unsaturated and/or can contain further heteroatoms; and
R¹⁵ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28) or is bonded to one of R¹², R¹³ or R¹⁴, if present, with formation of an optionally substituted heterocyclic 4- to 6-membered ring system which includes the nitrogen atom to which R¹⁵ is bonded and can be saturated or unsaturated and/or can contain further heteroatoms, and or optionally represents a direct bond via which the radical of the formula (III) is bonded to the rest of the conjugate.

13. Conjugate according to Claim 12, **characterized in that**
R⁷ represents a direct bond or an atom from the group consisting of N, O and S, via which the radical of the formula (II) is bonded to the rest of the conjugate;
and the other radicals of the formula (III) are as defined in Claim 12.

14. Conjugate according to Claim 12, **characterized in that**
R¹⁵ represents a direct bond, via which the radical of the formula (III) is bonded to the rest of the conjugate;
and the other radicals of the formula (III) are as defined in Claim 12.

15. Conjugate according to Claim 12, **characterized in that**
the radical of the formula (III) is linked to the rest of the conjugate via a radical in the α- or β-position relative to the carboxyl group,
and the other radicals of the formula (III) are as defined in Claim 12.

16. Conjugate according to Claim 2 or 3, **characterized in that**
IA is a non-peptide radical of the formula (IV) addressing an αᵥβ₃ integrin receptor,
wherein
R¹⁸ represents a direct bond or an atom from the group consisting of N, O and S, via which the radical of the formula (IV) is bonded to the rest of the conjugate;
and the other radicals are as defined in Claim 2 or 3.

17. Conjugate according to Claim 2 or 3, **characterized in that**
IA is a non-peptide radical of the formula (IV) addressing an αᵥβ₃ integrin receptor,
wherein
R¹⁹ represents a direct bond, via which the radical of the formula (IV) is bonded to the rest of the conjugate;
and the other radicals are as defined in Claim 2 or 3.

18. Process for the preparation of conjugates according to Claim 1, comprising
[A] the reaction of a compound from the group of compounds of the formulae (II), (III) and (IV), which has a free or optionally activated carboxyl function,
with a compound of the formula (Ia) which has a free primary or secondary amino group
CT-LI (Ia)
in which all radicals have the meaning indicated in Claim 1,
in the presence of a base;
or
[B] the reaction of a compound from the group of compounds of the formulae (II), (III) and (IV), which has a free primary or secondary amino function,
with a carbonic acid derivative such as, for example, phosgene, thiophosgene or a chloroformic acid ester, if appropriate in the presence of a base,
followed by the reaction with a compound of the formula (Ia) which has a free primary or secondary amino group
CT-LI (Ia)
in which all radicals have the meaning indicated in Claim 1,
and
if appropriate the removal of protective groups and/or derivatization of nitrogen atoms present at preferred points of time in the preparation process and/or conversion of the compound obtained into the free acid and/or conversion of the compound obtained into one of its physiological salts by reaction with an inorganic or organic base or acid;
or
[C] the reaction of a compound from the group of compounds of the formulae (II), (III) and (IV), which contains a free primary or secondary amino function,
with a compound of the formula (Ia) which contains a free or optionally activated carboxyl function
CT-LI (Ia)
in which all radicals have the meaning indicated in Claim 1,
in the presence of a base;
and
if appropriate the removal of protective groups and/or derivatization of nitrogen atoms present at preferred points in time in the preparation process and/or conversion of the compound obtained into the free acid and/or conversion of the compound obtained into one of its physiological salts by reaction with an inorganic or organic base or acid.

19. Process according to Claim 18, **characterized in that** several steps of the process are carried out on a solid phase.

20. Medicament, comprising at least one of the conjugates according to one of Claims 1 to 17.

21. Use of compounds according to one of Claims 1 to 17 for the production of medicaments for the treatment of carcinomatous disorders.
